# EUROPEAN PATENT APPLICATION

(11) **EP 4 591 888 A2**
(43) Date of publication of application: **30.07.2025**
(21) Application number: 25177759.5
(22) Date of filing: 17.06.2019
(51) Int. Cl.: A61K 48/00

(54) **ADENO-ASSOCIATED VIRUS VECTOR DELIVERY OF MUSCLE SPECIFIC MICRO-DYSTROPHIN TO TREAT MUSCULAR DYSTROPHY**

(30) Priority: 18.06.2018 US 201862686668 P; 02.10.2018 US 201862740402 P; 30.10.2018 US 201862752841 P; 25.03.2019 US 201962823649 P; 11.06.2019 US 201962860220 P
(62) Divisional of application: 19749864.5
(71) Applicant: Research Institute at Nationwide Children's Hospital, Columbus, Ohio 43205 (US)
(72) Inventor: RODINO-KLAPAC, Louise, Groveport 43125 (US); MENDELL, Jerry, R, Columbus 43235 (US)
(74) Representative: D Young & Co LLP

(57) **Abstract**

The invention provides gene therapy vectors, such as adeno-associated virus (AAV) vectors, expressing a miniaturized human micro-dystrophin gene and method of using these vectors to express micro-dystrophin in skeletal muscle s including diaphragm and cardiac muscle and to protect muscle fibers from injury, increase muscle strength and reduce and/or prevent fibrosis in subjects suffering from muscular dystrophy.

## Description

This application claims priority benefit to U.S. Provisional Patent Application No. 62/686,668, filed June 18, 2018; U.S. Provisional Patent Application No. 62/740,402, filed October 2, 2018; U.S. Provisional Patent Application No. 62/752,841, filed October 30,2018; U.S. Provisional Patent Application No. 62/823,649, filed March 25, 2019 and U.S. Provisional Patent Application No. 62/860,220 filed June 11, 2018 each of which are incorporated by reference herein in their entirety.

### INCORPORATION BY REFERENCE OF MATERIAL SUBMITTED ELECTRONICALLY

This application contains, as a separate part of the disclosure, a Sequence Listing in computer-readable form which is incorporated by reference in its entirety and identified as follows: Filename: 53169ARTW_Seqlisting.txt; Size: 60,103 bytes, created; June 17, 2019.

### FIELD OF INVENTION

The invention provides gene therapy vectors, such as adeno-associated virus (AAV) vectors, expressing a miniaturized human micro-dystrophin gene and method of using these vectors to express micro-dystrophin in skeletal muscles including diaphragm and cardiac muscle and to protect muscle fibers from injury, increase muscle strength and reduce and/or prevent fibrosis in subjects suffering from muscular dystrophy.

### BACKGROUND

The importance of muscle mass and strength for daily activities, such as locomotion and breathing, and for whole body metabolism is unequivocal. Deficits in muscle function produce muscular dystrophies (MDs) that are characterized by muscle weakness and wasting and have serious impacts on quality of life. The most well-characterized MDs result from mutations in genes encoding members of the dystrophin-associated protein complex (DAPC). These MDs result from membrane fragility associated with the loss of sarcolemmal-cytoskeleton tethering by the DAPC. Duchenne Muscular Dystrophy (DMD) is one of the most devastating muscle disease affecting 1 in 5000 newborn males.

DMD is caused by mutations in the DMD gene leading to reductions in mRNA and the absence of dystrophin, a 427 kD sarcolemmal protein associated with the dystrophin-associated protein complex (DAPC) (Hoffman et al., Cell 51(6):919-28, 1987). The DAPC is composed of multiple proteins at the muscle sarcolemma that form a structural link between the extra-cellular matrix (ECM) and the cytoskeleton via dystrophin, an actin binding protein, and alpha-dystroglycan, a laminin-binding protein. These structural links act to stabilize the muscle cell membrane during contraction and protect against contraction-induced damage. With dystrophin loss, membrane fragility results in sarcolemmal tears and an influx of calcium, triggering calcium-activated proteases and segmental fiber necrosis (Straub et al., Curr Opin. Neurol. 10(2): 168-75, 1997). This uncontrolled cycle of muscle degeneration and regeneration ultimately exhausts the muscle stem cell population (Sacco et al., Cell, 2010. 143(7): p. 1059-71; Wallace et al., Annu Rev Physiol, 2009. 71: p. 37-57), resulting in progressive muscle weakness, endomysial inflammation, and fibrotic scarring.

Without membrane stabilization from dystrophin or a micro-dystrophin, DMD will manifest uncontrolled cycles of tissue injury and repair ultimately replace lost muscle fibers with fibrotic scar tissue through connective tissue proliferation. Fibrosis is characterized by the excessive deposits of ECM matrix proteins, including collagen and elastin. ECM proteins are primarily produced from cytokines such as TGFβ that is released by activated fibroblasts responding to stress and inflammation. Although the primary pathological feature of DMD is myofiber degeneration and necrosis, fibrosis as a pathological consequence has equal repercussions. The overproduction of fibrotic tissue restricts muscle regeneration and contributes to progressive muscle weakness in the DMD patient. In one study, the presence of fibrosis on initial DMD muscle biopsies was highly correlated with poor motor outcome at a 10-year follow-up (Desguerre et al., J Neuropathol Exp Neurol, 2009. 68(7): p. 762-7). These results point to fibrosis as a major contributor to DMD muscle dysfunction and highlight the need for early intervention prior to overt fibrosis.

There is a need for treatments that increase muscle strength and protect against muscle injury in patients suffering from DMD.

### SUMMARY OF INVENTION

The present invention is directed to gene therapy vectors, e.g. AAV, expressing the micro-dystrophin gene to skeletal muscles including diaphragm and cardiac muscle to protect muscle fibers from injury, increase muscle strength and reduce and/or prevent fibrosis.

The invention provides for therapies and approaches for increasing muscular force and/or increasing muscle mass using gene therapy vectors to deliver micro-dystrophin to address the gene defect observed in DMD. Example 2 describes a systemic gene delivery clinical trial for Duchenne muscular dystrophy, in this study the subjects received 2x1014 vg/kg AAVrh74.MHCK7.micro-dystrophin. The clinical study described in Example 3 provides a novel pivotal clinical protocol that includes a randomized double-blind placebo controlled design. At study initiation, subjects are randomized and either receive 2x10¹⁴ vg/kg AAVrh74.MHCK7.micro-dystrophin or lactated ringers.

The invention provides for nucleic acid molecules comprising the nucleotide sequence of SEQ ID NO: 3, 8 or 9. The invention also provides for rAAV comprising the nucleic acid sequence of SEQ ID NO: 9 or nucleotides 1-4977 of SEQ ID NO: 8 or nucleotides 55-5021 of SEQ ID NO: 3, and rAAV particles comprising the nucleic acid sequence of SEQ ID NO: 9 or nucleotides 1-4977 of SEQ ID NO: 8 or nucleotides 55-5021 of SEQ ID NO: 3.

Another aspect of the invention provides for compositions comprising a nucleic acid molecule comprising the nucleotide sequence of SEQ ID NO: 3, 8 or 9, rAAV comprising the nucleic acid sequence of SEQ ID NO: 9 or nucleotides 1-4977 of SEQ ID NO: 8 or nucleotides 55-5021 of SEQ ID NO: 3, and rAAV particles comprising the nucleic acid sequence of SEQ ID NO: 9 or nucleotides 1-4977 of SEQ ID NO: 8 or nucleotides 55-5021 of SEQ ID NO: 3. Any of the methods disclosed herein may be carried out with these compositions.

The invention provides for methods of treating a muscular dystrophy in a human subject in need thereof comprising the step of administering a recombinant adeno-virus associated (rAAV) rAAV.MHCK7.microdystrophin, wherein the rAAV is administered by a systemic route of administration at a dose of about 5.0x10¹² vg/kg to about 1.0x10¹⁵vg/kg. The muscular dystrophy may be Duchenne muscular dystrophy or Becker's muscular dystrophy.

For example, the dose of rAAV administered is about 5.0x10¹² vg/kg to about 1.0x10¹⁴ vg/kg, or about 5.0x10¹² vg/kg to1.0x10¹⁴ vg/kg, or about 5.0x10¹² vg/kg to about 2.0x10¹⁴ vg/kg, or about 5.0x10¹² vg/kg to about 1.0x10¹⁴ vg/kg, or about 5.0x10¹² vg/kg to about 5.0x10¹³ vg/kg, or about 5.0x10¹² vg/kg to about 2.0x10¹³ vg/kg, or about 5.0x10¹² vg/kg to about 1.0x10¹³ vg/kg, or 1.0x10¹⁴ vg/kg to about 1.0x10¹⁵ vg/kg, or 1.0x10¹³ vg/kg to about 1.0x10¹⁴ vg/kg, or about 1.0x10¹³ vg/kg to1.0x10¹⁴ vg/kg, or about 1.0x10¹³ vg/kg to about 2.0x10¹⁴ vg/kg, or about 1.0x10¹³ vg/kg to about 1.0x10¹⁴ vg/kg, or about 1.0x10¹³ vg/kg to about 5.0x10¹³ vg/kg, or about 1.0x10¹³ vg/kg to about 3.0x10¹⁴ vg/kg, or about 1.0x10¹³ vg/kg to about 5.0x10¹⁴ vg/kg, or about 1.0x10¹³ vg/kg to about 6.0x10¹⁴ vg/kg, or 1.0x10¹³ vg/kg to about 1.0x10¹⁵ vg/kg, or 5.0x10¹³ vg/kg to about 1.0x10¹⁴ vg/kg, or about 5.0x10¹³ vg/kg to1.0x10¹⁴ vg/kg, or about 5.0x10¹³ vg/kg to about 2.0x10¹⁴ vg/kg, or about 5.0x10¹³ vg/kg to about 1.0x10¹⁴vg/kg, or about 5.0x10¹³ vg/kg to about 3.0x10¹⁴ vg/kg, or about 5.0x10¹³ vg/kg to about 5.0x10¹⁴ vg/kg, or about 5.0x10¹³ vg/kg to about 6.0x10¹⁴ vg/kg, or 5.0x10¹³ vg/kg to about 1.0x10¹⁵ vg/kg, or 1.0x10¹⁴ vg/kg to about 6.0x10¹⁴ vg/kg, or 1.0x10¹⁴ vg/kg to about 5.0x10¹⁴ vg/kg, or 1.0x10¹⁴ vg/kg to about 4.0x10¹⁴ vg/kg, or 1.0x10¹⁴ vg/kg to about 1.0x10¹⁵ vg/kg, or 1.0x10¹⁴ vg/kg to about 3.0x10¹⁴ vg/kg, or about 1.0x10¹⁴ vg/kg to about 2.5x10¹⁴ vg/kg, or 1.0x10¹⁴ vg/kg to about 2.0x10¹⁴ vg/kg, or about 1.25x10¹⁴ vg/kg to about 3.75x10¹⁴ vg/kg, or about 1.25x10¹⁴ vg/kg to 6.0x10¹⁴, or about 1.25x10¹⁴ vg/kg to 5.0x10¹⁴, or about 1.25x10¹⁴ vg/kg to 4.0x10¹⁴, or about 1.25x10¹⁴ vg/kg to 1.0x10¹⁵, or about 1.25x10¹⁴ vg/kg to about 3.5x10¹⁴ vg/kg, or about 1.25x10¹⁴ vg/kg to about 3.0x10¹⁴ vg/kg, or about 1.25x10¹⁴ vg/kg to about 2.75x10¹⁴ vg/kg, or about 1.25x10¹⁴ vg/kg to about 2.5x10¹⁴ vg/kg, or about 1.25x10¹⁴ vg/kg to about 2.0x10¹⁴ vg/kg, or 1.25x10¹⁴ vg/kg to about 3.75x10¹⁴ vg/kg, or about 1.25x10¹⁴ vg/kg to about 3.5 x10¹⁴ vg/kg, or 1.5x10¹⁴ vg/kg to about 1.0x10¹⁵ vg/kg, or about 1.5x10¹⁴ vg/kg to 6.0x10¹⁴, or about 1.5x10¹⁴ vg/kg to 5.0x10¹⁴, or about 1.5x10¹⁴ vg/kg to 4.0x10¹⁴, or about 1.5x10¹⁴ vg/kg to about 3.75x10¹⁴ vg/kg, or about 1.5x10¹⁴ vg/kg to about 3.5x10¹⁴ vg/kg, or about 1.5x10¹⁴ vg/kg to about 3.25x10¹⁴ vg/kg, or about 1.5x10¹⁴ vg/kg to about 3.0x10¹⁴ vg/kg, or about 1.5x10¹⁴ vg/kg to about 2.75x10¹⁴ vg/kg, or about 1.5x10¹⁴ vg/kg to about 2.5x10¹⁴ vg/kg, or about 1.5x10¹⁴ vg/kg to about 2.0x10¹⁴ vg/kg, or 1.75x10¹⁴ vg/kg to about 1.0x10¹⁵ vg/kg, or about 1.75x10¹⁴ vg/kg to 6.0x10¹⁴, or about 1.75x10¹⁴ vg/kg to 5.0x10¹⁴, or about 1.75x10¹⁴ vg/kg to 4.0x10¹⁴, or about 1.75x10¹⁴ vg/kg to about 3.75x10¹⁴ vg/kg, or about 1.75x10¹⁴ vg/kg to about 3.5x10¹⁴ vg/kg, or about 1.75x10¹⁴ vg/kg to about 3.25x10¹⁴ vg/kg, or about 1.75x10¹⁴ vg/kg to about 3.0x10¹⁴ vg/kg, or about 1.75x10¹⁴ vg/kg to about 2.75x10¹⁴ vg/kg, or about 1.75x10¹⁴ vg/kg to about 2.5x10¹⁴ vg/kg, or about 1.75x10¹⁴ vg/kg to about 2.25x10¹⁴ vg/kg, or about 1.75x10¹⁴ vg/kg to about 2.0x10¹⁴ vg/kg, or about 2.0x10¹⁴ vg/kg to 1.0x10¹⁵, or about 2.0x10¹⁴ vg/kg to 6.0x10¹⁴, or about 2.0x10¹⁴ vg/kg to 5.0x10¹⁴, or about 2.0x10¹⁴ vg/kg to about 4.0x10¹⁴ vg/kg, or about 2.0x10¹⁴ vg/kg to about 3.75x10¹⁴ vg/kg, or about 2.0x10¹⁴ vg/kg to about 3.5x10¹⁴ vg/kg, or about 2.0x10¹⁴ vg/kg to about 3.25x10¹⁴ vg/kg.

In one embodiment, the methods of the invention comprise systemically administering rAAV wherein the systemic route of administration is an intravenous route and the dose of the rAAV administered is about 2.0 x10¹⁴ vg/kg. In another embodiment, the methods of the invention comprise systemically administering rAAV wherein the systemic route of administration is an intravenous route and the dose of the rAAV administered is about 5.0x10¹² vg/kg, or about 6.0x10¹² vg/kg, or about 7.0x10¹² vg/kg, or about 8.0x10¹² vg/kg, or about 9.0x10¹² vg/kg, or about 1.0x10¹³ vg/kg, or about 1.25x10¹³ vg/kg, or about 1.5x10¹³ vg/kg, or about 1.75x10¹³ vg/kg, or about 2.25x10¹³ vg/kg, or about 2.5x10¹³ vg/kg, or about 2.75x10¹³ vg/kg, or about 3.0x10¹³ vg/kg, or about 3.25x10¹³ vg/kg, or about 3.5x10¹³ vg/kg, or about 3.75x10¹³ vg/kg, or about 4.0x10¹³ vg/kg, or about 5.0x10¹³ vg/kg, or about 6.0x10¹³ vg/kg, or about 7.0x10¹³ vg/kg, or about 8.0x10¹³ vg/kg, or about 9.0x10¹³ vg/kg, or about 1.0x10¹⁴ vg/kg, or about 1.25x10¹⁴ vg/kg, or about 1.5x10¹⁴ vg/kg, or about 1.75x10¹⁴ vg/kg, or about 2.25x10¹⁴ vg/kg, or about 2.5x10¹⁴ vg/kg, or about 2.75x10¹⁴ vg/kg, or about 3.0x10¹⁴ vg/kg, or about 3.25x10¹⁴ vg/kg, or about 3.5x10¹⁴ vg/kg, or about 3.75x10¹⁴ vg/kg, or about 4.0x10¹⁴ vg/kg, or about 5.0x10¹⁴ vg/kg, or about 6.0x10¹⁴ vg/kg, or about 1x10¹⁵ vg/kg. In one embodiment, the rAAV is AAVrh74.MHCK7.microdystrophin or AAVrh74.MCK.microdystrophin. In one embodiment, the rAAV is the AAVrh74.MHCK7.microdystrophin of SEQ ID NO: 9, nucleotides 55-5021 of SEQ ID NO: 3, nucleotides 1-4977 of SEQ ID NO: 8 or nucleotides 56-5022 of SEQ ID NO: 6. In one embodiment, the rAAV is the AAVrh74.MCK.microdystrophin of nucleotides 56-4820 of SEQ ID NO: 5.

In any of the methods of the invention, the dose of rAAV can be administered at about 5 mL/kg to about 15 mL/kg, or about 8 mL/kg to about 12 mL/kg, or 8 mL/kg to about 10 mL/kg, or 5 mL/kg to about 10 mL/kg or about 10 mL/kg to 12 mL/k, or about 10 mL/kg to 15 mL/kg or 10 mL/kg to about 20 mL/kg. In a particular embodiment, the dose or the rAAV is administered in about 10 mL/kg. In one embodiment, the rAAV is AAVrh74.MHCK7.microdystrophin or AAVrh74.MCK.microdystrophin. In one embodiment, the rAAV is the AAVrh74.MHCK7.microdystrophin of SEQ ID NO: 9, nucleotides 55-5021 of SEQ ID NO: 3, nucleotides 1-4977 of SEQ ID NO: 8 or nucleotides 56-5022 of SEQ ID NO: 6. In one embodiment, the rAAV is the AAVrh74.MCK.microdystrophin of nucleotides 56-4820 of SEQ ID NO: 5.

In any of the methods of the invention, the dose of rAAV can be administered by injection, infusion or implantation. For example, the dose of rAAV is administered by infusion over approximately one hour. In addition, the dose of rAAV is administered by an intravenous route through a peripheral limb vein, such as a peripheral arm vein or a peripheral leg vein. Alternatively, the infusion may be administered over approximately 30 minutes, or approximately 1.5 hours, or approximately 2 hours, or approximately 2.5 hours or approximately 3 hours. In one embodiment, the rAAV is AAVrh74.MHCK7.microdystrophin. In one embodiment, the AAVrh74.MHCK7.microdystrophin is the AAVrh74.MHCK7.microdystrophin of SEQ ID NO: 9, nucleotides 55-5021 of SEQ ID NO: 3, nucleotides 1-4977 of SEQ ID NO: 8 or nucleotides 56-5022 of SEQ ID NO: 6. In one embodiment, the rAAV is AAVrh74.MCK.microdystrophin. In one embodiment, the AAVrh74.MCK.microdystrophin is the AAVrh74.MCK.microdystrophin of nucleotides 56-4820 of SEQ ID NO: 5.

The rAAV administered by any of the methods of the invention can comprise the human micro-dystrophin nucleotide sequence of SEQ ID NO: 1, the MHCK7 promoter sequence of SEQ ID NO: 2 or SEQ ID NO:7. In addition, the rAAV administered by any of the methods of the invention comprises the human micro-dystrophin nucleotide sequence of SEQ ID NO: 1 and the MHCK7 promoter sequence of SEQ ID NO: 2 or SEQ ID NO:7. For example, the rAAV can comprise the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. In one embodiment, the rAAV is AAVrh74.MHCK7.microdystrophin. In one embodiment, the AAVrh74.MHCK7.microdystrophin is the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ IDNO: 6.

In one embodiment, the rAAV is AAVrh74.MCK.microdystrophin. In one embodiment, the AAVrh74.MCK.microdystrophin is the AAVrh74.MCK.microdystrophin of nucleotides 56-4820 of SEQ ID NO: 5.

In any of the methods of the invention, the rAAV administered is of the serotype AAVrh7.4.

In some embodiments, the methods of the invention treat Duchenne muscular dystrophy or Becker's muscular dystrophy. An exemplary embodiment is a method of treating Duchenne muscular dystrophy or Becker's muscular dystrophy in a human subject in need thereof comprising the step of administering a dose recombinant adeno-virus associated (rAAV) rAAV.MHCK7.microdystrophin, wherein the route of administration is intravenous infusion and the dose of the rAAV administered is about 2x10¹⁴ vg/kg over approximately one hour, and wherein the rAAV vector comprises the AAVrh74.MHCK7.micro-dystrophin construct nucleotide sequence of SEQ ID NO: 9 or of nucleotides 55-5021 of SEQ ID NO: 3, nucleotides 1-4977 of SEQ ID NO: 8, or nucleotides 56-5022 of SEQ IDNO: 6. In one embodiment, the rAAV is AAVrh74.MHCK7.microdystrophin. In one embodiment, the AAVrh74.MHCK7.microdystrophin is the AAVrh74.MHCK7.microdystrophin of SEQ ID NO: 9 or of nucleotides 55-5021 of SEQ ID NO: 3, nucleotides 1-4977 of SEQ ID NO: 8, or nucleotides 56-5022 of SEQ IDNO: 6. In one embodiment, the rAAV is AAVrh74.MCK.microdystrophin. In one embodiment, the AAVrh74.MCK.microdystrophin is the AAVrh74.MCK.microdystrophin of nucleotides 56-4820 ofSEQ ID NO: 5.

In one embodiment, the invention provides for a rAAV comprising a muscle specific control element nucleotide sequence, and a nucleotide sequence encoding the micro-dystrophin protein. For example, the nucleotide sequence encodes a functional micro-dystrophin protein, wherein the nucleotide has, e.g., at least 65%, at least 70%, at least 75%, at least 80%, 81%, 82%, 83%, 84%, 85%, 86%, 87%, 88%, or 89%, more typically at least 90%, 91%, 92%, 93%, or 94% and even more typically at least 95%, 96%, 97%, 98%, 99%, or 100% sequence identity to SEQ ID NO: 1, wherein the protein retains micro-dystrophin activity. The micro-dystrophin protein provides stability to the muscle membrane during muscle contraction, e.g. micro-dystrophin acts as a shock absorber during muscle contraction. In one embodiment, the rAAV is AAVrh74.MHCK7.microdystrophin. In one embodiment, the AAVrh74.MHCK7.microdystrophin is the AAVrh74.MHCK7.microdystrophin of SEQ ID NO: 9 or of nucleotides 55-5021 of SEQ ID NO: 3, nucleotides 1-4977 of SEQ ID NO: 8, or nucleotides 56-5022 of SEQ IDNO: 6. In one embodiment, the rAAV is AAVrh74.MCK.microdystrophin. In one embodiment, the AAVrh74.MCK.microdystrophin is the AAVrh74.MCK.microdystrophin of nucleotides 56-4820 of SEQ ID NO: 5.

The invention also provides for rAAV wherein the nucleotide sequence comprises a nucleotide sequence that hybridizes under stringent conditions to the nucleic acid sequence of SEQ ID NO: 1, or compliments thereof, and encodes a functional micro-dystrophin protein.

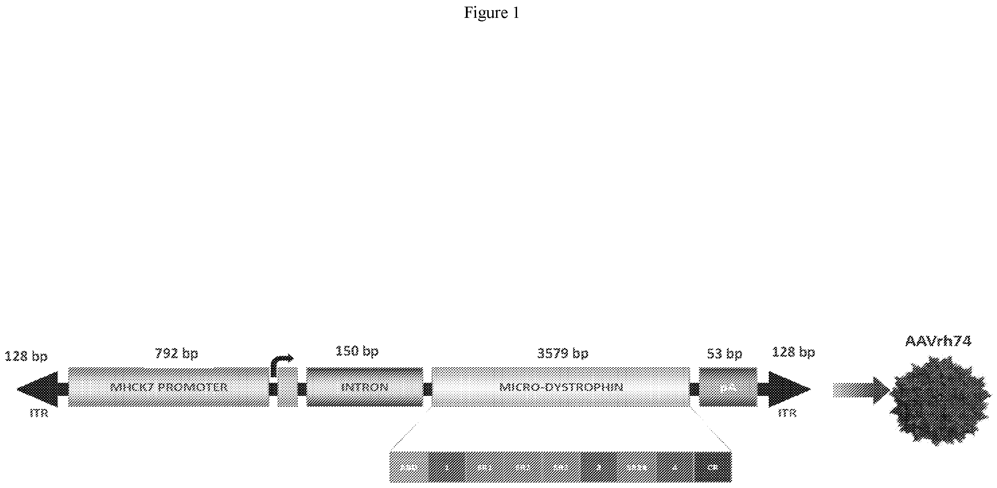

In one embodiment, the rAAV is a non-replicating, recombinant adeno-associated virus (AAV) termed AAVrh74.MHCK7.micro-dystrophin of SEQ ID NO: 9, nucleotides 55-5021 of SEQ ID NO: 3, nucleotides 1-4977 of SEQ ID NO: 8, or nucleotides 56-5022 of SEQ IDNO: 6. This vector genome contains minimal elements required for gene expression, including AAV2 inverted terminal repeats (ITR), the micro-dystrophin, SV40 intron (SD/SA), and synthetic polyadenylation (Poly A) signal, all under the control of the MHCK7 promoter/enhancer. The schematic of the vector genome and expression cassette is shown Figure 1. The AAVrh74 serotype can be employed to achieve efficient gene transfer in skeletal and cardiac muscle following IV administration.

The term "stringent" is used to refer to conditions that are commonly understood in the art as stringent. Hybridization stringency is principally determined by temperature, ionic strength, and the concentration of denaturing agents such as formamide. Examples of stringent conditions for hybridization and washing are 0.015 M sodium chloride, 0.0015 M sodium citrate at 65-68°C or 0.015 M sodium chloride, 0.0015M sodium citrate, and 50% formamide at 42°C. See Sambrook et al., Molecular Cloning: A Laboratory Manual, 2nd Ed., Cold Spring Harbor Laboratory, (Cold Spring Harbor, N.Y. 1989). More stringent conditions (such as higher temperature, lower ionic strength, higher formamide, or other denaturing agent) may also be used, however, the rate of hybridization will be affected. In instances wherein hybridization of deoxyoligonucleotides is concerned, additional exemplary stringent hybridization conditions include washing in 6x SSC 0.05% sodium pyrophosphate at 37°C (for 14-base oligos), 48°C (for 17-base oligos), 55°C (for 20-base oligos), and 60°C (for 23-base oligos).

Other agents may be included in the hybridization and washing buffers for the purpose of reducing non-specific and/or background hybridization. Examples are 0.1% bovine serum albumin, 0.1% polyvinyl-pyrrolidone, 0.1% sodium pyrophosphate, 0.1% sodium dodecylsulfate, NaDodSO4, (SDS), ficoll, Denhardt's solution, sonicated salmon sperm DNA (or other non-complementary DNA), and dextran sulfate, although other suitable agents can also be used. The concentration and types of these additives can be changed without substantially affecting the stringency of the hybridization conditions. Hybridization experiments are usually carried out at pH 6.8-7.4, however, at typical ionic strength conditions, the rate of hybridization is nearly independent of pH. See Anderson et al., Nucleic Acid Hybridisation: A Practical Approach, Ch. 4, IRL Press Limited (Oxford, England). Hybridization conditions can be adjusted by one skilled in the art in order to accommodate these variables and allow DNAs of different sequence relatedness to form hybrids.

The term "muscle specific control element" refers to a nucleotide sequence that regulates expression of a coding sequence that is specific for expression in muscle tissue. These control elements include enhancers and promoters. The invention provides for constructs comprising the muscle specific control elements MCKH7 promoter, the MCK promoter and the MCK enhancer.

The term "operably linked" refers to the positioning of the regulatory element nucleotide sequence, e.g. promoter nucleotide sequence, to confer expression of said nucleotide sequence by said regulatory element.

In one aspect, the invention provides for a rAAV wherein the muscle specific control element is a human skeletal actin gene element, cardiac actin gene element, myocyte-specific enhancer binding factor (MEF), muscle creatine kinase (MCK), truncated MCK (tMCK), myosin heavy chain (MHC), hybrid α-myosin heavy chain enhancer-/MCK enhancer-promoter (MHCK7), C5-12, murine creatine kinase enhancer element, skeletal fast-twitch troponin c gene element, slow-twitch cardiac troponin c gene element, the slow-twitch troponin i gene element, hypoxia-inducible nuclear factors, steroid-inducible element or glucocorticoid response element (GRE).

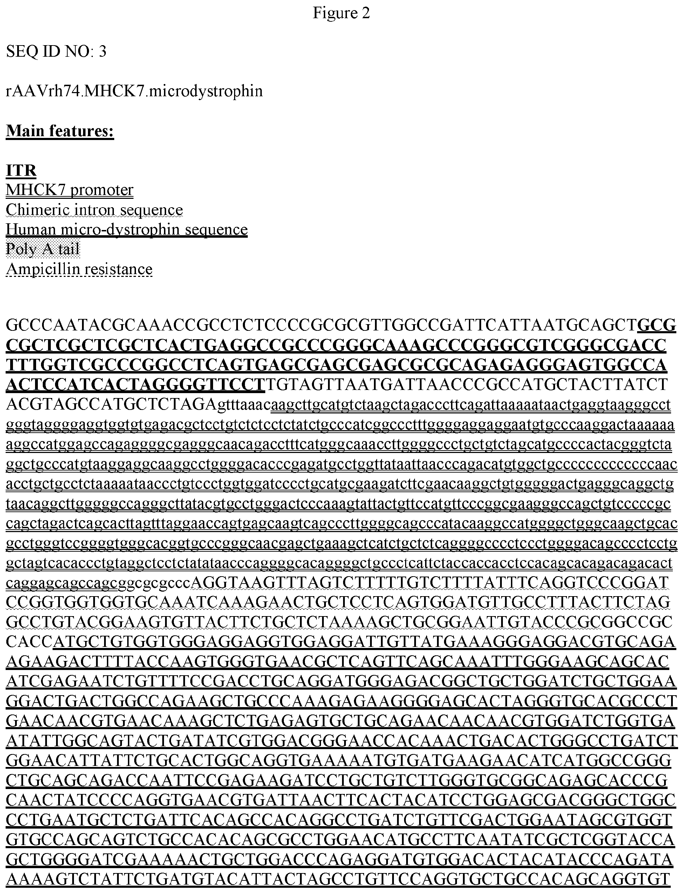

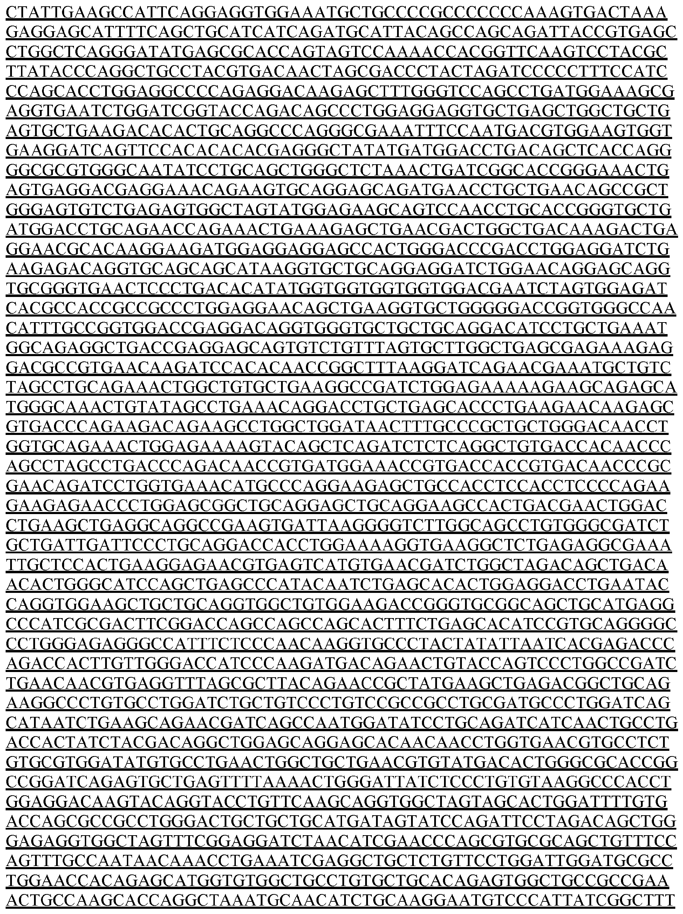

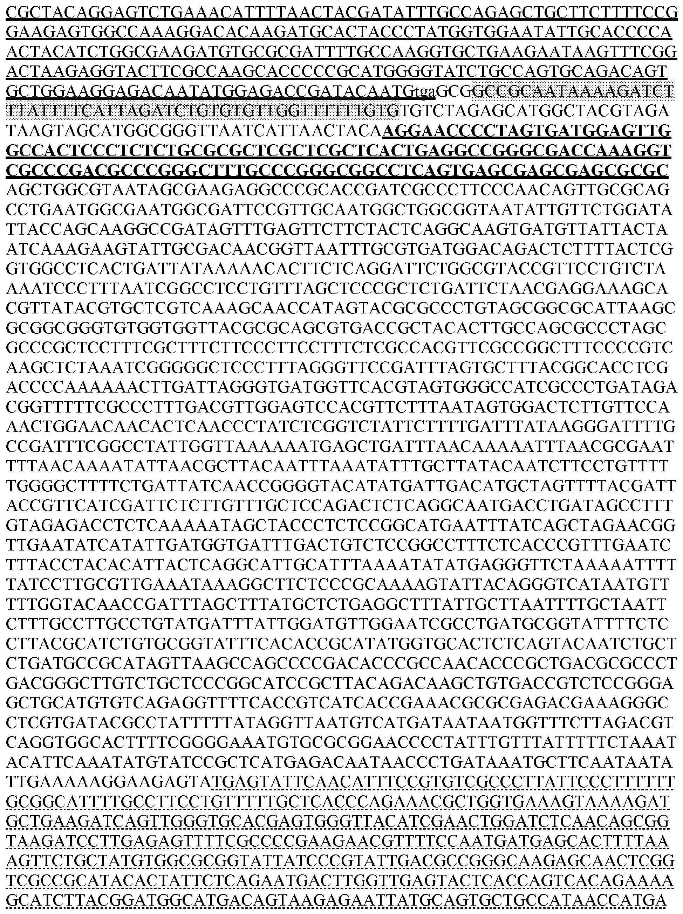

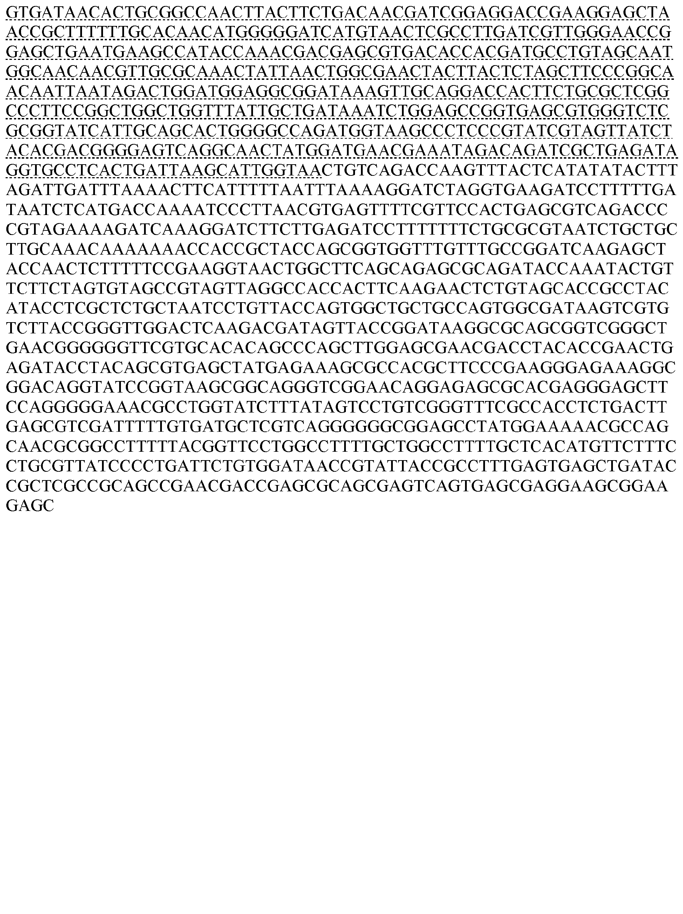

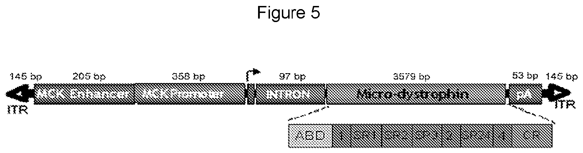

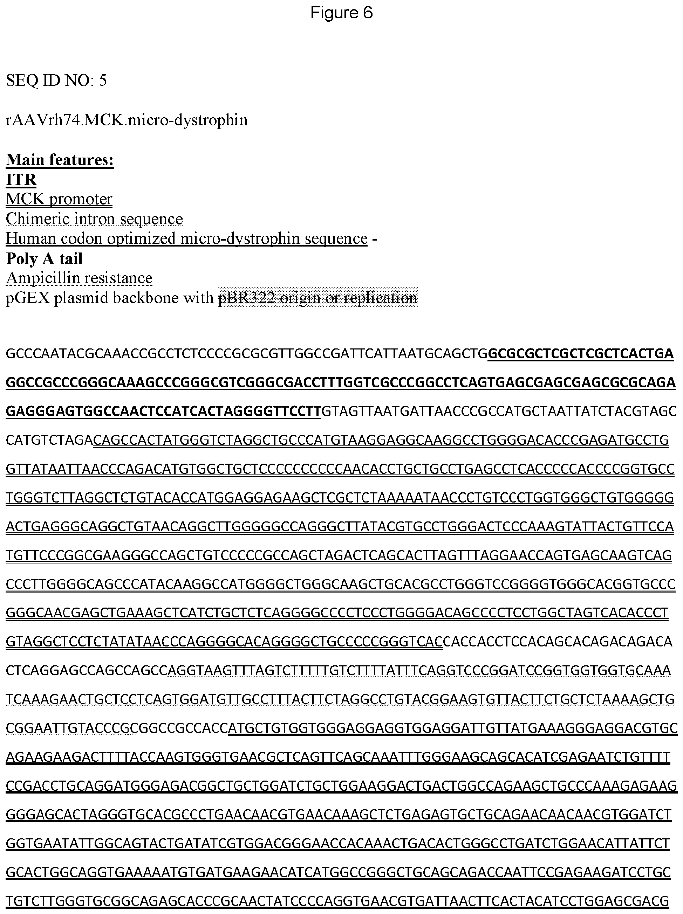

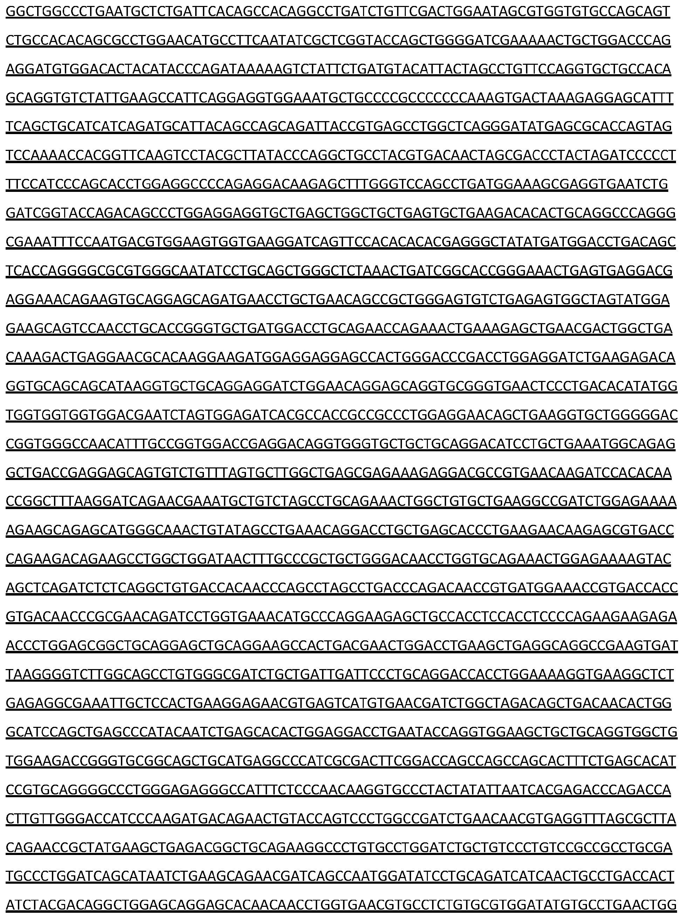

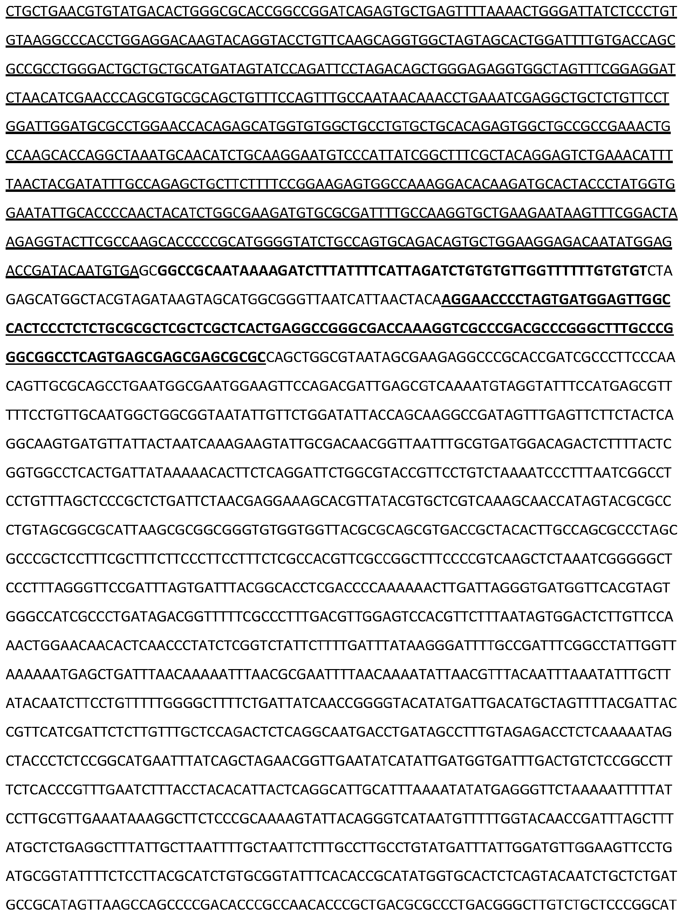

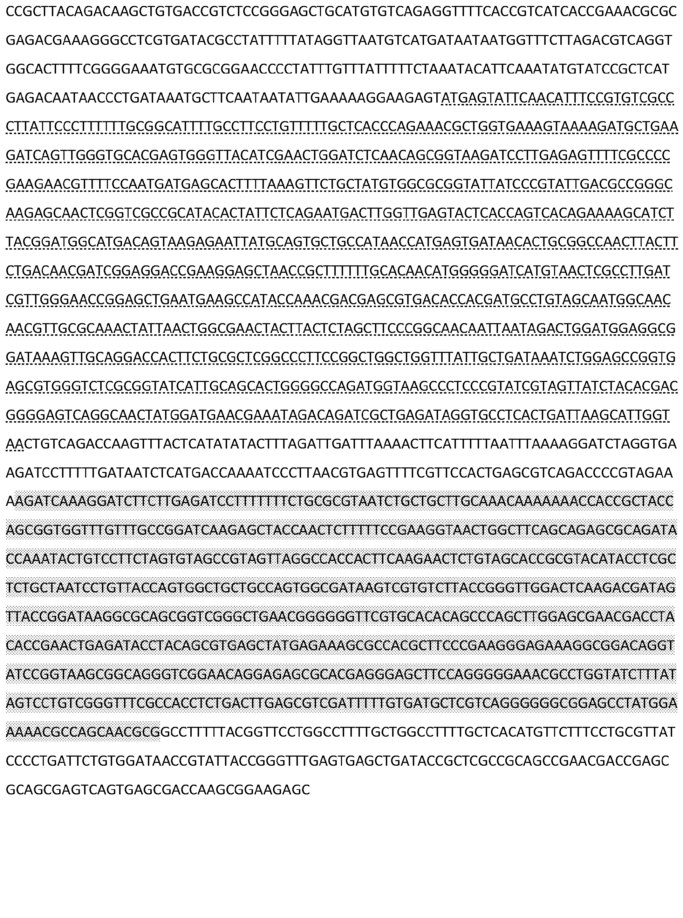

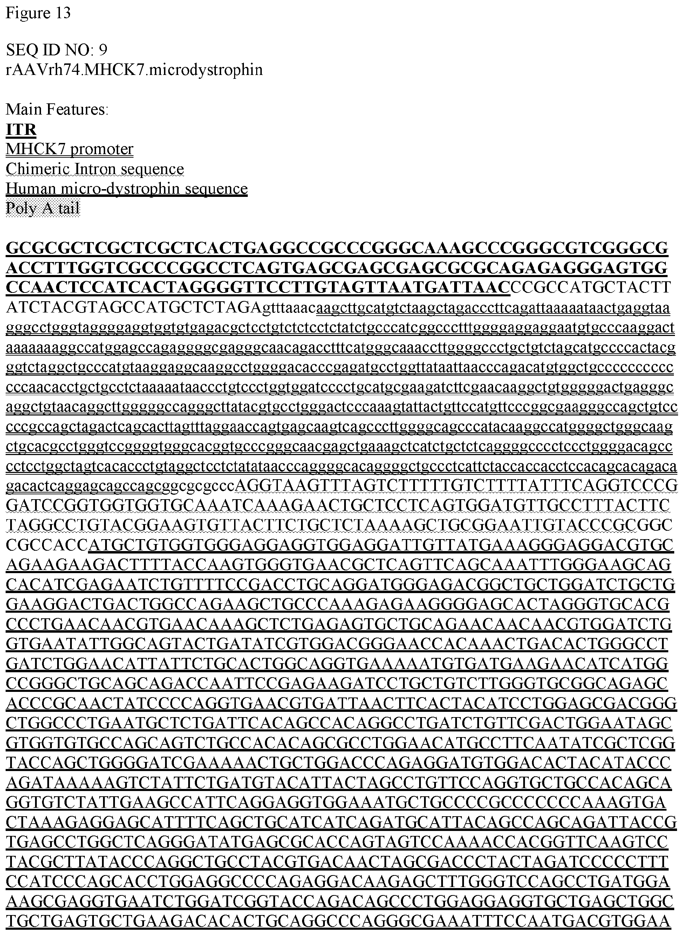

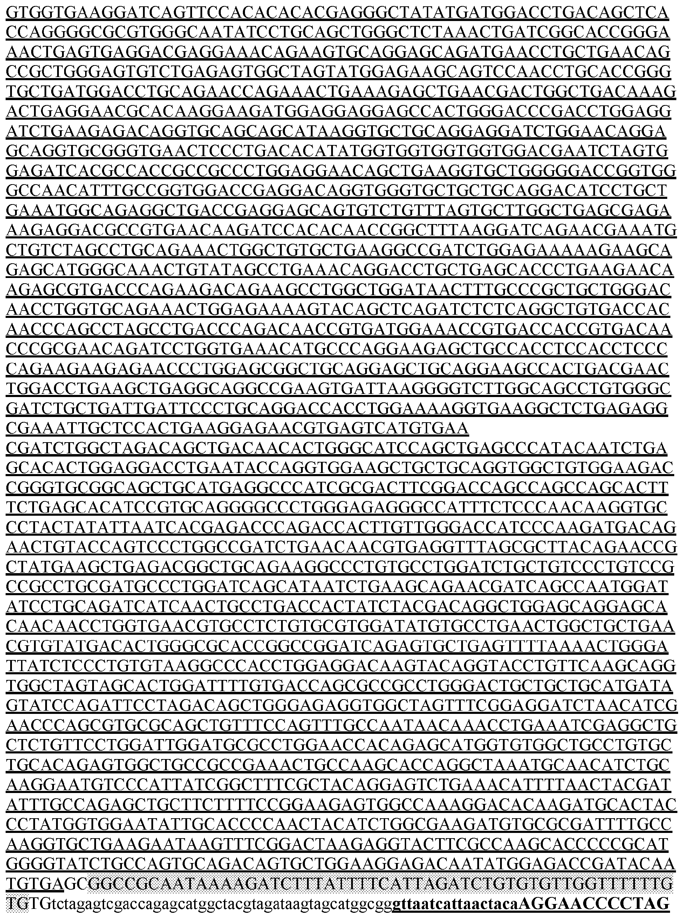

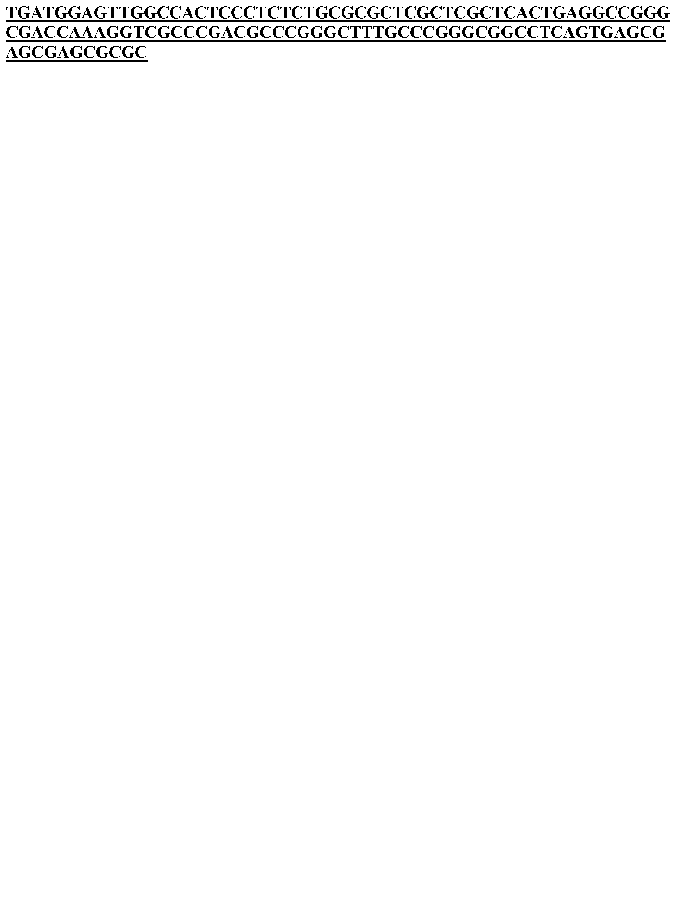

For example, the muscle specific control element is the MHCK7 promoter nucleotide sequence SEQ ID NO: 2 or SEQ ID NO: 7, or the muscle specific control element is MCK nucleotide sequence SEQ ID NO: 4. In addition, in any of the rAAV vectors of the invention, the muscle specific control element nucleotide sequence, e.g. MHCK7 or MCK nucleotide sequence, is operably linked to the nucleotide sequence encoding the micro-dystrophin protein. For example, the MHCK7 promoter nucleotide sequence (SEQ ID NO: 2 or SEQ ID NO: 7) is operably linked to the human micro-dystrophin coding sequence (SEQ ID NO: 1) as set out in the construct provided in Figure 1 or Figure 2 (SEQ ID NO: 3) or Figure 13 (SEQ ID NO: 9). In another example, the MCK promoter (SEQ ID NO: 4) is operably linked to the human micro-dystrophin coding sequence (SEQ ID NO: 1) as set out in the construct provided in Figure 5 or Figure 6 (SEQ ID NO: 5). In another aspect, the invention provides for a rAAV vector comprising the nucleotide sequence of SEQ ID NO: 1 and SEQ ID NO: 2, or SEQ ID NO: 1 and SEQ ID NO: 7. The invention also provides for a rAAV vector comprising the nucleotide sequence of SEQ ID NO: 1 and SEQ ID NO: 4.

In a further aspect, the invention provides for an rAAV construct contained in the plasmid comprising the nucleotide sequence of SEQ ID NO: 3, SEQ ID NO: 5, SEQ ID NO: 6, or SEQ ID NO: 8, . For example, the AAVrh74.MHCK7.microdystrophin vector comprises the nucleotide sequence within and inclusive of the ITR's of SEQ ID NO: 3 and shown in Figure 2. The rAAV vector comprises the 5' ITR, MHCK7 promoter, a chimeric intron sequence, the coding sequence for the human micro-dystrophin gene, polyA, and 3' ITR. In one embodiment, the vector comprises nucleotides 55-5021 of SEQ ID NO:3. The plasmid set forth in SEQ ID NO:3 further comprises ampicillin resistance and the pGEX plasmid backbone with pBR322 origin of replication.

In another aspect, the invention provides for a rAAV comprising the nucleotide sequence of SEQ ID NO: 9. For example, the AAVrh74.MHCK7.microdystrophin vector construct comprises the nucleotide sequence of SEQ ID NO: 9 and shown in Figure 13. This rAAV vector construct comprises the MHCK7 promoter, a chimeric intron sequence, the coding sequence for the human micro-dystrophin gene, and polyA. In one embodiment, the rAAV vector construct further comprises an ITR 5' to the promoter, and an ITR 3' to the polyA. In one embodiment, the rAAV is AAVrh74.

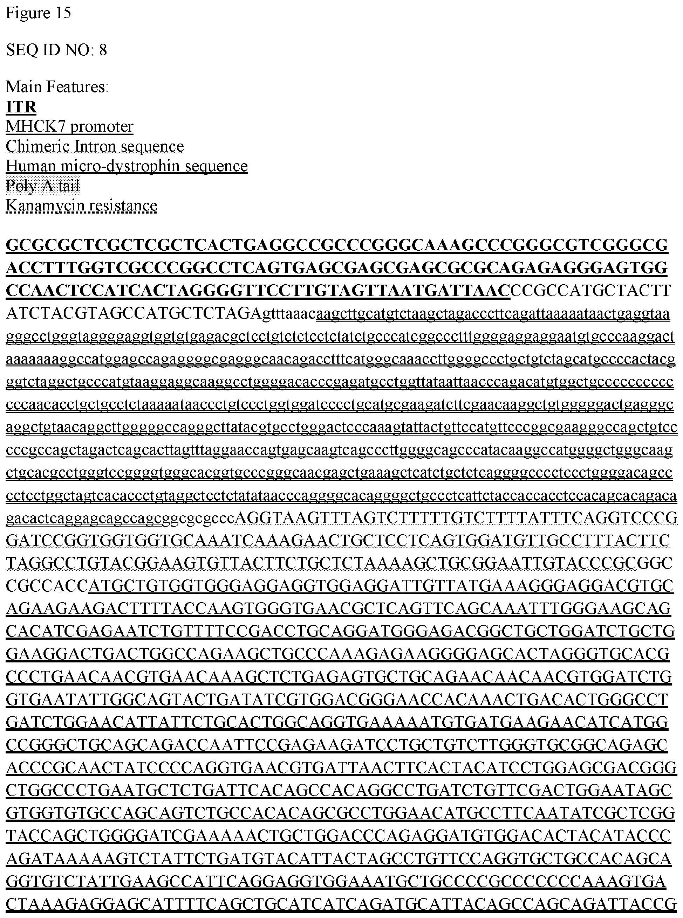

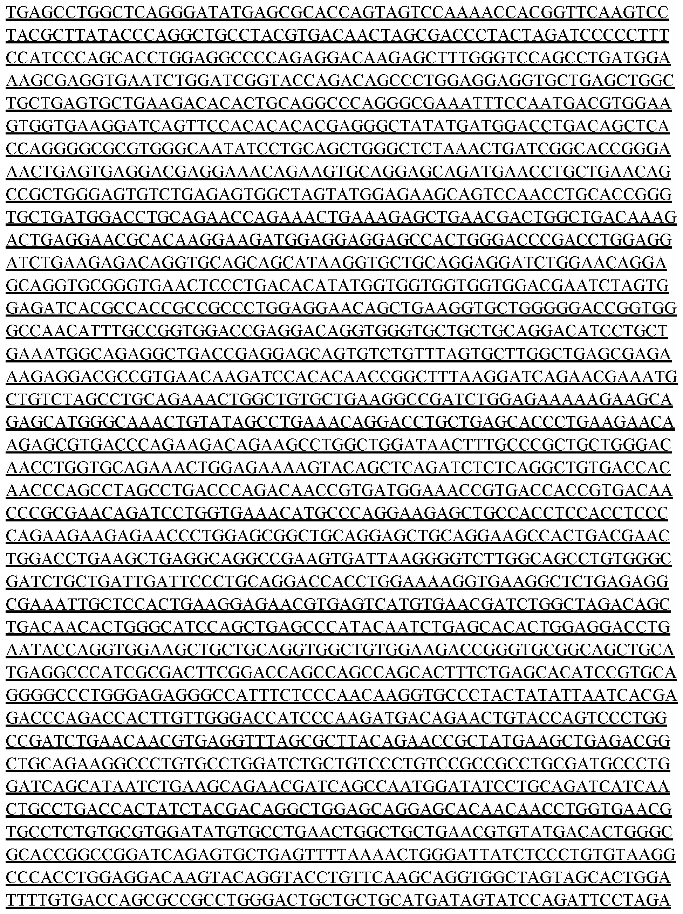

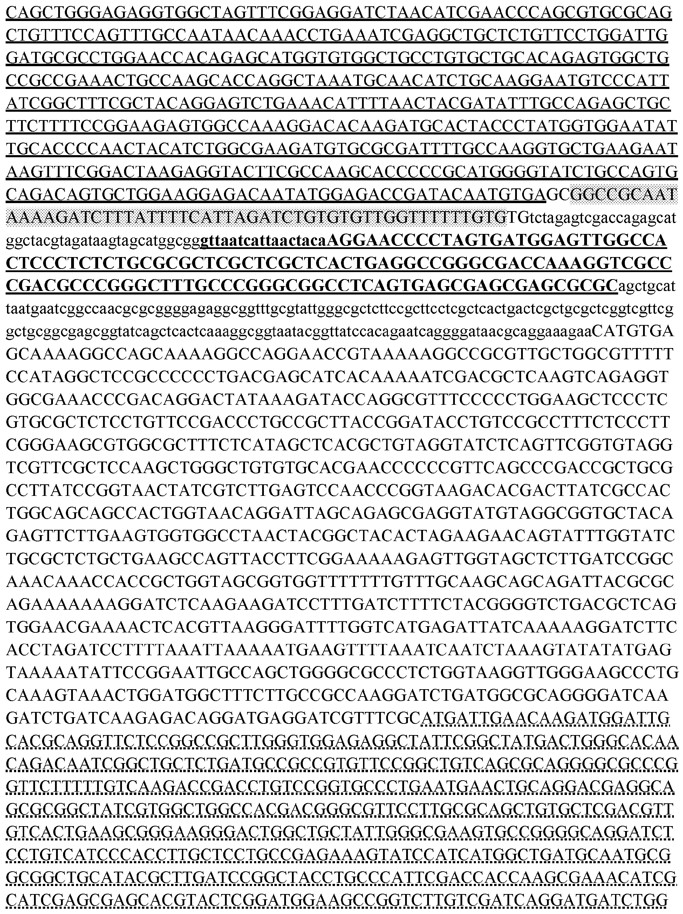

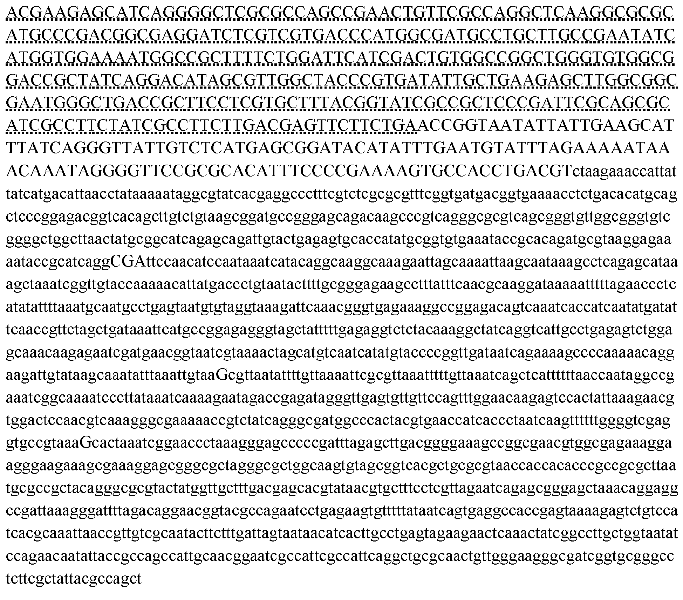

In another aspect, the AAVrh74.MHCK7.microdystrophin vector comprises the nucleotide sequence within and inclusive of the ITR's of SEQ ID NO: 8 and shown in Figure 15. The rAAV vector comprises the 5' ITR, MHCK7 promoter, a chimeric intron sequence, the coding sequence for the human micro-dystrophin gene, polyA, and 3' ITR. In one embodiment, the vector comprises nucleotides 1-4977 of SEQ ID NO:9. The plasmid set forth in SEQ ID NO:3 further comprises kanamycin resistance and the pGEX plasmid backbone with pBR322 origin of replication.

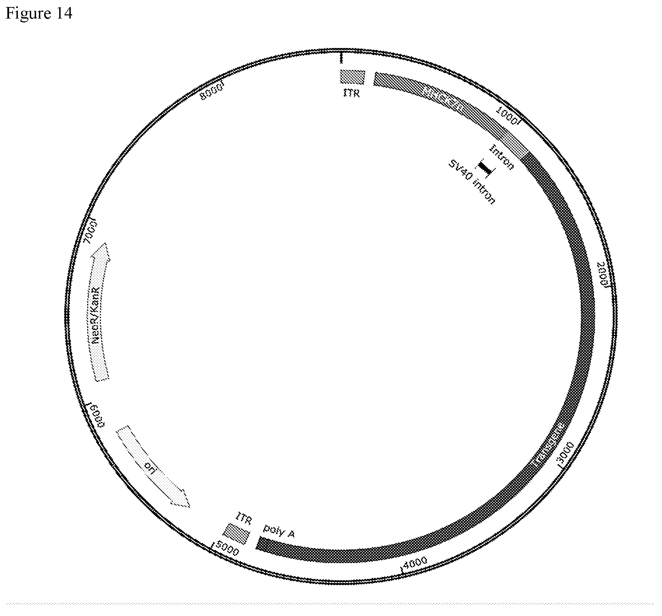

In another aspect, the invention provides for a plasmid comprising the AAVrh74.MHCK7.microdystrophin vector construct. In one embodiment, the plasmid comprises the 5' ITR, MHCK7 promoter, a chimeric intron sequence, the coding sequence for the human micro-dystrophin gene, polyA, and 3' ITR. In one embodiment, the plasmid comprises kanamycin resistance and optionally comprises the pGEX plasmid backbone with pBR322 origin of replication. In a particular embodiment, the plasmid is set forth in SEQ ID NO:8, and shown in Figures 14 and 15.

The invention provides for a recombinant AAV vector comprising the human micro-dystrophin nucleotide sequence of SEQ ID NO: 1 and the MHCK7 promoter nucleotide sequence of SEQ ID NO: 2 or SEQ ID NO:7. This rAAV vector is the AAV serotype AAVrh.74.

The invention also provides for a rAAV comprising the AAVrh74.MHCK7.micro-dystrophin construct nucleotide sequence within and inclusive of the ITR's in SEQ ID NO: 3, the nucleotide sequence within and inclusive of the ITR's in SEQ ID NO: 8 or the nucleotide sequence as set forth in SEQ ID NO: 9. This rAAV vector is the AAV serotype AAVrh.74.

The rAAV vectors of the invention may be any AAV serotype, such as the serotype AAVrh.74, AAV1, AAV2, AAV4, AAV5, AAV6, AAV7, AAV8, AAV9, AAV10, AAV11, AAV12 or AAV13.

The invention also provides for pharmaceutical compositions (or sometimes referred to herein as simply "compositions") comprising any of the rAAV vectors of the invention.

In another embodiment, the invention provides for methods of producing a rAAV vector particle comprising culturing a cell that has been transfected with any rAAV vector of the invention and recovering rAAV particles from the supernatant of the transfected cells. The invention also provides for viral particles comprising any of the recombinant AAV vectors of the invention.

In any of the methods of treating a muscular dystrophy, the level of micro-dystrophin gene expression in a cell of the subject is increased after administration of the rAAV. Expression of the micro-dystrophin gene in the cell is detected by measuring the micro-dystrophin protein level by Western blot in muscle biopsied before and after administration of the rAAV. In particular, the level of micro-dystrophin protein is increased by at least about 70% to at least about 80%, or at least about 70% to at least about 90%, or at least about 80% to at least about 90% after administration of rAAV compared to the level of micro-dystrophin before administration of rAAV. For example, the level of micro-dystrophin protein is increased by at least about 70% or at least about 71% or at least about 72% or at least about 73% or at least about 74% or at least about 75% or at least about 76% or at least about 77% or at least about 78% or at least about 79% or at least about 80%, or at least about 81%, or at least about 82%, or at least about 83%, or at least about 84%, or at least about 85% after administration of rAAV compared to the level of micro-dystrophin before administration of rAAV.

In addition, expression of the micro-dystrophin gene in the cell is detected by measuring the micro-dystrophin protein level by immunohistochemistry in muscle biopsies before and after administration of the rAAV. The level of micro-dystrophin protein is increased by at least about 70% to at least about 80%, or at least about 70% to at least about 90%, or at least about 80% to at least about 90% after administration of rAAV compared to the level of micro-dystrophin before administration of rAAV. For example, the level of micro-dystrophin protein is increased by at least about 70% or at least about 71% or at least about 72% or at least about 73% or at least about 74% or at least about 75% or at least about 76% or at least about 77% or at least about 78% or at least about 79% or at least about 80%, or at least about 81%, or at least about 82%, or at least about 83%, or at least about 84%, or at least about 85% after administration of rAAV compared to the level of micro-dystrophin before administration of rAAV.

In any of the methods of treating a muscular dystrophy, the serum CK level in the subject is decreased after administration of the rAAV as compared to serum CK level before administration of the rAAV. For example, the serum CK level in the subject is decreased by about 65 % to about 90% or about 65% to about 95% or about 75% to about 90% or about 80% to about 90% or about 85% to about 95% or about 87% to about 95% or about 87% to about 90% by 60 days after administration of the rAAV as compared to the serum CK level before administration of the rAAV. In particular, in any of the methods of treating a muscular dystrophy of the invention, the serum CK level in the subject is decreased by about 87% by 60 days after administration of the rAAV as compared to the serum CK level before administration of the rAAV or in any of the methods of treating a muscular dystrophy of the invention, the serum CK level in the subject is decreased by about 72% by 60 days after administration of the rAAV as compared to the serum CK level before administration of the rAAV, or in any of the methods of treating a muscular dystrophy of the invention, the serum CK level in the subject is decreased by about 73% by 60 days after administration of the rAAV as compared to the serum CK level before administration of the rAAV, or in any of the methods of treating a muscular dystrophy of the invention, the serum CK level in the subject is decreased by about 78% by 60 days after administration of the rAAV as compared to the serum CK level before administration of the rAAV. or in any of the methods of treating a muscular dystrophy of the invention, the serum CK level in the subject is decreased by about 95% by 60 days after administration of the rAAV as compared to the serum CK level before administration of the rAAV. In any of the methods of treating a muscular dystrophy, the number of micro-dystrophin positive fibers in the muscle tissue of the subject is increased after administration of the rAAV as compared to the number of micro-dystrophin positive fibers before administration of the rAAV. For example, the number of micro-dystrophin positive fibers is detected by measuring the micro-dystrophin protein level by Western blot or immunohistochemistry on muscle biopsies before and after administration of the rAAV.

In any of the methods of treating a muscular dystrophy, administration of the rAAV upregulates expression of DAPC proteins such as alpha-sarcoglycan or beta-sarcoglycan. For example, the level of alpha-sarcoglycan in the subject is increased after administration of the rAAV as compared to the level of alpha-sarcoglycan before administration of the rAAV. In addition, the level of beta-sarcoglycan in the subject is increased after administration of the rAAV as compared to the level of the beta-sarcoglycan before administration of the rAAV. The level of alpha-sarcoglycan or beta-sarcoglycan is detected by measuring the alpha-sarcoglycan or beta-sarcoglycan protein level by Western blot or immunohistochemistry on muscle biopsies before and after administration of the rAAV.

In any of the methods of the treating a muscular dystrophy, disease progression in the subject is delayed after administration of the rAAV as measured by any of: the six minute walk test, time to rise, ascend 4 steps, ascend and descend 4 steps, North Star Ambulatory Assessment (NSAA), 10 meter timed test, 100 meter timed test, hand held dynamometry (HHD), Timed Up and Go, and/or Gross Motor Subtest Scaled (Bayley-III) score.

For example, in any of the methods, the subject has at least a 6-point improvement in NSAA score at least 270 days after administration of the rAAV as compared to NSAA score before administration of the rAAV. Further, in any of the methods, the subject has at least about 0.8 second improvement in time to rise at least 270 days after administration of the rAAV as compared to time to rise before administration of the rAAV. In addition, in any of the methods, the subject has at least about 1.2 second improvement in time to ascend 4 steps test at least 270 days after administration of the rAAV as compared to time to ascend 4 steps test before administration of the rAAV. In addition, in any of the methods, the subject has at least about 7 second improvement in the 100 m timed test at least 270 days after administration of the rAAV as compared to the 100 m timed test before administration of the rAAV.

In another embodiment, the invention provides for methods of expressing micro-dystrophin gene in a patient cell comprising administering to the patient the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8 or nucleotides 56-5022 of SEQ ID NO: 6. For example, expression of the micro-dystrophin gene in the patient cell is detected by measuring the micro-dystrophin protein level by Western blot or immunohistochemistry in muscle biopsies before and after administration of the rAAV.MHCK7.micro-dystrophin construct. In addition, the expression of the micro-dystrophin gene is measured in the patient by detecting greater the number of vector genomes per nucleus, wherein 1vector genome per nucleus is about 50% micro-dystrophin expression and great than 1 copy per nucleus is consistent with micro-dystrophin expression level. For example, the cells have 1.2 vector copies per nucleus, or 1.3 vector copies per nucleus, or 1.4 vector copies per nucleus, or 1.5 vector copies per nucleus, or 1.6 vector copies per nucleus, or 1.7 vector copies per nucleus, or 1.8 vector copies per nucleus, or 1.9 vector copies per nucleus.

In a further embodiment, the invention provides for methods of decreasing serum CK levels in a patient in need thereof, the method comprising administering to the patient the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. For example, the serum CK level in the patient is decreased by at least about 65% to about 90% or about 65% to about 95% or about 75% to about 90% or about 80% to about 90% or about 85% to about 95% or about 87% to about 95% or about 87% to about 90% by 60 days after administration of the rAAV as compared to the serum CK level before administration of the rAAV. In particular, the serum CK level in the subject is decreased by about 87% by 60 days after administration of the rAAV as compared to the serum CK level before administration of the rAAV or in any of the methods of treating a muscular dystrophy of the invention, the serum CK level in the subject is decreased by about 72% by 60 days after administration of the rAAV as compared to the serum CK level before administration of the rAAV, or in any of the methods of treating a muscular dystrophy of the invention, the serum CK level in the subject is decreased by about 73% by 60 days after administration of the rAAV as compared to the serum CK level before administration of the rAAV, or in any of the methods of treating a muscular dystrophy of the invention, the serum CK level in the subject is decreased by about 78% by 60 days after administration of the rAAV as compared to the serum CK level before administration of the rAAV, or in any of the methods of treating a muscular dystrophy of the invention, the serum CK level in the subject is decreased by about 95% by 60 days after administration of the rAAV as compared to the serum CK level before administration of the rAAV.

The invention also provides for methods of increasing micro-dystrophin positive fibers in a patient muscle tissue comprising administering to the patient the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. For example, the number of micro-dystrophin positive fibers is detected by measuring the dystrophin protein level by Western blot or immunohistochemistry on muscle biopsies before and after administration of the rAAV. In addition, the expression of the micro-dystrophin gene is measured in the patient by detecting greater the number of vector genomes per nucleus, wherein 1vector genome per nucleus is about 50% micro-dystrophin expression and great than 1 copy per nucleus is consistent with micro-dystrophin expression level. For example, the cells have 1.2 vector copies per nucleus, or 1.3 vector copies per nucleus, or 1.4 vector copies per nucleus, or 1.5 vector copies per nucleus, or 1.6 vector copies per nucleus, or 1.7 vector copies per nucleus, or 1.8 vector copies per nucleus, or 1.9 vector copies per nucleus.

In another embodiment, the invention provides for methods of increasing the expression of alpha-sarcoglycan in a patient in need thereof comprising administering to the patient the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. For example, the level of alpha-sarcoglycan is detected by measuring the alpha-sarcoglycan protein level by Western blot or immunohistochemistry on muscle biopsies before and after administration of the rAAV.

In addition, the invention provides for methods of increasing the expression of beta-sarcoglycan in a patient in need thereof comprising administering to the patient the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. For example, the level of beta-sarcoglycan is detected by measuring the beta-sarcoglycan protein level by Western blot or immunohistochemistry on muscle biopsies before and after administration of the rAAV.

The invention also provides for methods of treating a patient with Duchenne muscular dystrophy or Becker muscular dystrophy comprising administering to the patient the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6, such that disease progression in the patient is delayed as measured by any of: the six minute walk test, time to rise, ascend 4 steps, ascend and descend 4 steps, North Star Ambulatory Assessment (NSAA), 10 meter timed test, 100 meter timed test, hand held dynamometry (HHD), Timed Up and Go, and/or Gross Motor Subtest Scaled (Bayley-III) score.

For example, in any of the methods, the subject has at least a 6-point improvement in NSAA score at least 270 days after administration of the rAAV as compared to NSAA score before administration of the rAAV. Further, in any of the methods, the subject has at least about 0.8 second improvement in time to rise at least 270 days after administration of the rAAV as compared to time to rise before administration of the rAAV. In addition, in any of the methods, the subject has at least about 1.2 second improvement in time to ascend 4 steps test at least 270 days after administration of the rAAV as compared to time to ascend 4 steps test before administration of the rAAV. In addition, in any of the methods, the subject has at least about 7 second improvement in the 100 m timed test at least 270 days after administration of the rAAV as compared to the 100 m timed test before administration of the rAAV.

"Fibrosis" refers to the excessive or unregulated deposition of extracellular matrix (ECM) components and abnormal repair processes in tissues upon injury, including skeletal muscle, cardiac muscle, liver, lung, kidney, and pancreas. The ECM components that are deposited include fibronectin and collagen, e.g. collagen 1, collagen 2 or collagen 3.

The invention also provides for methods of reducing or preventing fibrosis in a subject suffering from muscular dystrophy comprising administering a therapeutically effective amount of a rAAV comprising the human micro-dystrophin nucleotide sequence of SEQ ID NO: 1 and the MHCK7 promoter nucleotide sequence of SEQ ID NO: 2 or SEQ ID NO: 7; or a rAAV vector comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. In one embodiment, the rAAV is AAVrh74.MHCK7.microdystrophin. In one embodiment, the AAVrh74.MHCK7.microdystrophin is the AAVrh74.MHCK7.microdystrophin of nucleotides 55-5021 of SEQ ID NO: 3. In another embodiment, the AAVrh74.MHCK7.microdystrophin is the AAVrh74.MHCK7.microdystrophin of SEQ ID NO: 9. In another embodiment, the AAVrh74.MHCK7.microdystrophin is the AAVrh74.MHCK7.microdystrophin of nucleotides 1-4977 of SEQ ID NO: 8 or nucleotides 56-5066 of SEQ ID NO: 6. In a further embodiment, the rAAV is AAVrh74.MCK.microdystrophin. In one embodiment, the AAVrh74.MCK.microdystrophin is the AAVrh74.MCK.microdystrophin of nucleotides 56-4820 of SEQ ID NO: 5.

In another embodiment, the invention provides for methods of preventing fibrosis in a subject in need thereof, comprising administering a therapeutically effective amount of the human micro-dystrophin nucleotide sequence of SEQ ID NO: 1 and the MHCK7 promoter nucleotide sequence of SEQ ID NO: 2 or SEQ ID NO:7; or rAAV vector comprising the AAV74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. For example, any of the rAAV of the invention can be administered to subjects suffering from muscular dystrophy to prevent fibrosis, e.g. the rAAV of the invention expressing a human micro-dystrophin protein administered before fibrosis is observed in the subject. In addition, the rAAV of the invention expressing a human micro-dystrophin gene can be administered to a subject at risk of developing fibrosis, such as those suffering or diagnosed with muscular dystrophy, e.g. DMD. The rAAV of the invention can be administered to the subject suffering from muscular dystrophy in order to prevent new fibrosis in these subjects.

The invention contemplates administering rAAV before fibrosis is observed in the subject. In addition, the rAAV can be administered to a subject at risk of developing fibrosis, such as those suffering or diagnosed with a muscular dystrophy, e.g. DMD. The rAAV can be administered to the subject suffering from muscular dystrophy who already has developed fibrosis in order to prevent new fibrosis in these subjects.

The invention also provides for methods of increasing muscular force and/or muscle mass in a subject suffering from a muscular dystrophy comprising administering a therapeutically effective amount of the human micro-dystrophin nucleotide sequence of SEQ ID NO: 1 and the MHCK7 promoter nucleotide sequence of SEQ ID NO: 2 or SEQ ID NO: 7; or a rAAV comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6.

The invention contemplates administering rAAV vectors to subjects diagnosed with DMD before fibrosis is observed in the subject or before the muscle force has been reduced or before the muscle mass has been reduced.

The invention also contemplates administering the human micro-dystrophin nucleotide sequence of SEQ ID NO: 1 and the MHCK7 promoter nucleotide sequence of SEQ ID NO: 2 or SEQ ID NO:7; or a rAAV comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 to a subject suffering from a muscular dystrophy who already has developed fibrosis, in order to prevent new fibrosis in these subjects or to reduce fibrosis in these subjects. The invention also provides for administering the human micro-dystrophin nucleotide sequence of SEQ ID NO: 1 and the MHCK7 promoter nucleotide sequence of SEQ ID NO: 2 or SEQ ID NO:7; or a rAAV vector comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 to the subject suffering from a muscular dystrophy who already has reduced muscle force or has reduced muscle mass in order to protect the muscle from further injury.

In any of the methods of the invention, the subject may be suffering from a muscular dystrophy such as DMD or any other dystrophin-associated muscular dystrophy.

In other embodiments of any of the methods of the invention described herein, the serum CK level in the subject is decreased after administration of the rAAV as compared to the serum CK level before administration of the rAAV by a percentage level selected from the group consisting of:
a) at least 78% by 90, 180, or 270 days after the administration;
b) at least 46, 55, 56, 57, 58, 59, 60, 61, 62, 63, 64, 65, 66, 67, 68, 69, 70 or 85% by 270 days after the administration;
c) at least 72, 73, 74, or 95% by 180 days after the administration;
d) at least 87, 88, 93 or 95% by 90 days after the administration;
e) at least 70 % by 270 days after the administration;
f) 70 to 95% by 90, 180, or 270 days after the administration;
g) at least 56, 57, 58, 59, 60, 61, 62, 63, 64, 65, 66, 67, 68, 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration; and
h) at least 56, 57, 58, 59, 60, 61, 62, 63, 64, 65, 66, 67, 68, 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration.

In another embodiment, the invention provides for compositions for treating a muscular dystrophy in a human subject in need, wherein the composition comprises a dose of recombinant adeno-virus associated (rAAV) rAAV.MHCK7.microdystrophin, wherein composition is formulated for a systemic route of administration and the dose of the rAAV is about 1x10¹⁴ vg/kg to about 4x 10¹⁴ vg/kg. In one embodiment, the rAAV is AAVrh74.MHCK7.microdystrophin. In one embodiment, the AAVrh74.MHCK7.microdystrophin is the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ IDNO: 6. In one embodiment, the rAAV is AAVrh74.MCK.microdystrophin. In one embodiment, the AAVrh74.MCK.microdystrophin is the AAVrh74.MCK.microdystrophin of nucleotides 56-4820 of SEQ ID NO: 5.

For example, the composition of the invention comprises a dose of rAAV of about 5.0x10¹² vg/kg to about 1.0x10¹⁴ vg/kg, or about 5.0x10¹² vg/kg to1.0x10¹⁴ vg/kg, or about 5.0x10¹² vg/kg to about 2.0x10¹⁴ vg/kg, or about 5.0x10¹² vg/kg to about 1.0x10¹⁴ vg/kg, or about 5.0x10¹² vg/kg to about 5.0x10¹³ vg/kg, or about 5.0x10¹² vg/kg to about 2.0x10¹³ vg/kg, or about 5.0x10¹² vg/kg to about 1.0x10¹³ vg/kg, or 1.0x10¹⁴ vg/kg to about 1.0x10¹⁵ vg/kg, or 1.0x10¹³ vg/kg to about 1.0x10¹⁴ vg/kg, or about 1.0x10¹³ vg/kg to1.0x10¹⁴ vg/kg, or about 1.0x10¹³ vg/kg to about 2.0x10¹⁴ vg/kg, or about 1.0x10¹³ vg/kg to about 1.0x10¹⁴ vg/kg, or about 1.0x10¹³ vg/kg to about 5.0x10¹³ vg/kg, or about 1.0x10¹³ vg/kg to about 3.0x10¹⁴vg/kg, or about 1.0x10¹³ vg/kg to about 5.0x10¹⁴ vg/kg, or about 1.0x10¹³ vg/kg to about 6.0x10¹⁴ vg/kg, or 1.0x10¹³ vg/kg to about 1.0x10¹⁵ vg/kg, or 5.0x10¹³ vg/kg to about 1.0x10¹⁴ vg/kg, or about 5.0x10¹³ vg/kg to1.0x10¹⁴ vg/kg, or about 5.0x10¹³ vg/kg to about 2.0x10¹⁴ vg/kg, or about 5.0x10¹³ vg/kg to about 1.0x10¹⁴vg/kg, or about 5.0x10¹³ vg/kg to about 3.0x10¹⁴ vg/kg, or about 5.0x10¹³ vg/kg to about 5.0x10¹⁴ vg/kg, or about 5.0x10¹³ vg/kg to about 6.0x10¹⁴ vg/kg, or 5.0x10¹³ vg/kg to about 1.0x10¹⁵ vg/kg, or 1.0x10¹⁴ vg/kg to about 6.0x10¹⁴ vg/kg, or 1.0x10¹⁴ vg/kg to about 5.0x10¹⁴ vg/kg, or 1.0x10¹⁴ vg/kg to about 4.0x10¹⁴ vg/kg, or 1.0x10¹⁴ vg/kg to about 1.0x10¹⁵ vg/kg, or 1.0x10¹⁴ vg/kg to about 3.0x10¹⁴ vg/kg, or about 1.0x10¹⁴ vg/kg to about 2.5x10¹⁴ vg/kg, or 1.0x10¹⁴ vg/kg to about 2.0x10¹⁴ vg/kg, or about 1.25x10¹⁴ vg/kg to about 3.75x10¹⁴ vg/kg, or about 1.25x10¹⁴ vg/kg to 6.0x10¹⁴, or about 1.25x10¹⁴ vg/kg to 5.0x10¹⁴, or about 1.25x10¹⁴ vg/kg to 4.0x10¹⁴, or about 1.25x10¹⁴ vg/kg to 1.0x10¹⁵, or about 1.25x10¹⁴ vg/kg to about 3.5 x10¹⁴ vg/kg, or about 1.25x10¹⁴ vg/kg to about 3.0x10¹⁴ vg/kg, or about 1.25x10¹⁴ vg/kg to about 2.75x10¹⁴ vg/kg, or about 1.25x10¹⁴ vg/kg to about 2.5x10¹⁴ vg/kg, or about 1.25x10¹⁴ vg/kg to about 2.0x10¹⁴ vg/kg, or 1.25x10¹⁴ vg/kg to about 3.75x10¹⁴ vg/kg, or about 1.25x10¹⁴ vg/kg to about 3.5 x10¹⁴ vg/kg, or 1.5x10¹⁴ vg/kg to about 1.0x10¹⁵ vg/kg, or about 1.5x10¹⁴ vg/kg to 6.0x10¹⁴, or about 1.5x10¹⁴ vg/kg to 5.0x10¹⁴, or about 1.5x10¹⁴ vg/kg to 4.0x10¹⁴, or about 1.5x10¹⁴ vg/kg to about 3.75x10¹⁴ vg/kg, or about 1.5x10¹⁴ vg/kg to about 3.5x10¹⁴ vg/kg, or about 1.5x10¹⁴ vg/kg to about 3.25x10¹⁴ vg/kg, or about 1.5x10¹⁴ vg/kg to about 3.0x10¹⁴ vg/kg, or about 1.5x10¹⁴ vg/kg to about 2.75x10¹⁴ vg/kg, or about 1.5x10¹⁴ vg/kg to about 2.5x10¹⁴ vg/kg, or about 1.5x10¹⁴ vg/kg to about 2.0x10¹⁴ vg/kg, or 1.75x10¹⁴ vg/kg to about 1.0x10¹⁵ vg/kg, or about 1.75x10¹⁴ vg/kg to 6.0x10¹⁴, or about 1.75x10¹⁴ vg/kg to 5.0x10¹⁴, or about 1.75x10¹⁴ vg/kg to 4.0x10¹⁴, or about 1.75x10¹⁴ vg/kg to about 3.75x10¹⁴ vg/kg, or about 1.75x10¹⁴ vg/kg to about 3.5x10¹⁴ vg/kg, or about 1.75x10¹⁴ vg/kg to about 3.25x10¹⁴ vg/kg, or about 1.75x10¹⁴ vg/kg to about 3.0x10¹⁴ vg/kg, or about 1.75x10¹⁴ vg/kg to about 2.75x10¹⁴ vg/kg, or about 1.75x10¹⁴ vg/kg to about 2.5x10¹⁴ vg/kg, or about 1.75x10¹⁴ vg/kg to about 2.25x10¹⁴ vg/kg, or about 1.75x10¹⁴ vg/kg to about 2.0x10¹⁴ vg/kg, or about 2.0x10¹⁴ vg/kg to 1.0x10¹⁵, or about 2.0x10¹⁴ vg/kg to 6.0x10¹⁴, or about 2.0x10¹⁴ vg/kg to 5.0x10¹⁴, or about 2.0x10¹⁴ vg/kg to about 4.0x10¹⁴ vg/kg, or about 2.0x10¹⁴ vg/kg to about 3.75x10¹⁴ vg/kg, or about 2.0x10¹⁴ vg/kg to about 3.5x10¹⁴ vg/kg, or about 2.0x10¹⁴ vg/kg to about 3.25x10¹⁴ vg/kg. In one embodiment, the rAAV is AAVrh74.MHCK7.microdystrophin. In one embodiment, the AAVrh74.MHCK7.microdystrophin is the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ IDNO: 6. In one embodiment, the rAAV is AAVrh74.MCK.microdystrophin. In one embodiment, the AAVrh74.MCK.microdystrophin is the AAVrh74.MCK.microdystrophin of nucleotides 56-4820 of SEQ ID NO: 5.

In one embodiment, the compositions of the invention are formulated for intravenous administration and comprise a dose of rAAV that is about 2.0 x10¹⁴ vg/kg. In another embodiment, the compositions of the invention are formulated for intravenous administration and comprise a dose of rAAV that is about 5.0x10¹² vg/kg, or about 6.0x10¹² vg/kg, or about 7.0x10¹² vg/kg, or about 8.0x10¹² vg/kg, or about 9.0x10¹² vg/kg, or about 1.0x10¹³ vg/kg, or about 1.25x10¹³ vg/kg, or about 1.5x10¹³ vg/kg, or about 1.75x10¹³ vg/kg, or about 2.25x10¹³ vg/kg, or about 2.5x10¹³ vg/kg, or about 2.75x10¹³ vg/kg, or about 3.0x10¹³ vg/kg, or about 3.25x10¹³ vg/kg, or about 3.5x10¹³ vg/kg, or about 3.75x10¹³ vg/kg, or about 4.0x10¹³ vg/kg, or about 5.0x10¹³ vg/kg, or about 6.0x10¹³ vg/kg, or about 7.0x10¹³ vg/kg, or about 8.0x10¹³ vg/kg, or about 9.0x10¹³ vg/kg, or about 1.0x10¹⁴ vg/kg, or about 1.25x10¹⁴ vg/kg, or about 1.5x10¹⁴ vg/kg, or about 1.75x10¹⁴ vg/kg, or about 2.25x10¹⁴ vg/kg, or about 2.5x10¹⁴ vg/kg, or about 2.75x10¹⁴ vg/kg, or about 3.0x10¹⁴ vg/kg, or about 3.25x10¹⁴ vg/kg, or about 3.5x10¹⁴ vg/kg, or about 3.75x10¹⁴ vg/kg, or about 4.0x10¹⁴ vg/kg, or about 5.0x10¹⁴ vg/kg, or about 6.0x10¹⁴ vg/kg, or about 1x10¹⁵. In one embodiment, the rAAV is AAVrh74.MHCK7.microdystrophin. In one embodiment, the AAVrh74.MHCK7.microdystrophin is the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ IDNO: 6. In another embodiment, the rAAV is AAVrh74.MCK.microdystrophin. In one embodiment, the AAVrh74.MCK.microdystrophin is the AAVrh74.MCK.microdystrophin of nucleotides 56-4820 of SEQ ID NO: 5.

In any of the compositions of the invention, the dose of rAAV is delivered in about 5mL/kg to about 15 mL/kg, or about 8 mL/kg to about 12 mL/kg, or 8 mL/kg to about 10 mL/kg, or 5 mL/kg to about 10 mL/kg or about 10 mL/kg to 12 mL/kg, or about 10 mL/kg to 15 mL/kg or 10 mL/kg to about 20 mL/kg. In a particular embodiment, the composition comprises a dose of the rAAV delivered in about 10 mL/kg. In one embodiment, the rAAV is AAVrh74.MHCK7.microdystrophin. In one embodiment, the AAVrh74.MHCK7.microdystrophin is the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ IDNO: 6. In another embodiment, the rAAV is AAVrh74.MCK.microdystrophin. In one embodiment, the AAVrh74.MCK.microdystrophin is the AAVrh74.MCK.microdystrophin of nucleotides 56-4820 of SEQ ID NO: 5.

The compositions of the invention are formulated for administration by injection, infusion or implantation. For example, the compositions are formulated for administration by infusion over approximately one hour. In addition, the compositions of the invention are formulated for intravenous administration through a peripheral limb vein such as a peripheral arm vein or a peripheral leg vein. Alternatively, the infusion may be administered over approximately 30 minutes, or approximately 1.5 hours, or approximately 2 hours, or approximately 2.5 hours or approximately 3 hours.

Any of the compositions of the invention comprise a rAAV comprising the human micro-dystrophin nucleotide sequence of SEQ ID NO: 1 and the MHCK7 promoter sequence of SEQ ID NO: 2 or SEQ ID NO:7 or a rAAV vector comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6.

In particular, the compositions of the invention are for treating Duchenne muscular dystrophy or Becker's muscular dystrophy. For example, the invention provides for compositions for treating Duchenne muscular dystrophy or Becker's muscular dystrophy in a human subject in need thereof wherein the composition comprises a dose of recombinant adeno-virus associated (rAAV) rAAV.MHCK7.microdystrophin, wherein the composition is formulated for administration by intravenous infusion over approximately one hour and the dose of the rAAV administered is about 2x 10¹⁴ vg/kg, and wherein the rAAV comprises the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6.

In another embodiment, the invention also provides a composition comprising rAAV for reducing fibrosis in a subject in need thereof. In addition, the invention provides a composition comprising a rAAV vectors for preventing fibrosis in a subject suffering from a muscular dystrophy.

The invention also provides for compositions comprising rAAV for increasing muscular force and/or muscle mass in a subject suffering from a muscular dystrophy. In a further embodiment, the invention provides for compositions comprising any of the rAAV of the invention for treatment of muscular dystrophy.

In other embodiments of any of the compositions of the invention, after administration of said composition to a human subject in need of treatment for muscular dystrophy, the serum CK level in the subject is decreased as compared to the serum CK level before administration of the composition by a percentage level selected from the group consisting of:
a) at least 78% by 90, 180, or 270 days after the administration;
b) at least 46, 55, 70, or 85 % by 270 days after the administration;
c) at least 72, 73, 74, or 95 % by 180 days after the administration;
d) at least 87, 99, 93 or 95% by 90 days after the administration;
e) at least 70 % by 270 days after the administration;
f) 70 to 95% by 90, 180, or 270 days after the administration;
g) at least 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration; and
h) 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration.

In another embodiment, the invention provides for use of a dose of recombinant adeno-virus associated (rAAV) rAAV.MHCK7.microdystrophin for the preparation of a medicament for the treatment of muscular dystrophy in a human subject in need thereof, wherein the medicament is formulated for a systemic route of administration and the dose of the rAAV is about 1x10¹⁴ vg/kg to about 4x 101⁴ vg/kg. In one embodiment, the rAAV is AAVrh74.MHCK7.microdystrophin. In one embodiment, the AAVrh74.MHCK7.microdystrophin is the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ ID NO: 6. In one embodiment, the rAAV is AAVrh74.MCK.microdystrophin. In one embodiment, the AAVrh74.MCK.microdystrophin is the AAVrh74.MCK.microdystrophin of nucleotides 56-4820 of SEQ ID NO: 5.

For example, the medicament comprises a dose of rAAV of about 5.0x10¹² vg/kg to about 1.0x10¹⁴ vg/kg, or about 5.0x10¹² vg/kg to1.0x10¹⁴ vg/kg, or about 5.0x10¹² vg/kg to about 2.0x10¹⁴ vg/kg, or about 5.0x10¹² vg/kg to about 1.0x10¹⁴ vg/kg, or about 5.0x10¹² vg/kg to about 5.0x10¹³ vg/kg, or about 5.0x10¹² vg/kg to about 2.0x10¹³ vg/kg, or about 5.0x10¹² vg/kg to about 1.0x10¹³ vg/kg, or 1.0x10¹⁴ vg/kg to about 1.0x10¹⁵ vg/kg, or 1.0x10¹³ vg/kg to about 1.0x10¹⁴ vg/kg, or about 1.0x10¹³ vg/kg to1.0x10¹⁴ vg/kg, or about 1.0x10¹³ vg/kg to about 2.0x10¹⁴ vg/kg, or about 1.0x10¹³ vg/kg to about 1.0x10¹⁴ vg/kg, or about 1.0x10¹³ vg/kg to about 5.0x10¹³ vg/kg, or about 1.0x10¹³ vg/kg to about 3.0x10¹⁴ vg/kg, or about 1.0x10¹³ vg/kg to about 5.0x10¹⁴ vg/kg, or about 1.0x10¹³ vg/kg to about 6.0x10¹⁴ vg/kg, or 1.0x10¹³ vg/kg to about 1.0x10¹⁵ vg/kg, or 5.0x10¹³ vg/kg to about 1.0x10¹⁴ vg/kg, or about 5.0x10¹³ vg/kg to1.0x10¹⁴ vg/kg, or about 5.0x10¹³ vg/kg to about 2.0x10¹⁴ vg/kg, or about 5.0x10¹³ vg/kg to about 1.0x10¹⁴ vg/kg, or about 5.0x10¹³ vg/kg to about 3.0x10¹⁴ vg/kg, or about 5.0x10¹³ vg/kg to about 5.0x10¹⁴ vg/kg, or about 5.0x10¹³ vg/kg to about 6.0x10¹⁴ vg/kg, or 5.0x10¹³ vg/kg to about 1.0x10¹⁵ vg/kg, or 1.0x10¹⁴ vg/kg to about 6.0x10¹⁴ vg/kg, or 1.0x10¹⁴ vg/kg to about 5.0x10¹⁴ vg/kg, or 1.0x10¹⁴ vg/kg to about 4.0x10¹⁴ vg/kg, or 1.0x10¹⁴ vg/kg to about 1.0x10¹⁵ vg/kg, or 1.0x10¹⁴ vg/kg to about 3.0x10¹⁴ vg/kg, or about 1.0x10¹⁴ vg/kg to about 2.5x10¹⁴ vg/kg, or 1.0x10¹⁴ vg/kg to about 2.0x10¹⁴ vg/kg, or about 1.25x10¹⁴ vg/kg to about 3.75x10¹⁴ vg/kg, or about 1.25x10¹⁴ vg/kg to 6.0x10¹⁴, or about 1.25x10¹⁴ vg/kg to 5.0x10¹⁴, or about 1.25x10¹⁴ vg/kg to 4.0x10¹⁴, or about 1.25x10¹⁴ vg/kg to 1.0x10¹⁵ or about 1.25x10¹⁴ vg/kg to about 3.5 x10¹⁴ vg/kg, or about 1.25x10¹⁴ vg/kg to about 3.0x10¹⁴ vg/kg, or about 1.25x10¹⁴ vg/kg to about 2.75x10¹⁴ vg/kg, or about 1.25x10¹⁴ vg/kg to about 2.5x10¹⁴ vg/kg, or about 1.25x10¹⁴ vg/kg to about 2.0x10¹⁴ vg/kg, or 1.25x10¹⁴ vg/kg to about 3.75x10¹⁴ vg/kg, or about 1.25x10¹⁴ vg/kg to about 3.5 x10¹⁴ vg/kg, or 1.5x10¹⁴ vg/kg to about 1.0x10¹⁵ vg/kg, or about 1.5x10¹⁴ vg/kg to 6.0x10⁴, or about 1.5x10¹⁴ vg/kg to 5.0x10¹⁴, or about 1.5x10¹⁴ vg/kg to 4.0x10¹⁴, or about 1.5x10¹⁴ vg/kg to about 3.75x10¹⁴ vg/kg, or about 1.5x10¹⁴ vg/kg to about 3.5x10¹⁴ vg/kg, or about 1.5x10¹⁴ vg/kg to about 3.25x10¹⁴ vg/kg, or about 1.5x10¹⁴ vg/kg to about 3.0x10¹⁴ vg/kg, or about 1.5x10¹⁴ vg/kg to about 2.75x10¹⁴ vg/kg, or about 1.5x10¹⁴ vg/kg to about 2.5x10¹⁴ vg/kg, or about 1.5x10¹⁴ vg/kg to about 2.0x10¹⁴ vg/kg, or 1.75x10¹⁴ vg/kg to about 1.0x10¹⁵ vg/kg, or about 1.75x10¹⁴ vg/kg to 6.0x10¹⁴, or about 1.75x10¹⁴ vg/kg to 5.0x10¹⁴, or about 1.75x10¹⁴ vg/kg to 4.0x10¹⁴, or about 1.75x10¹⁴ vg/kg to about 3.75x10¹⁴ vg/kg, or about 1.75x10¹⁴ vg/kg to about 3.5x10¹⁴ vg/kg, or about 1.75x10¹⁴ vg/kg to about 3.25x10¹⁴ vg/kg, or about 1.75x10¹⁴ vg/kg to about 3.0x10¹⁴ vg/kg, or about 1.75x10¹⁴ vg/kg to about 2.75x10¹⁴ vg/kg, or about 1.75x10¹⁴ vg/kg to about 2.5x10¹⁴ vg/kg, or about 1.75x10¹⁴ vg/kg to about 2.25x10¹⁴ vg/kg, or about 1.75x10¹⁴ vg/kg to about 2.0x10¹⁴ vg/kg, or about 2.0x10¹⁴ vg/kg to 1.0x10¹⁵, or about 2.0x10¹⁴ vg/kg to 6.0x10¹⁴, or about 2.0x10¹⁴ vg/kg to 5.0x10¹⁴, or about 2.0x10¹⁴ vg/kg to about 4.0x10¹⁴ vg/kg, or about 2.0x10¹⁴ vg/kg to about 3.75x10¹⁴ vg/kg, or about 2.0x10¹⁴ vg/kg to about 3.5x10¹⁴ vg/kg, or about 2.0x10¹⁴ vg/kg to about 3.25x10¹⁴ vg/kg. In one embodiment, the rAAV is AAVrh74.MHCK7.microdystrophin. In one embodiment, the AAVrh74.MHCK7.microdystrophin is the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ ID NO: 6. In one embodiment, the rAAV is AAVrh74.MCK.microdystrophin. In one embodiment, the AAVrh74.MCK.microdystrophin is the AAVrh74.MCK.microdystrophin of nucleotides 56-4820 of SEQ ID NO: 5.

In one embodiment, the medicaments of the invention are formulated for systemic administration of a dose of rAAV wherein the systemic route of administration is an intravenous route and the dose of the rAAV administered is about 2.0 x10¹⁴ vg/kg. In another embodiment, the medicament of the invention is formulated for systemic administration of a dose of rAAV wherein the systemic route of administration is an intravenous route and the dose of the rAAV is about 5.0x10¹² vg/kg, or about 6.0x10¹² vg/kg, or about 7.0x10¹² vg/kg, or about 8.0x10¹² vg/kg, or about 9.0x10¹² vg/kg, or about 1.0x10¹³ vg/kg, or about 1.25x10¹³ vg/kg, or about 1.5x10¹³ vg/kg, or about 1.75x10¹³ vg/kg, or about 2.25x10¹³ vg/kg, or about 2.5x10¹³ vg/kg, or about 2.75x10¹³ vg/kg, or about 3.0x10¹³ vg/kg, or about 3.25x10¹³ vg/kg, or about 3.5x10¹³ vg/kg, or about 3.75x10¹³ vg/kg, or about 4.0x10¹³ vg/kg, or about 5.0x10¹³ vg/kg, or about 6.0x10¹³ vg/kg, or about 7.0x10¹³ vg/kg, or about 8.0x10¹³ vg/kg, or about 9.0x10¹³ vg/kg, or about 1.0x10¹⁴ vg/kg, or about 1.25x10¹⁴ vg/kg, or about 1.5x10¹⁴ vg/kg, or about 1.75x10¹⁴ vg/kg, or about 2.25x10¹⁴ vg/kg, or about 2.5x10¹⁴ vg/kg, or about 2.75x10¹⁴ vg/kg, or about 3.0x10¹⁴ vg/kg, or about 3.25x10¹⁴ vg/kg, or about 3.5x10¹⁴ vg/kg, or about 3.75x10¹⁴ vg/kg, or about 4.0x10¹⁴ vg/kg, or about 5.0x10¹⁴ vg/kg, or about 6.0x10¹⁴ vg/kg, or about 1x10¹⁵ vg/kg. In one embodiment, the rAAV is AAVrh74.MHCK7.microdystrophin. In one embodiment, the AAVrh74.MHCK7.microdystrophin is the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ ID NO: 6. In one embodiment, the rAAV is AAVrh74.MCK.microdystrophin. In one embodiment, the AAVrh74.MCK.microdystrophin is the AAVrh74.MCK.microdystrophin of nucleotides 56-4820 of SEQ ID NO: 5.

In any of the uses of the invention, the medicament comprises a dose of rAAV in about 5mL/kg to about 15 mL/kg, or about 8 mL/kg to about 12 mL/kg, or 8 mL/kg to about 10 mL/kg, or 5 mL/kg to about 10 mL/kg or about 10 mL/kg to 12 mL/k, or about 10 mL/kg to 15 mL/kg or 10 mL/kg to about 20 mL/kg. In a particular embodiment, the dose or the rAAV is in about 10 mL/kg. In one embodiment, the rAAV is AAVrh74.MHCK7.microdystrophin. In one embodiment, the AAVrh74.MHCK7.microdystrophin is the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ ID NO: 6. In one embodiment, the rAAV is AAVrh74.MCK.microdystrophin. In one embodiment, the AAVrh74.MCK.microdystrophin is the AAVrh74.MCK.microdystrophin of nucleotides 56-4820 of SEQ ID NO: 5.

In any of the uses of the invention, the medicament is formulated for administration by injection, infusion or implantation. For example, the medicament is formulated for administration by infusion over approximately one hour. In addition, the medicament is formulated for intravenous administration through a peripheral limb vein, such as a peripheral arm vein or a peripheral leg vein. Alternatively, the infusion may be administered over approximately 30 minutes, or approximately 1.5 hours, or approximately 2 hours, or approximately 2.5 hours or approximately 3 hours.

In any of the uses of the invention, the medicament comprises an rAAV comprising the human micro-dystrophin nucleotide sequence of SEQ ID NO: 1 and the MHCK7 promoter sequence of SEQ ID NO: 2 or SEQ ID NO:7 or the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ ID NO: 6.

A particular use of the invention is for preparation of a medicament for the treatment of Duchenne muscular dystrophy or Becker's muscular dystrophy. For example, the invention provides for use of a dose of recombinant adeno-virus associated (rAAV) rAAV.MHCK7.microdystrophin for the preparation of a medicament for treating Duchenne muscular dystrophy in a or Becker's muscular dystrophy human subject in need thereof, wherein the medicament is formulated for administration by intravenous infusion over approximately one hour and the dose of the rAAV administered is about 2x 10¹⁴ vg/kg, and wherein the rAAV comprises the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ ID NO: 6.

In a further embodiment, the invention provides for use of a rAAV for preparation of a medicament for reducing fibrosis in a subject in need thereof. For example, the subject in need can be suffering from a muscular dystrophy, such as DMD or any other dystrophin associated muscular dystrophy.

In another embodiment, the invention provides for use of a rAAV for the preparation of a medicament to prevent fibrosis in a subject suffering from a muscular dystrophy.

In addition, the invention provides for use of a rAAV for the preparation of a medicament to increase muscular strength and/or muscle mass in a subject suffering from muscular dystrophy.

The invention also provides for use of the rAAV for the preparation of a medicament for treatment of muscular dystrophy.

The invention provides for use of a rAAV vector comprising the human micro-dystrophin nucleotide sequence of SEQ ID NO: 1 and the MHCK7 promoter nucleotide sequence of SEQ ID NO: 2 or SEQ ID NO:7 for preparation of a medicament for the treatment of a muscular dystrophy or a rAAV vector comprising the AAVrf74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ ID NO: 6 for treatment of muscular dystrophy.

In other embodiments of any of the uses of the invention, the serum CK level in the subject is decreased after administration of the rAAV to the subject as compared to the serum CK level before administration of the rAAV by a percentage level selected from the group consisting of:
a) at least 78% by 90, 180, or 270 days after the administration;
b) at least 46, 55, 70, or 95 % by 270 days after the administration;
c) at least 72, 73, 74, or 95 % by 180 days after the administration;
d) at least 87, 88, 93 or 95% by 90 days after the administration;
e) at least 70 % by 270 days after the administration;
f) 70 to 95% by 90, 180, or 270 days after the administration;
g) at least 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration; and
h) 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration.

In any of the compositions for treating a muscular dystrophy or the uses of a medicament for treating a muscular dystrophy, the level of micro-dystrophin gene expression in a cell of the subject is increased after administration of the composition or medicament. Expression of the micro-dystrophin gene in the cell is detected by measuring the micro-dystrophin protein level by Western blot in muscle biopsied before and after administration of the composition or medicament. In particular, the level of micro-dystrophin protein is increased by at least about 70% to at least about 80%, or at least about 70% to at least about 90%, or at least about 80% to at least about 90% after administration of the composition or medicament compared to the level of micro-dystrophin before administration of the composition or medicament. For example, the level of micro-dystrophin protein is increased by at least about 70% or at least about 71% or at least about 72% or at least about 73% or at least about 74% or at least about 75% or at least about 76% or at least about 77% or at least about 78% or at least about 79% or at least about 80%, or at least about 81%, or at least about 82%, or at least about 83%, or at least about 84%, or at least about 85% after administration of the composition compared to the level of micro-dystrophin before administration of the composition or medicament.

In addition, expression of the micro-dystrophin gene in the cell is detected by measuring the micro-dystrophin protein level by immunohistochemistry in muscle biopsies before and after administration of the composition or medicament. The level of micro-dystrophin protein is increased by at least about 70% to at least about 80%, or at least about 70% to at least about 90%, or at least about 80% to at least about 90% after administration of rAAV compared to the level of micro-dystrophin before administration of the composition or medicament. For example, the level of micro-dystrophin protein is increased by at least about 70% or at least about 71% or at least about 72% or at least about 73% or at least about 74% or at least about 75% or at least about 76% or at least about 77% or at least about 78% or at least about 79% or at least about 80%, or at least about 81%, or at least about 82%, or at least about 83%, or at least about 84%, or at least about 85% after administration of the composition or medicament compared to the level of micro-dystrophin before administration of the composition or medicament.

In any of the compositions for treating a muscular dystrophy, the serum CK level in the subject is decreased after administration of the rAAV as compared to serum CK level before administration of the composition or medicament. For example, the serum CK level in the subject is decreased by about 65 % to about 90% or about 65% to about 95% or about 75% to about 90% or about 80% to about 90% or about 85% to about 95% or about 87% to about 95% or about 87% to about 90% by 60 days after administration of the composition or medicament as compared to the serum CK level before administration of the composition or medicament. In particular, in any of the compositions for treating a muscular dystrophy of the invention , the serum CK level in the subject is decreased by about 87% by 60 days after administration of the composition or medicament as compared to the serum CK level before administration of the composition or medicament or in any of the compositions for treating a muscular dystrophy or the uses of a medicament for treating a muscular dystrophy of the invention, the serum CK level in the subject is decreased by about 72% by 60 days after administration of the composition or medicament as compared to the serum CK level before administration of the composition or medicament, or in any of the compositions for treating a muscular dystrophy of the invention, the serum CK level in the subject is decreased by about 73% by 60 days after administration of the composition or medicament as compared to the serum CK level before administration of the composition or medicament, or in any of the compositions for treating a muscular dystrophy or the uses of a medicament for treating a muscular dystrophy of the invention, the serum CK level in the subject is decreased by about 78% by 60 days after administration of the composition or medicament as compared to the serum CK level before administration of the composition or in any of the compositions for treating a muscular dystrophy or the uses of a medicament for treating a muscular dystrophy of the invention, the serum CK level in the subject is decreased by about 95% by 60 days after administration of the composition or medicament as compared to the serum CK level before administration of the composition or medicament. In any of the composition s for treating a muscular dystrophy or the uses of a medicament for treating a muscular dystrophy, the number of micro-dystrophin positive fibers in the muscle tissue of the subject is increased after administration of the composition or medicament as compared to the number of micro-dystrophin positive fibers before administration of the composition or medicament. For example, the number of micro-dystrophin positive fibers is detected by measuring the micro-dystrophin protein level by Western blot or immunohistochemistry on muscle biopsies before and after administration of the composition or medicament.

In any of the compositions for treating a muscular dystrophy or the uses of a medicament for treating a muscular dystrophy, administration of the composition or medicament upregulates expression of DAPC proteins such as alpha-sarcoglycan or beta-sarcoglycan. For example, the level of alpha-sarcoglycan in the subject is increased after administration of the composition or medicament as compared to the level of alpha-sarcoglycan before administration of the composition or medicament. In addition, the level of beta-sarcoglycan in the subject is increased after administration of the composition or medicament as compared to the level of the beta-sarcoglycan before administration of the composition or medicament. The level of alpha-sarcoglycan or beta-sarcoglycan is detected by measuring the alpha-sarcoglycan or beta-sarcoglycan protein level by Western blot or immunohistochemistry on muscle biopsies before and after administration of the composition or medicament.

In any of the compositions for treating a muscular dystrophy or the uses of a medicament for treating a muscular dystrophy, disease progression in the subject is delayed after administration of the composition or medicament as measured by any of: the six minute walk test, time to rise, ascend 4 steps, ascend and descend 4 steps, North Star Ambulatory Assessment (NSAA), 10 meter timed test, 100 meter timed test, hand held dynamometry (HHD), Timed Up and Go, and/or Gross Motor Subtest Scaled (Bayley-III) score.

For example, after administration of any of the compositions for treating a muscular dystrophy or the uses of a medicament for treating a muscular dystrophy, the subject has at least a 6-point improvement in NSAA score at least 270 days after administration of the composition or medicament as compared to NSAA score before administration of the rAAV. Further, in any of the methods, the subject has at least about 0.8 second improvement in time to rise at least 270 days after administration of the composition or medicament as compared to time to rise before administration of the composition or medicament. In addition, in any of the methods or uses of the invention, the subject has at least about 1.2 second improvement in time to ascend 4 steps test at least 270 days after administration of the composition or medicament as compared to time to ascend 4 steps test before administration of the composition or medicament. In addition, in any of the methods or uses of the invention, the subject has at least about 7 second improvement in the 100 m timed test at least 270 days after administration of the composition or medicament as compared to the 100 m timed test before administration of the composition or medicament.

In another embodiment, the invention provides for compositions for expressing micro-dystrophin gene in a patient cell comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8 or nucleotides 56-5022 of SEQ ID NO: 6. In a further embodiment, the invention provides for use of a dose of a AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8 or nucleotides 56-5022 of SEQ ID NO: 6 for the preparation of a medicament for expressing micro-dystrophin gene in a patient cell. For example, expression of the micro-dystrophin gene in the patient cell is detected by measuring the micro-dystrophin protein level by Western blot or immunohistochemistry in muscle biopsies before and after administration of the rAAV.MHCK7.micro-dystrophin construct. In addition, the expression of the micro-dystrophin gene is measured in the patient by detecting greater the number of vector genomes per nucleus, wherein 1 vector genome per nucleus is about 50% micro-dystrophin expression and great than 1 copy per nucleus is consistent with micro-dystrophin expression level. For example, the cells have 1.2 vector copies per nucleus, or 1.3 vector copies per nucleus, or 1.4 vector copies per nucleus, or 1.5 vector copies per nucleus, or 1.6 vector copies per nucleus, or 1.7 vector copies per nucleus, or 1.8 vector copies per nucleus, or 1.9 vector copies per nucleus.

In a further embodiment, the invention provides for compositions for decreasing serum CK levels in a patient in need thereof, the composition comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. In addition, the invention provides for use of a dose of AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 for the preparation of a medicament for decreasing serum CK levels in a patient in need thereof. For example, the serum CK level in the patient is decreased by at least about 65% to about 90% or about 65% to about 95% or about 75% to about 90% or about 80% to about 90% or about 85% to about 95% or about 87% to about 95% or about 87% to about 90% by 60 days after administration of the composition or medicament as compared to the serum CK level before administration of the composition or medicament. In particular, the serum CK level in the subject is decreased by about 87% by 60 days after administration of the composition or medicament as compared to the serum CK level before administration of the composition or medicament, or decreased by about 72% by 60 days after administration of the composition or medicament as compared to the serum CK level before administration of the composition or medicament, or decreased by about 73% by 60 days after administration of the composition or medicament as compared to the serum CK level before administration of the composition or medicament, or decreased by about 78% by 60 days after administration of the composition or medicament as compared to the serum CK level before administration of the composition or medicament, or decreased by about 95% by 60 days after administration of the composition or medicament as compared to the serum CK level before administration of the composition or medicament.

The invention also provides for compositions for increasing micro-dystrophin positive fibers in a patient muscle tissue comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. In addition, the invention provides for use of a dose of AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 for the preparation of a medicament for increasing micro-dystrophin positive fibers in a patient muscle tissue. For example, the number of micro-dystrophin positive fibers is detected by measuring the dystrophin protein level by Western blot or immunohistochemistry on muscle biopsies before and after administration of the composition or medicament. In addition, the expression of the micro-dystrophin gene is measured in the patient by detecting greater the number of vector genomes per nucleus, wherein 1 vector genome per nucleus is about 50% micro-dystrophin expression and great than 1 copy per nucleus is consistent with micro-dystrophin expression level. For example, the cells have 1.2 vector copies per nucleus, or 1.3 vector copies per nucleus, or 1.4 vector copies per nucleus, or 1.5 vector copies per nucleus, or 1.6 vector copies per nucleus, or 1.7 vector copies per nucleus, or 1.8 vector copies per nucleus, or 1.9 vector copies per nucleus.

In another embodiment, the invention provides for compositions for increasing the expression of alpha-sarcoglycan in a patient in need thereof comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. The invention also provides for use of a dose of AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 for the preparation of a medicament for increasing the expression of alpha-sarcoglycan in a patient in need thereof. For example, the level of alpha-sarcoglycan is detected by measuring the alpha-sarcoglycan protein level by Western blot or immunohistochemistry on muscle biopsies before and after administration of the composition or medicament.

In addition, the invention provides for compositions for increasing the expression of beta-sarcoglycan in a patient in need thereof comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. The invention also provides for use of the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 for the preparation of a medicament for increasing the expression of beta-sarcoglycan in a patient in need thereof. For example, the level of beta-sarcoglycan is detected by measuring the beta-sarcoglycan protein level by Western blot or immunohistochemistry on muscle biopsies before and after administration of the composition or medicament.

The invention also provides for use of a dose of AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 for the preparation of a medicament for treating a patient with Duchenne muscular dystrophy or Becker muscular dystrophy, such that administration of the medicament results in disease progression in the patient is delayed as measured by any of: the six minute walk test, time to rise, ascend 4 steps, ascend and descend 4 steps, North Star Ambulatory Assessment (NSAA), 10 meter timed test, 100 meter timed test, hand held dynamometry (HHD), Timed Up and Go, and/or Gross Motor Subtest Scaled (Bayley-III) score.

For example, the subject has at least a 6-point improvement in NSAA score at least 270 days after administration of the composition or medicament as compared to NSAA score before administration of the composition or medicament. Further, the subject has at least about 0.8 second improvement in time to rise at least 270 days after administration of the composition or medicament as compared to time to rise before administration of the composition or medicament. In addition, the subject has at least about 1.2 second improvement in time to ascend 4 steps test at least 270 days after administration of the composition or medicament as compared to time to ascend 4 steps test before administration of the composition or medicament. In addition, the subject has at least about 7 second improvement in the 100 m timed test at least 270 days after administration of the composition or medicament as compared to the 100 m timed test before administration of the composition or medicament.

### BRIEF DESCRIPTION OF THE DRAWINGS

**Figure 1** illustrates the rAAV.MHCK7.micro-dystrophin construct. In this construct, the cDNA expression cassette is flanked by AAV2 inverted terminal repeat sequences (ITR). The construct is characterized by an in-frame rod deletion (R4-R23), while hinges 1, 2 and 4 (H₁, H₂ and H₄) and the cysteine rich domain remain producing a 138 kDa protein. The expression of the micro-dystrophin protein (3579 bp) is guided by a MHCK7 promoter (795 bp). The intron and 5' UTR are derived from plasmid pCMVβ (Clontech). The micro-dystrophin cassette had a consensus Kozak immediately in front of the ATG start and a small 53 bp synthetic polyA signal for mRNA termination. The human micro-dystrophin cassette contained the (R4-R23/Δ71-78) domains as previously described by Harper et al. (Nature Medicine 8, 253-261 (2002)).
**Figure 2** provides the nucleic acid sequence (SEQ ID NO: 3) AAVrh74.MHCK7.micro-dystrophin.
**Figure 3** provides the pNLREP2-Caprh74 AAV helper plasmid map.
**Figure 4** provides the Ad Helper plasmid pHELP.
**Figure 5** illustrates the rAAV.MCK.micro-dystrophin plasmid construct.
**Figure 6** provides the nucleic acid sequence (SEQ ID NO: 5) rAAVrh74.MCK.micro-dystrophin.
**Figure 7** demonstrates micro-dystrophin gene expression in muscle fibers of gastrocnemius muscle biopsy as measured by immunocytochemistry.
**Figures 8A- 8C** provide Western blots demonstrating micro-dystrophin protein expression at the correct molecular weight. In Fig. 8C, Subject 4 samples (*) were diluted 1:4 (to linear range) as ULDQ (>80%) exceeded in initial analysis, and mean values were multiplied by the dilution correction factor for final value in comparison to normal. Mean Micro-dystrophin Expression Vs. Normal was 182.7% in Method 1 and 222.0% in Method 2.
**Figures 9A-9C** demonstrates administration of rAAVrh74.MHCK7. micro-dystrophin upregulates expression of the DAPC proteins, alpha-sarcoglycan and beta-sarcoglycan.
**Figure 10** demonstrates sustained dramatic reduction in Creatine Kinase (CK) with administration of rAAVrh74.MHCK7. micro-dystrophin.
**Figure 11** provides the mean CK change from baseline to day 270. This data demonstrated that CK significantly decreases over time after administration of rAAVrh74.MHCK7.micro-dystrophin.
**Figure 12** provides the mean NSAA change and the mean CK change from baseline to day 270. This data demonstrated that NSAA significantly increased over time after administration of rAAVrh74.MHCK7.micro-dystrophin.
   F provides the nucleic acid sequence (SEQ ID NO: 9) AAVrh74.MHCK7.micro-dystrophin.
**Figure 14** illustrates the AAVrh74.MHCK7.micro-dystrophin plasmid construct.

AAVrh74.MHCK7.micro-dystrophin plasmid construct, which comprises kanamycin resistance gene.

### DETAILED DESCRIPTION

The present invention provides for gene therapy vectors, e.g. rAAV vectors, overexpressing human micro-dystrophin and methods of reducing and preventing fibrosis in muscular dystrophy patients. Muscle biopsies taken at the earliest age of diagnosis of DMD reveal prominent connective tissue proliferation. Muscle fibrosis is deleterious in multiple ways. It reduces normal transit of endomysial nutrients through connective tissue barriers, reduces the blood flow and deprives muscle of vascular-derived nutritional constituents, and functionally contributes to early loss of ambulation through limb contractures. Over time, treatment challenges multiply as a result of marked fibrosis in muscle. This can be observed in muscle biopsies comparing connective tissue proliferation at successive time points. The process continues to exacerbate leading to loss of ambulation and accelerating out of control, especially in wheelchair-dependent patients.

Without early treatment including a parallel approach to reduce fibrosis it is unlikely that the benefits of exon skipping, stop-codon read-through, or gene replacement therapies can ever be fully achieved. Even small molecules or protein replacement strategies are likely to fail without an approach to reduce muscle fibrosis. Previous work in aged *mdx* mice with existing fibrosis treated with AAV.micro-dystrophin demonstrated that we could not achieve full functional restoration (Liu, M., et al., Mol Ther 11, 245-256 (2005)). It is also known that progression of DMD cardiomyopathy is accompanied by scarring and fibrosis in the ventricular wall.

As used herein, the term "AAV" is a standard abbreviation for adeno-associated virus. Adeno-associated virus is a single-stranded DNA parvovirus that grows only in cells in which certain functions are provided by a co-infecting helper virus. There are currently thirteen serotypes of AAV that have been characterized. General information and reviews of AAV can be found in, for example, Carter, 1989, Handbook of Parvoviruses, Vol. 1, pp. 169-228, and Berns, 1990, Virology, pp. 1743-1764, Raven Press, (New York). However, it is fully expected that these same principles will be applicable to additional AAV serotypes since it is well known that the various serotypes are quite closely related, both structurally and functionally, even at the genetic level. (See, for example, Blacklowe, 1988, pp. 165-174 of Parvoviruses and Human Disease, J. R. Pattison, ed.; and Rose, Comprehensive Virology 3:1-61 (1974)). For example, all AAV serotypes apparently exhibit very similar replication properties mediated by homologous rep genes; and all bear three related capsid proteins such as those expressed in AAV2. The degree of relatedness is further suggested by heteroduplex analysis which reveals extensive cross-hybridization between serotypes along the length of the genome; and the presence of analogous self-annealing segments at the termini that correspond to "inverted terminal repeat sequences" (ITRs). The similar infectivity patterns also suggest that the replication functions in each serotype are under similar regulatory control.

An "AAV vector" as used herein refers to a vector comprising one or more polynucleotides of interest (or transgenes) that are flanked by AAV terminal repeat sequences (ITRs). Such AAV vectors can be replicated and packaged into infectious viral particles when present in a host cell that has been transfected with a vector encoding and expressing rep and cap gene products.

An "AAV virion" or "AAV viral particle" or "AAV vector particle" refers to a viral particle composed of at least one AAV capsid protein and an encapsidated polynucleotide AAV vector. If the particle comprises a heterologous polynucleotide (i.e. a polynucleotide other than a wild-type AAV genome such as a transgene to be delivered to a mammalian cell), it is typically referred to as an "AAV vector particle" or simply an "AAV vector". Thus, production of AAV vector particle necessarily includes production of AAV vector, as such a vector is contained within an AAV vector particle.

### AAV

Adeno-associated virus (AAV) is a replication-deficient parvovirus, the single-stranded DNA genome of which is about 4.7 kb in length including 145 nucleotide inverted terminal repeat (ITRs). There are multiple serotypes of AAV. The nucleotide sequences of the genomes of the AAV serotypes are known. For example, the nucleotide sequence of the AAV serotype 2 (AAV2) genome is presented in Srivastava et al., J Virol, 45: 555-564 (1983) as corrected by Ruffing et al., J Gen Virol, 75: 3385-3392 (1994). As other examples, the complete genome of AAV-1 is provided in GenBank Accession No. NC_002077; the complete genome of AAV-3 is provided in GenBank Accession No. NC_1829; the complete genome of AAV-4 is provided in GenBank Accession No. NC_001829; the AAV-5 genome is provided in GenBank Accession No. AF085716; the complete genome of AAV-6 is provided in GenBank Accession No. NC_00 1862; at least portions of AAV-7 and AAV-8 genomes are provided in GenBank Accession Nos. AX753246 and AX753249, respectively (see also U.S. Patent Nos. 7,282,199 and 7,790,449 relating to AAV-8); the AAV-9 genome is provided in Gao et al., J. Virol., 78: 6381-6388 (2004); the AAV-10 genome is provided in Mol. Ther., 13(1): 67-76 (2006); and the AAV-11 genome is provided in Virology, 330(2): 375-383 (2004). Cloning of the AAVrh.74 serotype is described in Rodino-Klapac., et al. Journal of translational medicine 5, 45 (2007). Cis-acting sequences directing viral DNA replication (rep), encapsidation/packaging and host cell chromosome integration are contained within the ITRs. Three AAV promoters (named p5, p19, and p40 for their relative map locations) drive the expression of the two AAV internal open reading frames encoding rep and cap genes. The two rep promoters (p5 and p19), coupled with the differential splicing of the single AAV intron (*e.g.,* at AAV2 nucleotides 2107 and 2227), result in the production of four rep proteins (rep 78, rep 68, rep 52, and rep 40) from the rep gene. Rep proteins possess multiple enzymatic properties that are ultimately responsible for replicating the viral genome. The cap gene is expressed from the p40 promoter and it encodes the three capsid proteins VP1, VP2, and VP3. Alternative splicing and non-consensus translational start sites are responsible for the production of the three related capsid proteins. A single consensus polyadenylation site is located at map position 95 of the AAV genome. The life cycle and genetics of AAV are reviewed in Muzyczka, Current Topics in Microbiology and Immunology, 158: 97-129 (1992).

AAV possesses unique features that make it attractive as a vector for delivering foreign DNA to cells, for example, in gene therapy. AAV infection of cells in culture is noncytopathic, and natural infection of humans and other animals is silent and asymptomatic. Moreover, AAV infects many mammalian cells allowing the possibility of targeting many different tissues *in vivo.* Moreover, AAV transduces slowly dividing and non-dividing cells, and can persist essentially for the lifetime of those cells as a transcriptionally active nuclear episome (extrachromosomal element). The AAV proviral genome is infectious as cloned DNA in plasmids which makes construction of recombinant genomes feasible. Furthermore, because the signals directing AAV replication, genome encapsidation and integration are contained within the ITRs of the AAV genome, some or all of the internal approximately 4.3 kb of the genome (encoding replication and structural capsid proteins, rep-cap) may be replaced with foreign DNA such as a gene cassette containing a promoter, a DNA of interest and a polyadenylation signal. The rep and cap proteins may be provided *in trans.* Another significant feature of AAV is that it is an extremely stable and hearty virus. It easily withstands the conditions used to inactivate adenovirus (56°C to 65°C for several hours), making cold preservation of AAV less critical. AAV may even be lyophilized. Finally, AAV-infected cells are not resistant to superinfection.

Multiple studies have demonstrated long-term (> 1.5 years) recombinant AAV-mediated protein expression in muscle. See, Clark et al., Hum Gene Ther, 8: 659-669 (1997); Kessler et al., Proc Nat. Acad Sc. USA, 93: 14082-14087 (1996); and Xiao et al., J Virol, 70: 8098-8108 (1996). See also, Chao et al., Mol Ther, 2:619-623 (2000) and Chao et al., Mol Ther, 4:217-222 (2001). Moreover, because muscle is highly vascularized, recombinant AAV transduction has resulted in the appearance of transgene products in the systemic circulation following intramuscular injection as described in Herzog et al., Proc Natl Acad Sci USA, 94: 5804-5809 (1997) and Murphy et al., Proc Natl Acad Sci USA, 94: 13921-13926 (1997). Moreover, Lewis et al., J Virol, 76: 8769-8775 (2002) demonstrated that skeletal myofibers possess the necessary cellular factors for correct antibody glycosylation, folding, and secretion, indicating that muscle is capable of stable expression of secreted protein therapeutics.

Recombinant AAV genomes of the invention comprise nucleic acid molecule of the invention and one or more AAV ITRs flanking a nucleic acid molecule. AAV DNA in the rAAV genomes may be from any AAV serotype for which a recombinant virus can be derived including, but not limited to, AAV serotypes AAVrh.74, AAV-1, AAV-2, AAV-3, AAV-4, AAV-5, AAV-6, AAV-7, AAV-8, AAV-9, AAV-10, AAV-11, AAV-12 and AAV-13. Production of pseudotyped rAAV is disclosed in, for example, WO 01/83692. Other types of rAAV variants, for example rAAV with capsid mutations, are also contemplated. See, for example, Marsic et al., Molecular Therapy, 22(11): 1900-1909 (2014). As noted in the Background section above, the nucleotide sequences of the genomes of various AAV serotypes are known in the art. To promote skeletal muscle specific expression, AAV1, AAV6, AAV8 or AAVrh.74 can be used.

DNA plasmids of the invention comprise rAAV genomes of the invention. The DNA plasmids are transferred to cells permissible for infection with a helper virus of AAV (e.g., adenovirus, E1-deleted adenovirus or herpesvirus) for assembly of the rAAV genome into infectious viral particles. Techniques to produce rAAV particles, in which an AAV genome to be packaged, rep and cap genes, and helper virus functions are provided to a cell are standard in the art. Production of rAAV requires that the following components are present within a single cell (denoted herein as a packaging cell): a rAAV genome, AAV rep and cap genes separate from (*i.e.,* not in) the rAAV genome, and helper virus functions. The AAV rep and cap genes may be from any AAV serotype for which recombinant virus can be derived and may be from a different AAV serotype than the rAAV genome ITRs, including, but not limited to, AAV serotypes AAV-1, AAV-2, AAV-3, AAV-4, AAV-5, AAV-6, AAV-7, AAVrh.74, AAV-8, AAV-9, AAV-10, AAV-11, AAV-12 and AAV-13. Production of pseudotyped rAAV is disclosed in, for example, WO 01/83692 which is incorporated by reference herein in its entirety.

A method of generating a packaging cell is to create a cell line that stably expresses all the necessary components for AAV particle production. For example, a plasmid (or multiple plasmids) comprising a rAAV genome lacking AAV rep and cap genes, AAV rep and cap genes separate from the rAAV genome, and a selectable marker, such as a neomycin resistance gene, are integrated into the genome of a cell. AAV genomes have been introduced into bacterial plasmids by procedures such as GC tailing (Samulski et al., 1982, Proc. Natl. Acad. S6. USA, 79:2077-2081), addition of synthetic linkers containing restriction endonuclease cleavage sites (Laughlin et al., 1983, Gene, 23:65-73) or by direct, blunt-end ligation (Senapathy & Carter, 1984, J. Biol. Chem., 259:4661-4666). The packaging cell line is then infected with a helper virus such as adenovirus. The advantages of this method are that the cells are selectable and are suitable for large-scale production of rAAV. Other examples of suitable methods employ adenovirus or baculovirus rather than plasmids to introduce rAAV genomes and/or rep and cap genes into packaging cells.

General principles of rAAV production are reviewed in, for example, Carter, 1992, Current Opinions in Biotechnology, 1533-539; and Muzyczka, 1992, Curr. Topics in Microbial. and Immunol., 158:97-129). Various approaches are described in Ratschin et al., Mol. Cell. Biol. 4:2072 (1984); Hermonat et al., Proc. Natl. Acad. Sci. USA, 81:6466 (1984); Tratschin et al., Mol. Cell. Biol. 5:3251 (1985); McLaughlin et al., J. Virol., 62:1963 (1988); and Lebkowski et al., Mol. Cell. Biol., 7:349 (1988). Samulski et al., J. Virol., 63:3822-3828 (1989); U.S. Patent No. 5,173,414; WO 95/13365 and corresponding U.S. Patent No. 5,658.776 ; WO 95/13392; WO 96/17947; PCT/US98/18600; WO 97/09441 (PCT/US96/14423); WO 97/08298 (PCT/US96/13872); WO 97/21825 (PCT/US96/20777); WO 97/06243 (PCT/FR96/01064); WO 99/11764; Perrin et al. Vaccine 13:1244-1250 (1995); Paul et al. Human Gene Therapy 4:609-615 (1993); Clark et al. Gene Therapy 3:1124-1132 (1996); U.S. Patent. No. 5,786,211; U.S. Patent No. 5,871,982; and U.S. Patent. No. 6,258,595. The foregoing documents are hereby incorporated by reference in their entirety herein, with particular emphasis on those sections of the documents relating to rAAV production.

The invention thus provides packaging cells that produce infectious rAAV. In one embodiment packaging cells may be stably transformed cancer cells such as HeLa cells, 293 cells and PerC.6 cells (a cognate 293 line). In another embodiment, packaging cells are cells that are not transformed cancer cells, such as low passage 293 cells (human fetal kidney cells transformed with E1 of adenovirus), MRC-5 cells (human fetal fibroblasts), WI-38 cells (human fetal fibroblasts), Vero cells (monkey kidney cells) and FRhL-2 cells (rhesus fetal lung cells).

Recombinant AAV (*i.e.,* infectious encapsidated rAAV particles) of the invention comprise a rAAV genome. In exemplary embodiments, the genomes of both rAAV lack AAV rep and cap DNA, that is, there is no AAV rep or cap DNA between the ITRs of the genomes. Examples of rAAV that may be constructed to comprise the nucleic acid molecules of the invention are set out in International Patent Application No. PCT/US2012/047999 (WO 2013/016352) incorporated by reference herein in its entirety.

In an exemplary embodiment, the recombinant AAV vector of the inveiton is produced by the triple transfection method (Xiao et al. , J Virol 72, 2224-2232 (1998) using the AAV vector plasmids rAAV.MHCK7.micro-dystrophin, pNLRep2-Caprh74 and pHelp, rAAV contains the micro-dystrophin gene expression cassette flanked by AAV2 inverted terminal repeat sequences (ITR). It is this sequence that is encapsidated into AAVrh74 virions. The plasmid contains the micro-dystrophin sequence and the MHCK7 enhancer and core promoter elements of the muscle specific promoter to drive gene expression. The expression cassette also contains an SV40 intron (SD/SA) to promote high-level gene expression and the bovine growth hormone polyadenylation signal is used for efficient transcription termination.

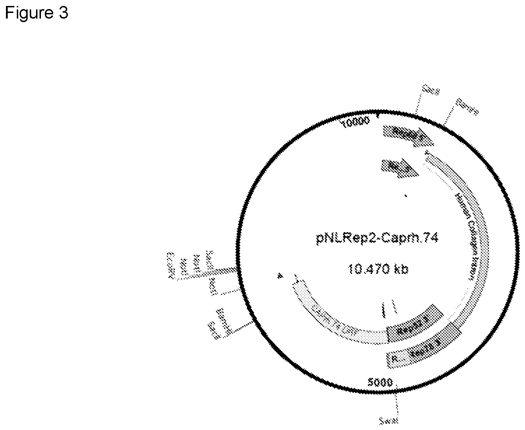

The pNLREP2-Caprh74 is an AAV helper plasmid that encodes the 4 wild-type AAV2 rep proteins and the 3 wild-type AAV VP capsid proteins from serotype rh74. A schematic map of the pNLREP2-Caprh74 plasmid is shown in Figure 3.

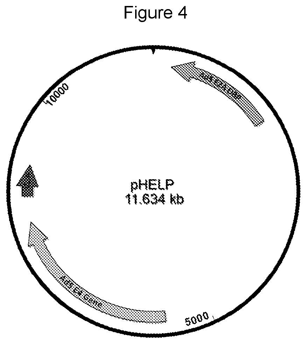

The pHELP adenovirus helper plasmid is 11,635 bp and was obtained from Applied Viromics. The plasmid contains the regions of adenovirus genome that are important for AAV replication, namely E2A, E4ORF6, and VA RNA (the adenovirus E1 functions are provided by the 293 cells). The adenovirus sequences present in this plasmid only represents ~40% of the adenovirus genome, and does not contain the *cis* elements critical for replication such as the adenovirus terminal repeats. Therefore, no infectious adenovirus is expected to be generated from such a production system. A schematic map of the pHELP plasmid is shown in Figure 4.

The rAAV may be purified by methods standard in the art such as by column chromatography or cesium chloride gradients. Methods for purifying rAAV vectors from helper virus are known in the art and include methods disclosed in, for example, Clark et al., Hum. Gene Ther., 10(6): 1031-1039 (1999); Schenpp and Clark, Methods Mol. Med., 69 427-443 (2002); U.S. Patent No. 6,566,118 and WO 98/09657.

In another embodiment, the invention contemplates compositions comprising rAAV of the present invention. Compositions of the invention comprise rAAV and a pharmaceutically acceptable carrier. The compositions may also comprise other ingredients such as diluents and adjuvants. Acceptable carriers, diluents and adjuvants are nontoxic to recipients and are preferably inert at the dosages and concentrations employed and include buffers and surfactants such as pluronics.

Titers of rAAV to be administered in methods of the invention will vary depending, for example, on the particular rAAV, the mode of administration, the treatment goal, the individual, and the cell type(s) being targeted, and may be determined by methods standard in the art. Titers of rAAV may range from about 1x10⁶, about 1x10⁷, about 1x10⁸, about 1x10⁹, about 1x10¹⁰, about 1x10¹¹, about 1x10¹², about 1x10¹³ to about 1x10¹⁴ or more DNase resistant particles (DRP) per ml. Dosages may also be expressed in units of viral genomes (vg). One exemplary method of determining encapsilated vector genome titer uses quantitative PCR such as the methods described in (Pozsgai et al., Mol. Ther. 25(4): 855-869, 2017).

Methods of transducing a target cell with rAAV, *in vivo* or *in vitro,* are contemplated by the invention. The *in vivo* methods comprise the step of administering an effective dose, or effective multiple doses, of a composition comprising a rAAV of the invention to an animal (including a human being) in need thereof. If the dose is administered prior to development of a disorder/disease, the administration is prophylactic. If the dose is administered after the development of a disorder/disease, the administration is therapeutic. In embodiments of the invention, an effective dose is a dose that alleviates (eliminates or reduces) at least one symptom associated with the disorder/disease state being treated, that slows or prevents progression to a disorder/disease state, that slows or prevents progression of a disorder/disease state, that diminishes the extent of disease, that results in remission (partial or total) of disease, and/or that prolongs survival. An example of a disease contemplated for prevention or treatment with methods of the invention is DMD.

Combination therapies are also contemplated by the invention. Combination as used herein includes both simultaneous treatment and sequential treatments. Combinations of methods of the invention with standard medical treatments (*e.g.*, corticosteroids) are specifically contemplated, as are combinations with novel therapies.

Administration of an effective dose of the compositions may be by routes standard in the art including, but not limited to, intramuscular, parenteral, intravenous, oral, buccal, nasal, pulmonary, intracranial, intraosseous, intraocular, rectal, or vaginal. Route(s) of administration and serotype(s) of AAV components of the rAAV (in particular, the AAV ITRs and capsid protein) of the invention may be chosen and/or matched by those skilled in the art taking into account the infection and/or disease state being treated and the target cells/tissue(s) that are to express the micro-dystrophin protein.

The invention provides for local administration and systemic administration of an effective dose of rAAV and compositions of the invention. For example, systemic administration is administration into the circulatory system so that the entire body is affected. Systemic administration includes enteral administration such as absorption through the gastrointestinal tract and parenteral administration through injection, infusion or implantation.

In particular, actual administration of rAAV of the present invention may be accomplished by using any physical method that will transport the rAAV recombinant vector into the target tissue of an animal. Administration according to the invention includes, but is not limited to, injection into muscle and injection into the bloodstream. Simply resuspending a rAAV in phosphate buffered saline has been demonstrated to be sufficient to provide a vehicle useful for muscle tissue expression, and there are no known restrictions on the carriers or other components that can be co-administered with the rAAV (although compositions that degrade DNA should be avoided in the normal manner with rAAV). Capsid proteins of a rAAV may be modified so that the rAAV is targeted to a particular target tissue of interest such as muscle. See, for example, WO 02/053703, the disclosure of which is incorporated by reference herein. Pharmaceutical compositions can be prepared as injectable formulations or as topical formulations to be delivered to the muscles by transdermal transport. Numerous formulations for both intramuscular injection and transdermal transport have been previously developed and can be used in the practice of the invention. The rAAV can be used with any pharmaceutically acceptable carrier for ease of administration and handling.

In one embodiment of the invention, the AAVrh74.MHCK7.microdystrophin described herein is formulated in a buffer containing 20 mM Tris (pH 8.0), 1mM magnesium chloride (MgCl₂), 200 mM sodium chloride (NaCl), and 0.001% poloxamer 188.

The dose of rAAV to be administered in methods disclosed herein will vary depending, for example, on the particular rAAV, the mode of administration, the treatment goal, the individual, and the cell type(s) being targeted, and may be determined by methods standard in the art. Titers of each rAAV administered may range from about 1x10⁶, about 1x10⁷, about 1x10⁸, about 1x10⁹, about 1x10¹⁰, about 1x10¹¹, about 1x10¹², about 1x10¹³, about 1x10¹⁴, about 2x10¹⁴, or to about 1x10¹⁵ or more DNase resistant particles (DRP) per ml. Dosages may also be expressed in units of viral genomes (vg) (*i.e.,* 1x10⁷ vg, 1x10⁸ vg, 1x10⁹ vg, 1x10¹⁰ vg, 1x10¹¹ vg, 1x10¹² vg, 1x10¹³ vg, 1x10¹⁴ vg, 2x10¹⁴ vg, 1x10¹⁵vg respectively). Dosages may also be expressed in units of viral genomes (vg) per kilogram (kg) of bodyweight (*i.e.,* 1x10¹⁰ vg/kg, 1x10¹¹ vg/kg, 1x10¹² vg/kg, 1x10¹³ vg/kg, 1x10¹⁴ vg/kg, 1.25x10¹⁴ vg/kg, 1.5x10¹⁴ vg/kg, 1.75x10¹⁴ vg/kg, 2.0x10¹⁴ vg/kg, 2.25x10¹⁴ vg/kg, 2.5x10¹⁴ vg/kg, 2.75x10¹⁴ vg/kg, 3.0x10¹⁴ vg/kg, 3.25x10¹⁴ vg/kg, 3.5x10¹⁴ vg/kg, 3.75x10¹⁴ vg/kg, 4.0x10¹⁴ vg/kg, 1x10¹⁵ vg/kg respectively). Methods for titering AAV are described in Clark et al., Hum. Gene Ther., 10: 1031-1039 (1999).

In particular, actual administration of rAAV of the present invention may be accomplished by using any physical method that will transport the rAAV recombinant vector into the target tissue of an animal. Administration according to the invention includes, but is not limited to, injection into muscle and injected into the bloodstream. Simply resuspending a rAAV in phosphate buffered saline has been demonstrated to be sufficient to provide a vehicle useful for muscle tissue expression, and there are no known restrictions on the carriers or other components that can be co-administered with the rAAV (although compositions that degrade DNA should be avoided in the normal manner with rAAV). Capsid proteins of a rAAV may be modified so that the rAAV is targeted to a particular target tissue of interest such as muscle. See, for example, WO 02/053703, the disclosure of which is incorporated by reference herein. Pharmaceutical compositions can be prepared as injectable formulations or as topical formulations to be delivered to the muscles by transdermal transport. Numerous formulations for both intramuscular injection and transdermal transport have been previously developed and can be used in the practice of the invention. The rAAV can be used with any pharmaceutically acceptable carrier for ease of administration and handling.

For purposes of intramuscular injection, solutions in an adjuvant such as sesame or peanut oil or in aqueous propylene glycol can be employed, as well as sterile aqueous solutions. Such aqueous solutions can be buffered, if desired, and the liquid diluent first rendered isotonic with saline or glucose. Solutions of rAAV as a free acid (DNA contains acidic phosphate groups) or a pharmacologically acceptable salt can be prepared in water suitably mixed with a surfactant such as hydroxpropylcellulose. A dispersion of rAAV can also be prepared in glycerol, liquid polyethylene glycols and mixtures thereof and in oils. Under ordinary conditions of storage and use, these preparations contain a preservative to prevent the growth of microorganisms. In this connection, the sterile aqueous media employed are all readily obtainable by standard techniques well-known to those skilled in the art.

The pharmaceutical carriers, diluents or excipients suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating actions of microorganisms such as bacteria and fungi. The carrier can be a solvent or dispersion medium containing, for example, water, ethanol, polyol (for example, glycerol, propylene glycol, liquid polyethylene glycol and the like), suitable mixtures thereof, and vegetable oils. The proper fluidity can be maintained, for example, by the use of a coating such as lecithin, by the maintenance of the required particle size in the case of a dispersion and by the use of surfactants. The prevention of the action of microorganisms can be brought about by various antibacterial and antifungal agents, for example, parabens, chlorobutanol, phenol, sorbic acid, thimerosal and the like. In many cases it will be preferable to include isotonic agents, for example, sugars or sodium chloride. Prolonged absorption of the injectable compositions can be brought about by use of agents delaying absorption, for example, aluminum monostearate and gelatin.

Sterile injectable solutions are prepared by incorporating rAAV in the required amount in the appropriate solvent with various other ingredients enumerated above, as required, followed by filter sterilization. Generally, dispersions are prepared by incorporating the sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze drying technique that yield a powder of the active ingredient plus any additional desired ingredient from the previously sterile-filtered solution thereof.

Transduction with rAAV may also be carried out *in vitro.* In one embodiment, desired target muscle cells are removed from the subject, transduced with rAAV and reintroduced into the subject. Alternatively, syngeneic or xenogeneic muscle cells can be used where those cells will not generate an inappropriate immune response in the subject.

Suitable methods for the transduction and reintroduction of transduced cells into a subject are known in the art. In one embodiment, cells can be transduced *in vitro* by combining rAAV with muscle cells, *e.g.,* in appropriate media, and screening for those cells harboring the DNA of interest using conventional techniques such as Southern blots and/or PCR, or by using selectable markers. Transduced cells can then be formulated into pharmaceutical compositions, and the composition introduced into the subject by various techniques, such as by intramuscular, intravenous, subcutaneous and intraperitoneal injection, or by injection into smooth and cardiac muscle, using *e.g.,* a catheter.

Transduction of cells with rAAV of the invention results in sustained expression of the micro-dystrophin protein. The present invention thus provides methods of administering/delivering rAAV which express micro-dystrophin protein to an animal, preferably a human being. These methods include transducing tissues (including, but not limited to, tissues such as muscle, organs such as liver and brain, and glands such as salivary glands) with one or more rAAV of the present invention. Transduction may be carried out with gene cassettes comprising tissue specific control elements. For example, one embodiment of the invention provides methods of transducing muscle cells and muscle tissues directed by muscle specific control elements, including, but not limited to, those derived from the actin and myosin gene families, such as from the myoD gene family (See Weintraub et al., Science, 251: 761-766 (1991)), the myocyte-specific enhancer binding factor MEF-2 (Cserjesi and Olson, Mol Cell Biol 11: 4854-4862 (1991)), control elements derived from the human skeletal actin gene (Muscat et al., Mol Cell Biol, 7: 4089-4099 (1987)), the cardiac actin gene, muscle creatine kinase sequence elements (See Johnson et al., Mol Cell Biol, 9:3393-3399 (1989)) and the murine creatine kinase enhancer (mCK) element, control elements derived from the skeletal fast-twitch troponin C gene, the slow-twitch cardiac troponin C gene and the slow-twitch troponin I gene: hypoxia-inducible nuclear factors (Semenza et al., Proc Natl Acad Sci USA, 88: 5680-5684 (1991)), steroid-inducible elements and promoters including the glucocorticoid response element (GRE) (See Mader and White, Proc. Natl. Acad. Sci. USA 90: 5603-5607 (1993)), and other control elements.

Muscle tissue is an attractive target for *in vivo* DNA delivery, because it is not a vital organ and is easy to access. The invention contemplates sustained expression of microdystrophin from transduced myofibers.

By "muscle cell" or "muscle tissue" is meant a cell or group of cells derived from muscle of any kind (for example, skeletal muscle and smooth muscle, *e.g.* from the digestive tract, urinary bladder, blood vessels or cardiac tissue). Such muscle cells may be differentiated or undifferentiated, such as myoblasts, myocytes, myotubes, cardiomyocytes and cardiomyoblasts.

The term "transduction" is used to refer to the administration/delivery of the coding region of the micro-dystrophin to a recipient cell either *in vivo* or *in vitro,* via a replication-deficient rAAV of the invention resulting in expression of micro-dystrophin by the recipient cell.

Thus, the invention provides methods of administering an effective dose (or doses, administered essentially simultaneously or doses given at intervals) of rAAV that encode micro-dystrophin to a subject in need thereof.

The following EXAMPLES are provided by way of illustration and not limitation. Described numerical ranges are inclusive of each integer value within each range and inclusive of the lowest and highest stated integer.

### EXAMPLES

### Example 1

### A) Generation of the AAVrh74.MHCK7.micro-dystrophin construct

The AAVrh74.MHCK7.micro-dystrophin plasmid contains a human micro-dystrophin cDNA expression cassette flanked by AAV2 inverted terminal repeat sequences (ITR) (see Fig. 1). The micro-dystrophin construct was characterized by an in-frame rod deletion (R4-R23), while hinges 1, 2 and 4 and cysteine rich domain remain producing a 138 kDa protein. The expression of the micro-dystrophin protein (3579 bp) was guided by a MHCK7 promoter (792 bp). The plasmid was constructed from the rAAV.MCK.micro-dystrophin plasmid by removing the MCK promoter and inserting the MHCK7 promoter. After the core promoter, the 53 bp endogenous mouse MCK Exon1 (untranslated) is present for efficient transcription initiation, followed by the SV40 late 16S/19S splice signals (150 bp) and a small 5'UTR (61 bp). The intron and 5' UTR are derived from plasmid pCMVβ (Clontech). The micro-dystrophin cassette had a consensus Kozak immediately in front of the ATG start and a small 53 bp synthetic polyA signal for mRNA termination. The human micro-dystrophin cassette contained the (R4-R23/Δ71-78) domains as previously described by Harper et al. (Nature Medicine 8, 253-261 (2002)). The complementary DNA was codon optimized for human usage and synthesized by GenScript (Piscataway, NJ) (Mol Ther 18, 109-117 (2010)). The only viral sequences included in this vector were the inverted terminal repeats of AAV2, which are required for both viral DNA replication and packaging. The micro-dystrophin cassette has a small 53 bp synthetic polyA signal for mRNA termination.

Previous studies have validated cardiac expression using MHCK7 promoter (Salva et al. Mol Ther 15, 320-329 (2007) and AAVrh74 achieving skeletal, diaphragm, and cardiac muscle expression (Sondergaard et al. Annals of clinical and Transl Neurology 2, 256-270 (2015)), The sequence of construct of Fig. 1 was encapsidated into AAVrh.74 virions. The molecular clone of the AAVrh.74 serotype was cloned from a rhesus macaque lymph node and is discussed in in Rodino-Klapac et al. Journal of Translational medicine 5, 45 (2007).

**Table 1 shows the molecular features of the plasmid AAVrh74.MHCK7.micro-dystrophin (SEQ ID NO: 3)**

| **Table 1. Molecular Features of plasmid rAAV.MHCK7.micro-dystrophin** | | | | |
|---|---|---|---|---|
| TYPE | START | END | NAME | DESCRIPTION |
| REGION | 55 | 182 | 5' ITR | Wild-type AAV2 inverted terminal repeat |
| REGION | 244 | 1035 | MHCK7 | Mouse myosin heavy chain complex - E box muscle creatine kinase fusion enhancer/promoter |
| REGION | 1045 | 1194 | Chimeric intron | 5' donor site from human β-globin gene and the branchpoint and 3' splice acceptor site from IgG heavy chain variable region |
| GENE | 1205 | 4783 | huDys cDNA | Human micro-dystrophin cDNA |
| REGION | 4786 | 4838 | PolyA | Synthetic PolyA |
| REGION | 4894 | 5021 | 3' ITR | Wild-type AAV2 inverted terminal repeat |
| GENE | 6760 | 7619 | AmpR | β-lactamase gene |
| REGION | 7823 | 8442 | Ori | Plasmid origin of replication |

### B) Generation of the AAVrh74.MHCK7.micro-dystrophin construct from and plasmid encoding Kanamycin (Kan) resistance

Cloning of MHCK7.µDys.KAN was achieved by isolating the MHCK7.µDys fragment from an MHCK7. µDys.AMP plasmid and the Kanamycin Backbone, and annealing them using the NEBuilder cloning workflow. The MHCK7.µDys fragment was isolated via restriction enzyme digestion with SnaBI. The digestion was performed in a 50µL total reaction in 1x CutSmart Buffer (NEB) and 1µL SnaBI, at 37°C for 1 hour. The resulting fragment was isolated via electrophoresis using a 1% Agarose gel, running at 105 volts for 1.5 hours. The band corresponding to the MHCK7.µDys insert was cut out and purified using a gel purification kit (Macherey-Nagel). The resulting fragment had a DNA concentration of 10ng/µL. The Kan backbone fragment was isolated via XbaI restriction enzyme digestion in a 50µL reaction with 1x CutSmart Buffer (NEB) and 1µL XbaI, at 37°C for 1 hour. The resulting fragment was isolated via electrophoresis using a 1% Agarose gel, running at 105 volts for 1.5 hours. The band corresponding to the Kan Backbone was cut out and purified via gel purification kit (Macherey-Nagel). The resulting fragment had a DNA concentration of 8.1ng/µL. The two fragments were annealed using the NEB Builder cloning workflow, which has the ability to join two fragments with overlapping sequences. The NEBuilder cloning reaction was performed per manufacturer protocol at 50°C for 15 minutes, using a 1:1 ratio of MHCK7. µDys to Kanamycin backbone in 1x NEBuilder HiFi DNA Assembly Master Mix for a total reaction volume of 20µL. The resulting clone was transformed into NEB^{®} Stable Competent E. coli (C3040) by adding 2.5µL cloning product to the cells followed by 30 minutes on ice, then 30 seconds at 42°C and an additional 5 minutes on ice. After transformation, 950µL of outgrowth media was added to the cells and allowed to grow at 30°C for 1.5 hours, shaking at 225rpm. Following outgrowth, 450µL of these cells was plated on a 50µg/mL kanamycin LB agar plate and incubated overnight at 30°C in a dry incubator. A colony was picked from this plate and grown up overnight in LB containing 50µg/mL kanamycin. DNA was isolated from 3mL of this culture using QIAprep^{®} Spin Miniprep Kit (Qiagen). This DNA was used to confirm the cloning product. The cloning product was confirmed via restriction enzyme digestion with PmeI, MscI, and SmaI followed by gel electrophoresis. The cloning product was additionally confirmed via sequencing. The resultant plasmid is set forth in SEQ ID NO:8, and shown in Figures 14 and 15. The sequence of construct of Fig. 13 which corresponds to that of SEQ ID NO:9, and nucleotides 1-4977 of SEQ ID NO: 8, was encapsidated into AAVrh.74 virions as described above.

### Example 2

### Systemic Gene Delivery Clinical Trial for Duchenne Muscular Dystrophy

This is a single-dose controlled trial using the rAAVrh74.MHCK7.micro-dystrophin of SEQ ID NO: 3, nucleotides 55-5021, for DMD subjects. Cohort A will include six subjects of ages 3 months to 3 years, and Cohort B will include six subjects of ages 4 years to 7 years old. All subjects will receive intravenous micro-dystrophin vector (2x10¹⁴ vg/kg in 10mL/kg). The rAAVrh74.MHCK7.micro-dystrophin is formulated in a buffer containing 20 mM Tris (pH 8.0), 1mM magnesium chloride (MgCl₂), 200 mM sodium chloride (NaCl), and 0.001% poloxamer 188.

In the study, the rAAVrh74.MHCK7.micro-dystrophin was infused via peripheral arm vein so that it can reach all the muscles in the body. Six DMD subjects ages 3 months to 3 years in Cohort A, and six DMD subjects ages 4 years to age 7 years in Cohort B, were enrolled. All subjects received intravenous micro-dystrophin vector (2x10¹⁴ vg/kg in 10mL/kg). The encapsilated vector genome titer for the administered dose was determined using quantitative PCR using a Prism 7500 Taqman detector system (PE Applied Biosystems) with primers directed against the MHCK7 promoter compared to a supercoiled DNA plasmid standard (Pozsgai et al. Mol. Ther. 25(4): 855- 869, 2017).

Subjects received infusions over 1 hour in the Pediatric Intensive Care Unit (PICU) at Nationwide Children's Hospital. Before the gene therapy, a muscle biopsy was performed at the screening visit. Subjects will have a second muscle biopsy to determine if the gene allowed for replacement of the missing dystrophin protein at 90 days post-delivery. After the gene transfer, patients were carefully monitored for any side effects of the treatment. This monitoring included blood and urine tests, as well as physical examination during the screening visits and on days 0, 1, 7, 14, 30, 60, 90, and 180, and at months 9, 12, 18, 24, 30 and 36 to make sure that there are no side effects from the gene injection.

The subjects of Cohort A (n=6) were between 3 months to 3 years of age, and received intravenous rAAVrh74.MHCK7.micro-dystrophin vector (2x10¹⁴ vg/kg in 10 mL/kg). One-day prior to gene transfer subjects in Cohort A were started on prednisone or deflazacort 1 mg/kg and maintained for 30 days while monitoring immune response. If negative at day 30, steroids were weaned over 1 week. If T cell response to AAV or micro-dys was >125 SFC/106 PBMCs, steroids were maintained until levels drop below this threshold.

The subjects of Cohort B (n=6) were between 4 years to 7 years of age, and received intravenous rAAVrh74.MHCK7.micro-dystrophin vector (2x10¹⁴ vg/kg in 10 mL/kg). These subjects were maintained on stable dose of corticosteroids throughout trial but may have been increased for short time if T cell response to AAV or micro-dystrophin was >125 SFC/106 PBMCs.

### Eligibility Criteria

Inclusion criteria for the study were as follows:
- Age of enrollment: Cohort A: 3 months to 7 years of age and Cohort B: between 4-7 years of age, inclusive.
- Molecular characterization of the DMD gene with frameshift (deletion or duplication), or premature stop codon mutation between exons 18 to 58.
- CK elevation >1000 U/L
- Cohort A subjects: below average on the Bayley-III motor assessment for gross motor defined as a scaled score of ≤9
- Cohort B: Below average on 100 meter timed test defined as < 80% predicted
- Males of any ethnic group.
- Ability to cooperate with motor assessment testing.
- Cohort A subjects: No previous treatment with corticosteroids.
- Cohort B subjects: Stable dose equivalent of oral corticosteroids for at least 12 weeks prior to screening and the dose is expected to remain constant (except for modifications to accommodate changes in weight) throughout the stud

Exclusion criteria for the study were as follows:
- Active viral infection based on clinical observations.
- Signs of cardiomyopathy, including echocardiogram with ejection fraction below 40%.
- Serological evidence of HIV infection, or Hepatitis B or C infection.
- Diagnosis of (or ongoing treatment for) an autoimmune disease.
- Abnormal laboratory values considered clinically significant
- Concomitant illness or requirement for chronic drug treatment that in the opinion of the PI creates unnecessary risks for gene transfer.
- Subjects with AAVrh74 or AAV8 antibody titers > 1:400 as determined by ELISA immunoassay.
- Medical condition or extenuating circumstance that, in the opinion of the investigator, might compromise the subject's ability to comply with the protocol required testing or procedures or compromise the subject's wellbeing, safety, or clinical interpretability.
- Severe infection (e.g., pneumonia, pyelonephritis, or meningitis) within 4 weeks before gene transfer visit (enrollment may be postponed).
- Received any investigational medication (other than corticosteroids) or exon skipping medications (including ExonDys 51^{®}), experimental or otherwise, in the last 6 months prior to screening for this study.
- Has had any type of gene therapy, cell based therapy (e.g. stem cell transplantation), or CRISPR/Cas9 therapy.
- Family does not want to disclose patient's study participation with primary care physician and other medical providers.

### Outcome Measures

The primary outcome measure was safety based on number of participants with adverse events (time frame: 3 years). Adverse effects were monitored and scored for severity and relatedness to the study article.

The secondary outcome measures were as follows:
Gross Motor Subtest Scaled (Bayley-III) score (time frame: screening, Day 30-3 Years): The Gross Motor Scaled Score measured motor development. The Bayley-III Gross Motor Subtest was scored for Cohort A on every follow up visit starting at Day 30 through 3 years. Any subject that was 43-47 months of age, inclusive, at time of screening had the scaled score calculated compared to normative data for 42 month old children. The Bayley-III provided normative data for children 1-42 months of age.

Physical Therapy Assessments The 100 Meter Timed Test (100m) (time frame: screening, Day 30-3 Years): The 100m was the primary motor outcome for Cohort B. The 100 Meter Timed Test was an exploratory outcome initiated for Cohort A as soon as the child was 3 years of age.

Physical Therapy Assessments North Star Ambulatory Assessment (NSAA) (time frame: screening, Day 30-3 Years): The North Star Ambulatory Assessment (NSAA) was an exploratory outcome initiated for Cohort A as soon as the child was four years of age and for cohort B. The NSAA measures the quality of ambulation in young boys with Duchenne Muscular Dystrophy.

Physical Therapy Assessments Timed Up and Go modified for children (TUG) (time frame: screening, Day 30-3 Years): Exploratory outcomes for Cohort B included the Timed Up and Go modified for children (TUG).

Physical Therapy Assessments Ascend and Descend of 4 steps (time frame: screening, Day 30-3 Years): Exploratory outcomes for Cohort B will include ascend and descend of 4 steps.

Physical Therapy Assessments Hand Held Dynamometry (HHD) (time frame: screening, Day 30-3 Years): Exploratory outcomes for Cohort B included hand held dynamometry (HHD) for knee extensors and knee flexors, and elbow flexors and elbow extensors.

Micro-dystrophin gene expression quantification by immunofluorescence (time frame: screening, Day 90): Micro-dystrophin gene expression levels were quantified by immunofluorescence and compared in pre and post muscle biopsies.

Micro-dystrophin gene expression quantification by western blot (time frame: screening, Day 90): Micro-dystrophin gene expression levels were quantified by western blot analysis and compared in pre and post muscle biopsies.

A decrease in CK following gene therapy (time frame: 3 years): Decrease in CK levels in circulating blood.

Cardiac magnetic resonance imaging (at 1 year).

### Micro-dystrophin Gene Expression

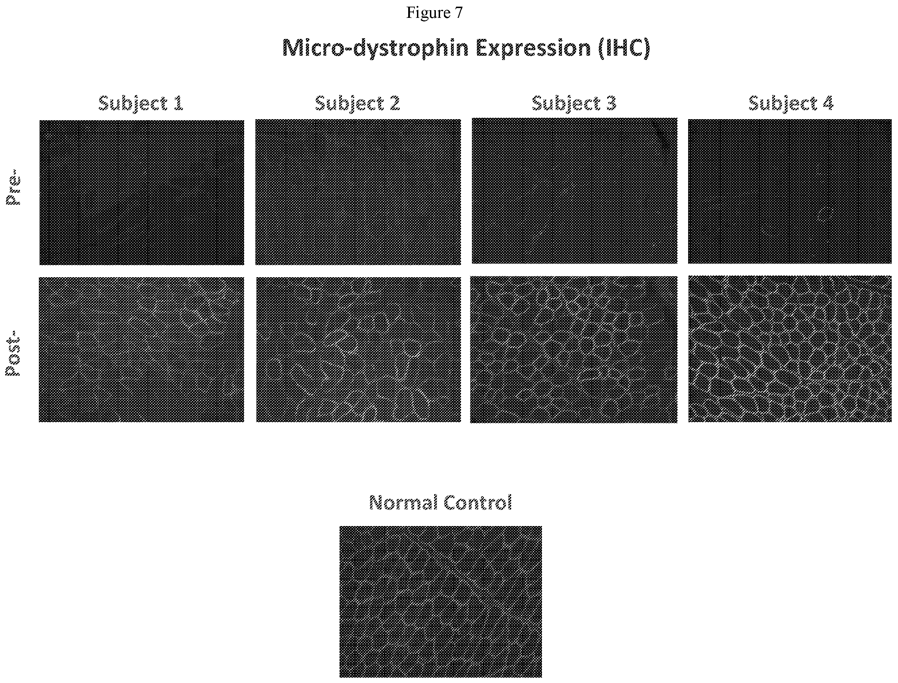

Change from Baseline in micro-dystrophin expression via immunofluorescence (IF) fiber intensity was analyzed and quantitated. As shown in Figure 7, Subject 1 (age 5) demonstrated 78% expression, of micro-dystrophin protein in the muscle fibers of the gastrocnemius muscle biopsy after administration of the rAAVrh74.MHCK7.micro-dystrophin, Subject 2 (age 4) demonstrated 73.5% expression of micro-dystrophin protein in the muscle fibers of the gastrocnemius muscle biopsy after administration of the rAAVrh74.MHCK7.micro-dystrophin, and Subject 3 (age 6) demonstrated 77.0% expression of micro-dystrophin protein in the muscle fibers of the gastrocnemius muscle biopsy after administration of the rAAVrh74.MHCK7.micro-dystrophin. Subject 4 (age 4) demonstrated 96.2% expression of micro-dystrophin in the muscle fibers of the gastrocnemius muscle biopsy after administration of the rAAVrh74.MHCK7.micro-dystrophin. All patients showed robust expression of transduced micro-dystrophin, which is properly localized to the muscle sarcolemma, as measured by immunohistochemistry. Mean gene expression, as measured by percentage of micro-dystrophin positive fibers was 76.2% and the mean intensity of the fibers was 74.5% compared to normal control.

| Subject | Mean Intensity | Percentage of Dystrophin-Positive Fibers |
|---|---|---|
| 1 | 82.0% | 78.0% |
| 2 | 59.0% | 73.5% |
| 3 | 83.0% | 77.0% |
| 4 | 160.0% | 96.2% |
| Mean | 96.0% | 81.2% |

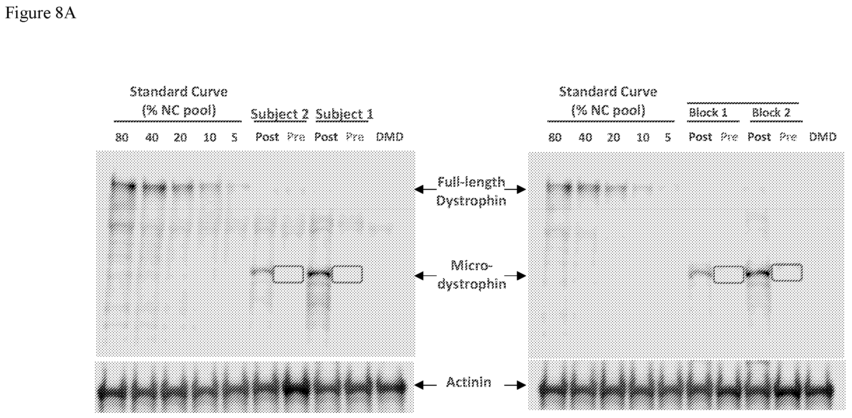

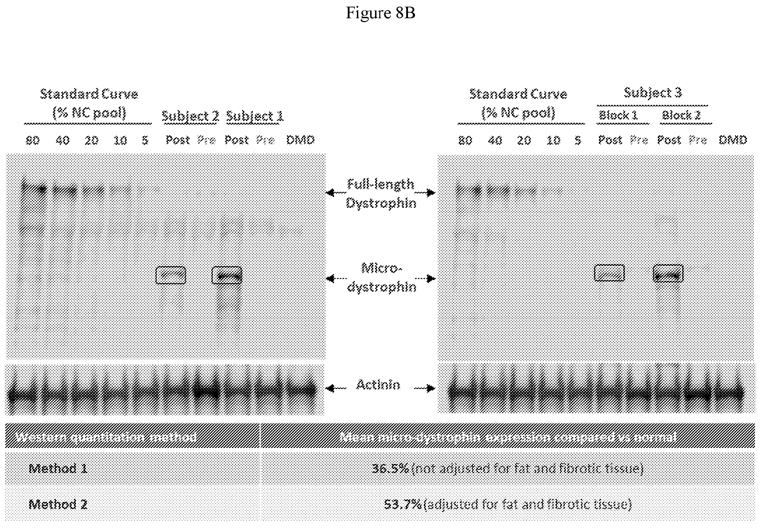

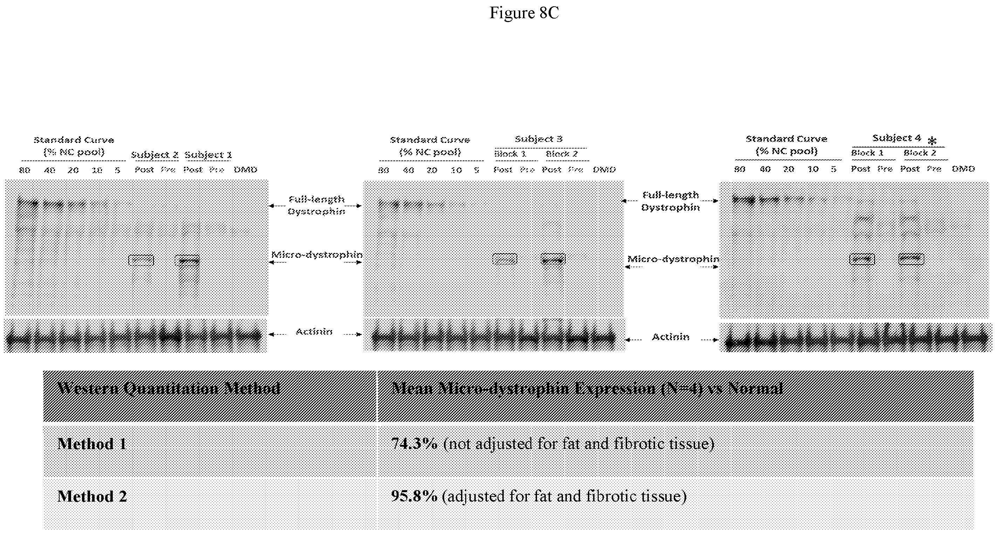

The change in micro-dystrophin gene expression from Baseline to Day 60 was also assessed by quantitating micro-dystrophin protein expression as measured by Western blot of biopsied muscle tissue. As shown in Figures 8A and 8B, Western blot analysis detected micro-dystrophin protein expression in Subject 1 (age 5), Subject 2 (age 4) and Subject 3 (age 6). Figure 8C provides the Western Blot analysis detecting micro-dystrophin protein expression in Subject 4 (age 4). All post-treatment biopsies showed robust levels of micro-dystrophin as measured by Western blot, with a mean for Subjects 1-4 of 74.3 compared to normal utilizing method 1, and 95.8% compared to normal pursuant to method 2 that adjusts for fat and fibrotic tissue.

For each subject, the vector genome copy per nucleus of the muscle fibers were measured. As shown in Table 2, the vector genome copy per nuclease was greater than 1 for each of the subjects after administration of rAAVrh74.MHCK7.micro-dystrophin. One copy of the vector indicates approximately 50% expression of the micro-dystrophin gene. A mean of 1.6 vector copies per cell nucleus was measured in Subjects 1-3, consistent with the high micro-dystrophin expression levels observed. When the values for Subject 4 were included, the mean vectors copies/µg DNA is >10⁵ with a mean of 3.3 vector copies per cell nucleus.

**Table 2**

| Subject | Vector copies/µg DNA | Copies per Nuclei |
|---|---|---|
| 1 | >10⁵ | 1.7 |
| 2 | >10⁵ | 1.3 |
| 3 | >10⁵ | 1.9 |

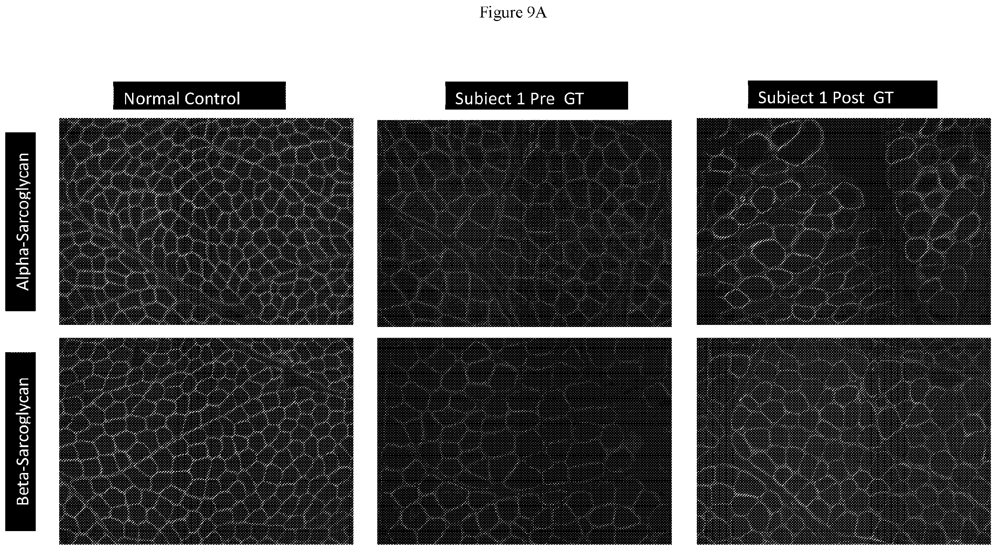

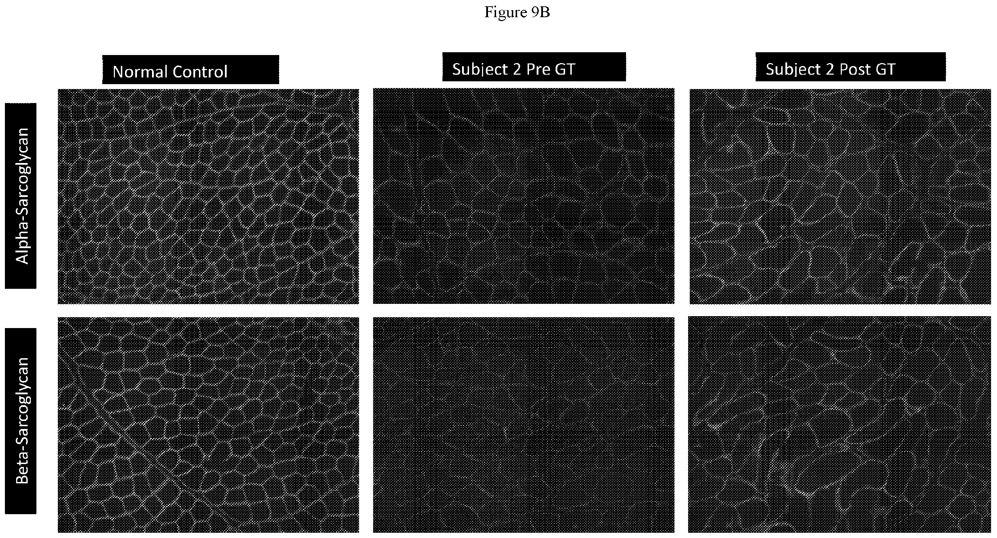

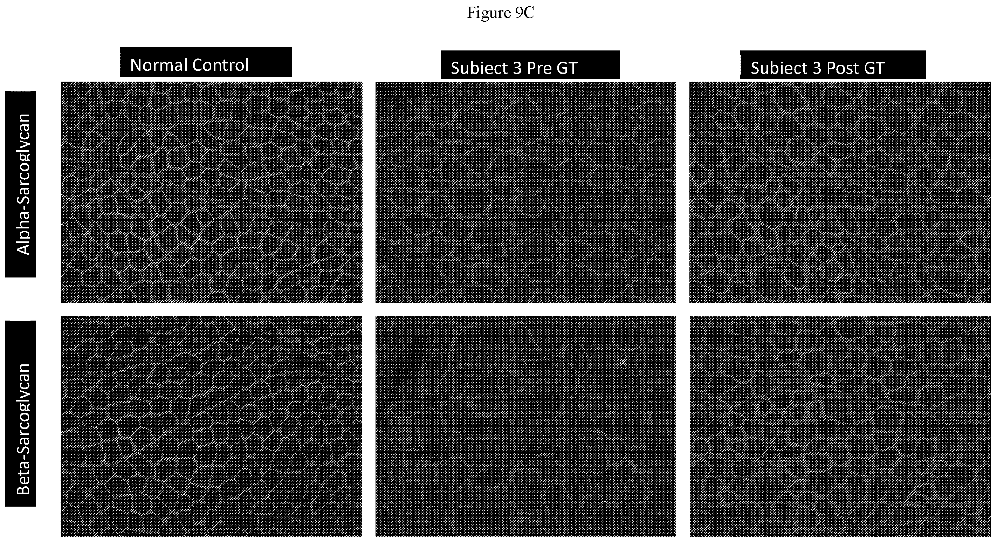

The protein levels of alpha-sarcoglycan and beta-sarcoglycan in muscle biopsy tissue were measured by immunohistochemistry before and after administration of rAAVrh74.MHCK7.micro-dystrophin. Administration of rAAVrh74.MHCK7 also resulted in upregulation of the DAPC proteins in the subjects. As shown in Figure 9, expression of alpha-sarcoglycan and beta-sarcoglycan in muscle biopsy tissue was increased compared to the level so these proteins in muscle biopsies before administration of rAAVrh74.MHCK7 in Subjects 1 (Fig. 9A), Subject 2 (Fig. 9B) and Subject 3 (Fig. 9C).

### Circulating Serum CK Levels

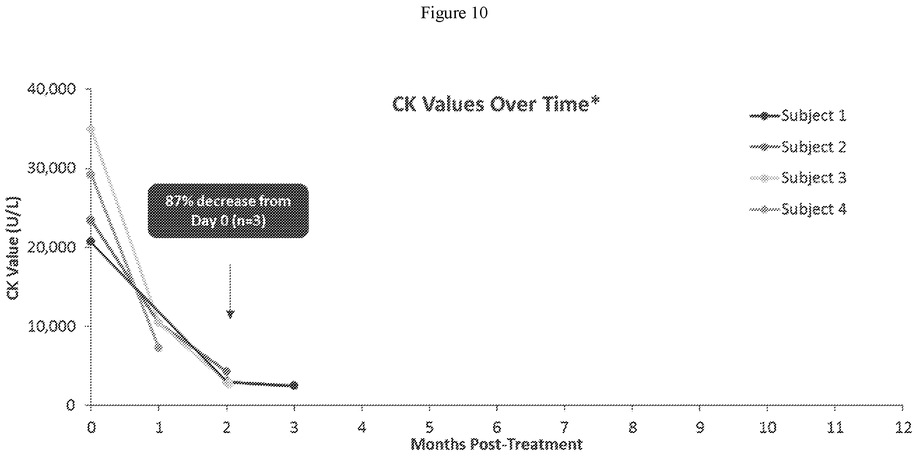

Blood samples were obtained every 30 days after administration intravenous infusion of rAAVrh74.MHCK7.micro-dystrophin vector (2x10¹⁴ vg/kg in 10 mL/kg). CK levels were measured at each visit and compared to the baseline level obtained before administration of the rAAVrh74.MHCK7.micro-dystrophin (Visit Day 0). Baseline serum CK levels (Units/Liter) are provided in Table 3 below. As shown in Figure 10, the level of circulating serum CK decreased about 87%, 2 months after administration of rAAVrh74.MHCK7.micro-dystrophin. All subjects showed significant decreases of serum creatine kinase (CK) levels, with a mean reduction of CK of over 87% at 2 months' post-treatment (n=3). CK is an enzyme associated with muscle damage and patients with DMD uniformly exhibit high levels of CK. Indeed, significantly elevated CK is often used as a preliminary diagnosis tool for DMD, which is then followed by confirmatory genetic testing.

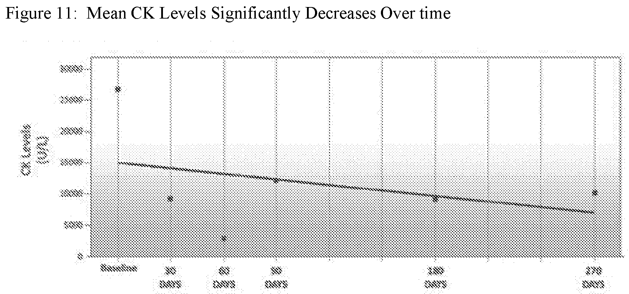

Table 4 and Figure 10 provide the CK levels for each subject. Figure 11 provides the mean CK levels over time, and demonstrates that the mean CK levels significantly decrease over time after administration of rAAVrh74.MHCK7.micro-dystrophin. The mean baseline CK level of 27,064 U/L (Mean for Table 3) is decreased by about 63% to a mean of 9,982 U/L (Mean, Day 270, Table 4).

**Table 3**

| Subject | Age (years) | CK Levels at Baseline Units/Liter (U/L) |
|---|---|---|
| 1 | 5 | 20691 |
| 2 | 4 | 23414 |
| 3 | 6 | 34942 |
| 4 | 4 | 29210 |

**Table 4: Change in CK levels from Baseline to Day 270**

| **Subject** | **Baseline** | **Day 30** | **Day 60** | **Day 90** | **Day 180** | **Day 270** | **Day 360** |
|---|---|---|---|---|---|---|---|
| 1 | | - | 2984 | 2444 | 18476 | 6317 | - |
| 2 | 23414 | 10427 | 4283 | 41920 | 6209 | 10494 | - |
| 3 | 34942 | 10430 | 2966 | 2546 | 9650 | 18855 | 6410 |
| 4 | 29210 | 7215 | 908 | 1382 | 2580 | 4262 | - |

### Efficacy Assessment

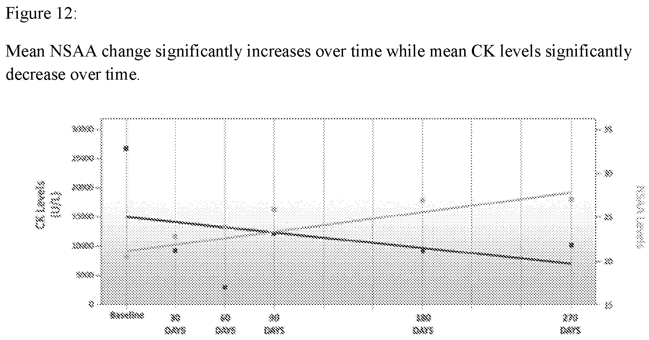

In addition to micro-dystrophin and CK levels, efficacy was measured by the following functional tests: Time to Rise from the floor, Ascend 4 steps, North Star Ambulatory Assessment (NSAA), Time to Rise Test, 4 Stairs Up Test, 10 Meter Timed Test (10m), and 100 Meter Timed Test (100m). The data is provided in Tables 5 and 6 below, and this data demonstrates consistent durable improvement at 9 months after administration of rAAVrh74.MHCK7.micro-dystrophin. The NSAA improvement over time is also provided in Figure 12.

**Table 5: NSAA Change from Baseline to Day 270**

| **Subject** | **Baseline** | **Day 30** | **Day 60** | **Day 90** | **Day 180** | **Day 270** | **Change from Baseline** |
|---|---|---|---|---|---|---|---|
| 1 | 18 | 22 | 24 | 23 | 25 | 26 | 8 |
| 2 | 19 | 21 | 23 | 25 | 27 | 27 | 8 |
| 3 | 26 | 28 | 28 | 30 | 30 | 28 | 2 |
| 4 | 19 | 20 | 20 | 25 | 25 | 27 | 8 |
| Mean Improvement | 20.5 | 22.75 | 23.75 | 25.75 | 26.75 | 27 | 6.5 |

**Table 6: Change from Baseline to Day 270**

| Subject | Assessment | NSAA | Time to Rise | 4 Stairs Up | 100m (sec) | 10 m |
|---|---|---|---|---|---|---|
| 1 | Baseline | 18 | 3.7 | 3.4 | 49.3 | 5.1 |
| | Day 270 | 26 | 3.0 | 2.3 | 43.2 | 4.3 |
| 2 | Baseline | 19 | 3.0 | 3.8 | 49.9 | 4.3 |
| | Day 180 | 27 | 3.7 | 2.6 | 48.6 | 3.9 |
| | Day 270 | 27 | 3.3 | 2.7 | 50.3 | |
| 3 | Baseline | 26 | 3.9 | 1.9 | 59.3 | 4.7 |
| | Day 180 | 30 | 3.4 | 1.8 | 48.4 | 4.1 |
| | Day 270 | 28 | 2.8 | 1.9 | 50.7 | |
| 4 | Baseline | 19 | 4.1 | 4.8 | 67.2 | 5.4 |
| | Day 90 | 25 | 2.3 | 2.2 | 50.7 | 4.4 |
| | Day 270 | 27 | 2.4 | 2.2 | 49.7 | |
| Average | Change from Baseline | 6.5 point improvement | .8 sec. improvement | 1.2 sec. improvement | 7.95 sec. improvement | 14% improvement |

### Safety Assessment

No serious adverse events (SAEs) were observed in the study. Three subjects had elevated gamma-glutamyl transferase (GGT) that resolved with increased steroids within a week and returned to baseline levels. There were no other clinically significant laboratory findings. Patients had transient nausea generally during the first week of therapy coincident with increased steroid dosing. This did not correlate with liver enzyme elevations or any other abnormality.

### Example 3

### Randomized Double-Blind Placebo Controlled Systemic gene delivery Phase I/IIa clinical trial

This is a randomized double-blind single-dose trial using rAAVrh74.MHCK7.micro-dystrophin for DMD subjects. The study includes twenty-four subjects ages 4 to 7 years old. Subjects are randomized to treatment or placebo at the time of enrollment. Twelve subjects receive intravenous rAAVrh74.MHCK7.micro-dystrophin vector (2x10¹⁴ vg/kg in approximately 10mL/kg) and twelve subjects will receive 10 mL/kg placebo (lactated ringers). Placebo subjects will roll over to treatment which will be given in the same manner as the 12 previously treated subjects one year after the last treated subject is dosed. Subjects receive infusions of rAAV carrying micro-dystrophin or lactated ringers over approximately 1 hour. Pre and post-treatment (90 Day) needle muscle biopsies are done on gastrocnemius muscles.

The primary objective of this study is the assessment of the safety of intravenous administration of rAAVrh74.MHCK7.micro-dystrophin for DMD subjects via peripheral limb vein. Safety endpoints are assessed by changes in hematology, serum chemistry, urinalysis, immunologic response to rAAVrh74 and micro-dystrophin, and reported history and observations of symptoms. Dystrophin gene expression serves as a primary outcome measure along with safety. Quantification is carried out using validated immunofluorescence and immunoblot assays. A decrease in CK following gene therapy serves as a secondary outcome. Efficacy is measured by the following functional tests: Time to Rise, Ascend 4 steps, North Star Ambulatory Assessment (NSAA), 10 Meter Timed Test (10m), 100 Meter Timed Test (100m). Exploratory measures include hand-held dynamometry (HHD) for knee extensors and knee flexors, and elbow flexors and elbow extensors.

Inclusion criteria for the study are as follows:
- Age of enrollment: between 4-7 years of age, inclusive.
- Molecular characterization of the DMD gene with frameshift (deletion or duplication), or premature stop codon mutation between exons 18 to 58.
- Indication of symptomatic muscular dystrophy: CK elevation >1000 U/L and Below mean percent predicted time on 100 meter walk test
- Males of any ethnic group will be eligible.
- Ability to cooperate with motor assessment testing.
- Stable dose equivalent of oral corticosteroids for at least 12 weeks prior to screening and the dose is expected to remain constant (except for potential modifications to accommodate changes in weight) throughout the study.

Exclusion criteria for the study are as follows:
- Active viral infection based on clinical observations.
- Signs of cardiomyopathy, including echocardiogram with ejection fraction below 40%.
- Serological evidence of HIV infection, or Hepatitis B or C infection.
- Diagnosis of (or ongoing treatment for) an autoimmune disease.
- Abnormal laboratory values considered clinically significant (GGT > 3XULN, bilirubin ≥ 3.0 mg/dL, creatinine ≥ 1.8 mg/dL, Hgb < 8 or > 18 g/Dl; WBC > 18,500 per cmm), platelets ≤ 50,000.
- Concomitant illness or requirement for chronic drug treatment that in the opinion of the PI creates unnecessary risks for gene transfer.
- Subjects with AAVrh74 or AAV8 antibody titers > 1:400 as determined by ELISA immunoassay. If endpoint titer is positive at screening, testing may be repeated prior to exclusion.
- Has a medical condition or extenuating circumstance that, in the opinion of the investigator, might compromise the subject's ability to comply with the protocol required testing or procedures or compromise the subject's wellbeing, safety, or clinical interpretability.
- Severe infection (e.g., pneumonia, pyelonephritis, or meningitis) within 4 weeks before gene transfer visit (enrollment may be postponed).
- Has received any investigational medication (other than corticosteroids) or exon skipping medications (including ExonDys 51^{®}), experimental or otherwise, in the last 6 months prior to screening for this study.
- Has had any type of gene therapy, cell based therapy (e.g. stem cell transplantation), or CRISPR/Cas9 therapy.
- Family does not want to disclose patient's study participation with primary care physician and other medical providers.

### Efficacy Assessments

Dystrophin gene expression serves as a primary outcome measure along with safety. Quantification is carried out using validated immunofluorescence and immunoblot assays. A decrease in CK following gene therapy serves as a secondary outcome. In addition, efficacy is measured by the following functional tests: Time to Rise from the floor, Ascend 4 steps, North Star Ambulatory Assessment (NSAA), 10 Meter Timed Test (10m), 100 Meter Timed Test (100m)]. Exploratory measures include hand-held dynamometry (HHD) for knee extensors and knee flexors, and elbow flexors and elbow extensors.

Muscle biopsies with ultrasound guidance are used to quantify transgene expression comparing baseline to Day 90. The biopsies are carried out on the same muscle as the original biopsies but on the opposite leg. One year after all subjects have been dosed, placebo crossover subjects will restart the study timeline at visit 1. Placebo subjects will not have the following performed at the second baseline screening: Cardiac MRI and Muscle Biopsy. Placebo subjects undergo a muscle biopsy at Day 90 (total of 3 muscle biopsies). Frozen sections are stained for dystrophin using indirect immunofluorescence (IF). Full slide scanning is performed and micro-dystrophin intensity and percent positive fibers is quantified using validated image scanning and MuscleMapTM analysis algorithm. Muscle morphometrics are performed blinded including fiber size histograms. Blinded frozen muscle biopsy shavings are used to perform quantitative protein analysis for micro-dystrophin using a validated western blot method.

Muscle needle biopsies of the gastrocnemius muscle (unless deemed contraindicated in a specific subject by the PI, in which case the PI will select an alternative muscle to biopsy) are used to quantify micro-dystrophin expression.

### Efficacy Analyses

The primary efficacy endpoint is the change from Baseline to Day 90 in the quantity of micro-dystrophin protein expression as measured by Western blot of biopsied muscle tissue. Treatment group differences for the primary efficacy endpoint are assessed with an analysis of covariance (ANCOVA) model with treatment as the fixed factor and baseline value as the covariate. The Wilcoxon rank-sum test is performed as a supportive analysis. Change from Baseline in micro-dystrophin expression via immunofluorescence (IF) fiber intensity is analyzed similarly.

The supportive efficacy endpoints include change from Baseline to each scheduled assessment of Time to Rise from the floor, Ascend 4 steps, NSAA, 10 Meter Timed Test (10m), 100 Meter Timed Test (100m), and change in CK. Exploratory measures include HHD for knee extensors and knee flexors, and elbow flexors and elbow extensors. Treatment group differences are assessed with ANCOVA model with treatment as the fixed factor and baseline value as the covariate. The Wilcoxon rank-sum test is performed as a supportive analysis.

### Example 4

The trials and studies described in Examples 2 and 3 above are alternatively carried out utilizing the rAAVrh74.MHCK7.micro-dystrophin construct set forth in SEQ ID NO: 9; as set forth in SEQ ID NO: 8, nucleotides 1-4977; or as set forth in SEQ ID NO: 6; nucleotides 56-5022.

### Example 5

### Generation of the pAAV.MCK.micro-dystrophin construct

The pAAV.MCK.micro-dystrophin plasmid was constructed by inserting the MCK expression cassette driving a codon optimized human micro-dystrophin cDNA sequence into the AAV cloning vector psub201 (Samulski et al., J. Virol. 61(10):3096-3101). A muscle-specific regulatory element was included in the construct to drive muscle-specific gene expression. This regulatory element comprised the mouse MCK core enhancer (206 bp) fused to the 351 bp MCK core promoter (proximal). After the core promoter, the construct comprises the 53 bp endogenous mouse MCK Exon1 (untranslated) for efficient transcription initiation, followed by the SV40 late 16S/19S splice signals (97 bp) and a small 5'UTR (61 bp). The intron and 5' UTR was derived from plasmid pCMVβ (Clontech). The micro-dystrophin cassette has a consensus Kozak immediately in front of the ATG start and a small 53 bp synthetic polyA signal for mRNA termination. The human micro-dystrophin cassette contains the (R4-R23/Δ71-78) domains as previously described by Harper et al. Nat. Med. 8(3):253-61, 2002

The pAAV.MCK.micro-dystrophin plasmid contained the human micro-dystrophin cDNA expression cassette flanked by AAV2 inverted terminal repeat sequences (ITR) (see Fig. 5). This sequence was encapsidated into AAVrh.74 virions. The molecular clone of the AAVrh.74 serotype was cloned from a rhesus macaque lymph node and is described in Rodino-Klapac et al. Journal of Tran. Med. 45 (2007).

### REFERENCES

1. Hoffman, E.P., Brown, R.H., Jr. & Kunkel, L.M. Dystrophin: the protein product of the Duchenne muscular dystrophy locus. Cell 51, 919-928 (1987).
2. Straub, V. & Campbell, K.P. Muscular dystrophies and the dystrophin-glycoprotein complex. Curr Opin Neurol 10, 168-175 (1997).
3. Sacco, A., et al. Short telomeres and stem cell exhaustion model Duchenne muscular dystrophy in mdx/mTR mice. Cell 143, 1059-1071 (2010).
4. Wallace, G.Q. & McNally, E.M. Mechanisms of muscle degeneration, regeneration, and repair in the muscular dystrophies. Annu Rev Physiol 71, 37-57 (2009).
5. Zhou, L. & Lu, H. Targeting fibrosis in Duchenne muscular dystrophy. J Neuropathol Exp Neurol 69, 771-776 (2010).
6. Desguerre, I., et al. Endomysial fibrosis in Duchenne muscular dystrophy: a marker of poor outcome associated with macrophage alternative activation. J Neuropathol Exp Neurol 68, 762-773 (2009).
7. DiPrimio, N., McPhee, S.W. & Samulski, R.J. Adeno-associated virus for the treatment of muscle diseases: toward clinical trials. Curr Opin Mol Ther 12, 553-560 (2010).
8. Mendell, J.R., et al. Sustained alpha-sarcoglycan gene expression after gene transfer in limb-girdle muscular dystrophy, type 2D. Ann Neurol 68, 629-638 (2010).
9. Mendell, J.R., et al. Limb-girdle muscular dystrophy type 2D gene therapy restores alpha-sarcoglycan and associated proteins. Ann Neurol 66, 290-297 (2009).
10. Mendell, J.R., et al. A phase 1/2a follistatin gene therapy trial for becker muscular dystrophy. Molecular therapy : the journal of the American Society of Gene Therapy 23, 192-201 (2015).
11. Carnwath, J.W. & Shotton, D.M. Muscular dystrophy in the mdx mouse: histopathology of the soleus and extensor digitorum longus muscles. J Neurol Sci 80, 39-54 (1987).
12. Coulton, G.R., Morgan, J.E., Partridge, T.A. & Sloper, J.C. The mdx mouse skeletal muscle myopathy: I. A histological, morphometric and biochemical investigation. Neuropathol Appl Neurobiol 14, 53-70 (1988).
13. Cullen, M.J. & Jaros, E. Ultrastructure of the skeletal muscle in the X chromosome-linked dystrophic (mdx) mouse. Comparison with Duchenne muscular dystrophy. Acta Neuropathol 77, 69-81 (1988).
14. Dupont-Versteegden, E.E. & McCarter, R.J. Differential expression of muscular dystrophy in diaphragm versus hindlimb muscles of mdx mice. Muscle Nerve 15, 1105-1110 (1992).
15. Stedman, H.H., et al. The mdx mouse diaphragm reproduces the degenerative changes of Duchenne muscular dystrophy. Nature 352, 536-539 (1991).
16. Deconinck, A.E., et al. Utrophin-dystrophin-deficient mice as a model for Duchenne muscular dystrophy. Cell 90, 717-727 (1997).
17. Grady, R.M., et al. Skeletal and cardiac myopathies in mice lacking utrophin and dystrophin: a model for Duchenne muscular dystrophy. Cell 90, 729-738 (1997).
18. Love, D.R., et al. An autosomal transcript in skeletal muscle with homology to dystrophin. Nature 339, 55-58 (1989).
19. Tinsley, J.M., et al. Primary structure of dystrophin-related protein. Nature 360, 591-593 (1992).
20. Tinsley, J., et al. Expression of full-length utrophin prevents muscular dystrophy in mdx mice. Nat Med 4, 1441-1444 (1998).
21. Squire, S., et al. Prevention of pathology in mdx mice by expression of utrophin: analysis using an inducible transgenic expression system. Hum Mol Genet 11, 3333-3344 (2002).
22. Rafael, J.A., Tinsley, J.M., Potter, A.C., Deconinck, A.E. & Davies, K.E. Skeletal muscle-specific expression of a utrophin transgene rescues utrophin-dystrophin deficient mice. Nat Genet 19, 79-82 (1998).
23. Zhou, L., et al. Haploinsufficiency of utrophin gene worsens skeletal muscle inflammation and fibrosis in mdx mice. J Neurol Sci 264, 106-111 (2008).
24. Gutpell, K.M., Hrinivich, W.T. & Hoffman, L.M. Skeletal Muscle Fibrosis in the mdx/utrn+/- Mouse Validates Its Suitability as a Murine Model of Duchenne Muscular Dystrophy. PloS one 10, e0117306 (2015).
25. Rodino-Klapac, L.R., et al. Micro-dystrophin and follistatin co-delivery restores muscle function in aged DMD model. Human molecular genetics 22, 4929-4937 (2013).
26. Nevo, Y., et al. The Ras antagonist, farnesylthiosalicylic acid (FTS), decreases fibrosis and improves muscle strength in dy/dy mouse model of muscular dystrophy. PloS one 6, e18049 (2011).
27. Rodino-Klapac, L.R., et al. A translational approach for limb vascular delivery of the micro-dystrophin gene without high volume or high pressure for treatment of Duchenne muscular dystrophy. J Transl Med 5, 45 (2007).
28. Mulieri, L.A., Hasenfuss, G., Ittleman, F., Blanchard, E.M. & Alpert, N.R. Protection of human left ventricular myocardium from cutting injury with 2,3-butanedione monoxime. Circ Res 65, 1441-1449 (1989).
29. Rodino-Klapac, L.R., et al. Persistent expression of FLAG-tagged micro dystrophin in nonhuman primates following intramuscular and vascular delivery. Molecular therapy : the journal of the American Society of Gene Therapy 18, 109-117 (2010).
30. Grose, W.E., et al. Homologous recombination mediates functional recovery of dysferlin deficiency following AAV5 gene transfer. PloS one 7, e39233 (2012).
31. Liu, M., et al. Adeno-associated virus-mediated microdystrophin expression protects young mdx muscle from contraction-induced injury. Mol Ther 11, 245-256 (2005).
32. Harper, S.Q., et al. Modular flexibility of dystrophin: implications for gene therapy of Duchenne muscular dystrophy. Nature medicine 8, 253-261 (2002).
33. Rodino-Klapac, L.R., et al. Persistent expression of FLAG-tagged micro dystrophin in nonhuman primates following intramuscular and vascular delivery. Mol Ther 18, 109-117 (2010).
34. Salva, M.Z., et al. Design of tissue-specific regulatory cassettes for high-level rAAV-mediated expression in skeletal and cardiac muscle. Mol Ther 15, 320-329 (2007).
35. Sondergaard, P.C., et al. AAV.Dysferlin Overlap Vectors Restore Function in Dysferlinopathy Animal Models. Annals of clinical and translational neurology 2, 256-270 (2015).
36. De, B.P., et al. High levels of persistent expression of alpha1-antitrypsin mediated by the nonhuman primate serotype rh.10 adeno-associated virus despite preexisting immunity to common human adeno-associated viruses. Mol Ther 13, 67-76 (2006).
37. Rodino-Klapac, L.R., et al. A translational approach for limb vascular delivery of the micro-dystrophin gene without high volume or high pressure for treatment of Duchenne muscular dystrophy. Journal of translational medicine 5, 45 (2007).
38. Bulfield et al., X chromosome-linked muscular dystrophy (mdx) in the mouse. Proc Natl Acad Sci U S A. 1984; 81(4): 1189-1192.
39. Sicinski et al., The molecular basis of muscular dystrophy in the mdx mouse: a point mutation. Science. 1989 30;244(4912):1578-80

### SEQUENCE LISTING

<110> RESEARCH INSTITUTE AT NATIONWIDE CHILDREN'S HOSPITAL
<120> ADENO-ASSOCIATED VIRUS VECTOR DELIVERY OF MUSCLE SPECIFIC MICRO-DYSTROPHIN TO TREAT MUSCULAR DYSTROPHY
<130> 28335/53169
<160> 9
<170> PatentIn version 3.5
<210> 1
   <211> 3579
   <212> DNA
   <213> Homo sapiens
<400> 1 
atgctgtggt gggaggaggt ggaggattgt tatgaaaggg aggacgtgca gaagaagact 60

tttaccaagt gggtgaacgc tcagttcagc aaatttggga agcagcacat cgagaatctg 120

ttttccgacc tgcaggatgg gagacggctg ctggatctgc tggaaggact gactggccag 180

aagctgccca aagagaaggg gagcactagg gtgcacgccc tgaacaacgt gaacaaagct 240

ctgagagtgc tgcagaacaa caacgtggat ctggtgaata ttggcagtac tgatatcgtg 300

gacgggaacc acaaactgac actgggcctg atctggaaca ttattctgca ctggcaggtg 360

aaaaatgtga tgaagaacat catggccggg ctgcagcaga ccaattccga gaagatcctg 420

ctgtcttggg tgcggcagag cacccgcaac tatccccagg tgaacgtgat taacttcact 480

acatcctgga gcgacgggct ggccctgaat gctctgattc acagccacag gcctgatctg 540

ttcgactgga atagcgtggt gtgccagcag tctgccacac agcgcctgga acatgccttc 600

aatatcgctc ggtaccagct ggggatcgaa aaactgctgg acccagagga tgtggacact 660

acatacccag ataaaaagtc tattctgatg tacattacta gcctgttcca ggtgctgcca 720

cagcaggtgt ctattgaagc cattcaggag gtggaaatgc tgccccgccc ccccaaagtg 780

actaaagagg agcattttca gctgcatcat cagatgcatt acagccagca gattaccgtg 840

agcctggctc agggatatga gcgcaccagt agtccaaaac cacggttcaa gtcctacgct 900

tatacccagg ctgcctacgt gacaactagc gaccctacta gatccccctt tccatcccag 960

cacctggagg ccccagagga caagagcttt gggtccagcc tgatggaaag cgaggtgaat 1020

ctggatcggt accagacagc cctggaggag gtgctgagct ggctgctgag tgctgaagac 1080

acactgcagg cccagggcga aatttccaat gacgtggaag tggtgaagga tcagttccac 1140

acacacgagg gctatatgat ggacctgaca gctcaccagg ggcgcgtggg caatatcctg 1200

cagctgggct ctaaactgat cggcaccggg aaactgagtg aggacgagga aacagaagtg 1260

caggagcaga tgaacctgct gaacagccgc tgggagtgtc tgagagtggc tagtatggag 1320

aagcagtcca acctgcaccg ggtgctgatg gacctgcaga accagaaact gaaagagctg 1380

aacgactggc tgacaaagac tgaggaacgc acaaggaaga tggaggagga gccactggga 1440

cccgacctgg aggatctgaa gagacaggtg cagcagcata aggtgctgca ggaggatctg 1500
 
gaacaggagc aggtgcgggt gaactccctg acacatatgg tggtggtggt ggacgaatct 1560

agtggagatc acgccaccgc cgccctggag gaacagctga aggtgctggg ggaccggtgg 1620

gccaacattt gccggtggac cgaggacagg tgggtgctgc tgcaggacat cctgctgaaa 1680

tggcagaggc tgaccgagga gcagtgtctg tttagtgctt ggctgagcga gaaagaggac 1740

gccgtgaaca agatccacac aaccggcttt aaggatcaga acgaaatgct gtctagcctg 1800

cagaaactgg ctgtgctgaa ggccgatctg gagaaaaaga agcagagcat gggcaaactg 1860

tatagcctga aacaggacct gctgagcacc ctgaagaaca agagcgtgac ccagaagaca 1920

gaagcctggc tggataactt tgcccgctgc tgggacaacc tggtgcagaa actggagaaa 1980

agtacagctc agatctctca ggctgtgacc acaacccagc ctagcctgac ccagacaacc 2040

gtgatggaaa ccgtgaccac cgtgacaacc cgcgaacaga tcctggtgaa acatgcccag 2100

gaagagctgc cacctccacc tccccagaag aagagaaccc tggagcggct gcaggagctg 2160

caggaagcca ctgacgaact ggacctgaag ctgaggcagg ccgaagtgat taaggggtct 2220

tggcagcctg tgggcgatct gctgattgat tccctgcagg accacctgga aaaggtgaag 2280

gctctgagag gcgaaattgc tccactgaag gagaacgtga gtcatgtgaa cgatctggct 2340

agacagctga caacactggg catccagctg agcccataca atctgagcac actggaggac 2400

ctgaatacca ggtggaagct gctgcaggtg gctgtggaag accgggtgcg gcagctgcat 2460

gaggcccatc gcgacttcgg accagccagc cagcactttc tgagcacatc cgtgcagggg 2520

ccctgggaga gggccatttc tcccaacaag gtgccctact atattaatca cgagacccag 2580

accacttgtt gggaccatcc caagatgaca gaactgtacc agtccctggc cgatctgaac 2640

aacgtgaggt ttagcgctta cagaaccgct atgaagctga gacggctgca gaaggccctg 2700

tgcctggatc tgctgtccct gtccgccgcc tgcgatgccc tggatcagca taatctgaag 2760

cagaacgatc agccaatgga tatcctgcag atcatcaact gcctgaccac tatctacgac 2820

aggctggagc aggagcacaa caacctggtg aacgtgcctc tgtgcgtgga tatgtgcctg 2880

aactggctgc tgaacgtgta tgacactggg cgcaccggcc ggatcagagt gctgagtttt 2940

aaaactggga ttatctccct gtgtaaggcc cacctggagg acaagtacag gtacctgttc 3000

aagcaggtgg ctagtagcac tggattttgt gaccagcgcc gcctgggact gctgctgcat 3060

gatagtatcc agattcctag acagctggga gaggtggcta gtttcggagg atctaacatc 3120

gaacccagcg tgcgcagctg tttccagttt gccaataaca aacctgaaat cgaggctgct 3180

ctgttcctgg attggatgcg cctggaacca cagagcatgg tgtggctgcc tgtgctgcac 3240

agagtggctg ccgccgaaac tgccaagcac caggctaaat gcaacatctg caaggaatgt 3300

cccattatcg gctttcgcta caggagtctg aaacatttta actacgatat ttgccagagc 3360

tgcttctttt ccggaagagt ggccaaagga cacaagatgc actaccctat ggtggaatat 3420

tgcaccccaa ctacatctgg cgaagatgtg cgcgattttg ccaaggtgct gaagaataag 3480

tttcggacta agaggtactt cgccaagcac ccccgcatgg ggtatctgcc agtgcagaca 3540
 gtgctggaag gagacaatat ggagaccgat acaatgtga 3579
<210> 2
   <211> 810
   <212> DNA
   <213> Adeno-associated virus
<400> 2 
gtttaaacaa gcttgcatgt ctaagctaga cccttcagat taaaaataac tgaggtaagg 60

gcctgggtag gggaggtggt gtgagacgct cctgtctctc ctctatctgc ccatcggccc 120

tttggggagg aggaatgtgc ccaaggacta aaaaaaggcc atggagccag aggggcgagg 180

gcaacagacc tttcatgggc aaaccttggg gccctgctgt ctagcatgcc ccactacggg 240

tctaggctgc ccatgtaagg aggcaaggcc tggggacacc cgagatgcct ggttataatt 300

aacccagaca tgtggctgcc cccccccccc caacacctgc tgcctctaaa aataaccctg 360

tccctggtgg atcccctgca tgcgaagatc ttcgaacaag gctgtggggg actgagggca 420

ggctgtaaca ggcttggggg ccagggctta tacgtgcctg ggactcccaa agtattactg 480

ttccatgttc ccggcgaagg gccagctgtc ccccgccagc tagactcagc acttagttta 540

ggaaccagtg agcaagtcag cccttggggc agcccataca aggccatggg gctgggcaag 600

ctgcacgcct gggtccgggg tgggcacggt gcccgggcaa cgagctgaaa gctcatctgc 660

tctcaggggc ccctccctgg ggacagcccc tcctggctag tcacaccctg taggctcctc 720

tatataaccc aggggcacag gggctgccct cattctacca ccacctccac agcacagaca 780

gacactcagg agccagccag cggcgcgccc 810
<210> 3
   <211> 8562
   <212> DNA
   <213> Adeno-associated virus
<400> 3 
gcccaatacg caaaccgcct ctccccgcgc gttggccgat tcattaatgc agctgcgcgc 60

tcgctcgctc actgaggccg cccgggcaaa gcccgggcgt cgggcgacct ttggtcgccc 120

ggcctcagtg agcgagcgag cgcgcagaga gggagtggcc aactccatca ctaggggttc 180

cttgtagtta atgattaacc cgccatgcta cttatctacg tagccatgct ctagagttta 240

aacaagcttg catgtctaag ctagaccctt cagattaaaa ataactgagg taagggcctg 300

ggtaggggag gtggtgtgag acgctcctgt ctctcctcta tctgcccatc ggccctttgg 360

ggaggaggaa tgtgcccaag gactaaaaaa aggccatgga gccagagggg cgagggcaac 420

agacctttca tgggcaaacc ttggggccct gctgtctagc atgccccact acgggtctag 480

gctgcccatg taaggaggca aggcctgggg acacccgaga tgcctggtta taattaaccc 540

agacatgtgg ctgccccccc ccccccaaca cctgctgcct ctaaaaataa ccctgtccct 600

ggtggatccc ctgcatgcga agatcttcga acaaggctgt gggggactga gggcaggctg 660

taacaggctt gggggccagg gcttatacgt gcctgggact cccaaagtat tactgttcca 720
 
tgttcccggc gaagggccag ctgtcccccg ccagctagac tcagcactta gtttaggaac 780

cagtgagcaa gtcagccctt ggggcagccc atacaaggcc atggggctgg gcaagctgca 840

cgcctgggtc cggggtgggc acggtgcccg ggcaacgagc tgaaagctca tctgctctca 900

ggggcccctc cctggggaca gcccctcctg gctagtcaca ccctgtaggc tcctctatat 960

aacccagggg cacaggggct gccctcattc taccaccacc tccacagcac agacagacac 1020

tcaggagcag ccagcggcgc gcccaggtaa gtttagtctt tttgtctttt atttcaggtc 1080

ccggatccgg tggtggtgca aatcaaagaa ctgctcctca gtggatgttg cctttacttc 1140

taggcctgta cggaagtgtt acttctgctc taaaagctgc ggaattgtac ccgcggccgc 1200

caccatgctg tggtgggagg aggtggagga ttgttatgaa agggaggacg tgcagaagaa 1260

gacttttacc aagtgggtga acgctcagtt cagcaaattt gggaagcagc acatcgagaa 1320

tctgttttcc gacctgcagg atgggagacg gctgctggat ctgctggaag gactgactgg 1380

ccagaagctg cccaaagaga aggggagcac tagggtgcac gccctgaaca acgtgaacaa 1440

agctctgaga gtgctgcaga acaacaacgt ggatctggtg aatattggca gtactgatat 1500

cgtggacggg aaccacaaac tgacactggg cctgatctgg aacattattc tgcactggca 1560

ggtgaaaaat gtgatgaaga acatcatggc cgggctgcag cagaccaatt ccgagaagat 1620

cctgctgtct tgggtgcggc agagcacccg caactatccc caggtgaacg tgattaactt 1680

cactacatcc tggagcgacg ggctggccct gaatgctctg attcacagcc acaggcctga 1740

tctgttcgac tggaatagcg tggtgtgcca gcagtctgcc acacagcgcc tggaacatgc 1800

cttcaatatc gctcggtacc agctggggat cgaaaaactg ctggacccag aggatgtgga 1860

cactacatac ccagataaaa agtctattct gatgtacatt actagcctgt tccaggtgct 1920

gccacagcag gtgtctattg aagccattca ggaggtggaa atgctgcccc gcccccccaa 1980

agtgactaaa gaggagcatt ttcagctgca tcatcagatg cattacagcc agcagattac 2040

cgtgagcctg gctcagggat atgagcgcac cagtagtcca aaaccacggt tcaagtccta 2100

cgcttatacc caggctgcct acgtgacaac tagcgaccct actagatccc cctttccatc 2160

ccagcacctg gaggccccag aggacaagag ctttgggtcc agcctgatgg aaagcgaggt 2220

gaatctggat cggtaccaga cagccctgga ggaggtgctg agctggctgc tgagtgctga 2280

agacacactg caggcccagg gcgaaatttc caatgacgtg gaagtggtga aggatcagtt 2340

ccacacacac gagggctata tgatggacct gacagctcac caggggcgcg tgggcaatat 2400

cctgcagctg ggctctaaac tgatcggcac cgggaaactg agtgaggacg aggaaacaga 2460

agtgcaggag cagatgaacc tgctgaacag ccgctgggag tgtctgagag tggctagtat 2520

ggagaagcag tccaacctgc accgggtgct gatggacctg cagaaccaga aactgaaaga 2580

gctgaacgac tggctgacaa agactgagga acgcacaagg aagatggagg aggagccact 2640

gggacccgac ctggaggatc tgaagagaca ggtgcagcag cataaggtgc tgcaggagga 2700

tctggaacag gagcaggtgc gggtgaactc cctgacacat atggtggtgg tggtggacga 2760
 
atctagtgga gatcacgcca ccgccgccct ggaggaacag ctgaaggtgc tgggggaccg 2820

gtgggccaac atttgccggt ggaccgagga caggtgggtg ctgctgcagg acatcctgct 2880

gaaatggcag aggctgaccg aggagcagtg tctgtttagt gcttggctga gcgagaaaga 2940

ggacgccgtg aacaagatcc acacaaccgg ctttaaggat cagaacgaaa tgctgtctag 3000

cctgcagaaa ctggctgtgc tgaaggccga tctggagaaa aagaagcaga gcatgggcaa 3060

actgtatagc ctgaaacagg acctgctgag caccctgaag aacaagagcg tgacccagaa 3120

gacagaagcc tggctggata actttgcccg ctgctgggac aacctggtgc agaaactgga 3180

gaaaagtaca gctcagatct ctcaggctgt gaccacaacc cagcctagcc tgacccagac 3240

aaccgtgatg gaaaccgtga ccaccgtgac aacccgcgaa cagatcctgg tgaaacatgc 3300

ccaggaagag ctgccacctc cacctcccca gaagaagaga accctggagc ggctgcagga 3360

gctgcaggaa gccactgacg aactggacct gaagctgagg caggccgaag tgattaaggg 3420

gtcttggcag cctgtgggcg atctgctgat tgattccctg caggaccacc tggaaaaggt 3480

gaaggctctg agaggcgaaa ttgctccact gaaggagaac gtgagtcatg tgaacgatct 3540

ggctagacag ctgacaacac tgggcatcca gctgagccca tacaatctga gcacactgga 3600

ggacctgaat accaggtgga agctgctgca ggtggctgtg gaagaccggg tgcggcagct 3660

gcatgaggcc catcgcgact tcggaccagc cagccagcac tttctgagca catccgtgca 3720

ggggccctgg gagagggcca tttctcccaa caaggtgccc tactatatta atcacgagac 3780

ccagaccact tgttgggacc atcccaagat gacagaactg taccagtccc tggccgatct 3840

gaacaacgtg aggtttagcg cttacagaac cgctatgaag ctgagacggc tgcagaaggc 3900

cctgtgcctg gatctgctgt ccctgtccgc cgcctgcgat gccctggatc agcataatct 3960

gaagcagaac gatcagccaa tggatatcct gcagatcatc aactgcctga ccactatcta 4020

cgacaggctg gagcaggagc acaacaacct ggtgaacgtg cctctgtgcg tggatatgtg 4080

cctgaactgg ctgctgaacg tgtatgacac tgggcgcacc ggccggatca gagtgctgag 4140

ttttaaaact gggattatct ccctgtgtaa ggcccacctg gaggacaagt acaggtacct 4200

gttcaagcag gtggctagta gcactggatt ttgtgaccag cgccgcctgg gactgctgct 4260

gcatgatagt atccagattc ctagacagct gggagaggtg gctagtttcg gaggatctaa 4320

catcgaaccc agcgtgcgca gctgtttcca gtttgccaat aacaaacctg aaatcgaggc 4380

tgctctgttc ctggattgga tgcgcctgga accacagagc atggtgtggc tgcctgtgct 4440

gcacagagtg gctgccgccg aaactgccaa gcaccaggct aaatgcaaca tctgcaagga 4500

atgtcccatt atcggctttc gctacaggag tctgaaacat tttaactacg atatttgcca 4560

gagctgcttc ttttccggaa gagtggccaa aggacacaag atgcactacc ctatggtgga 4620

atattgcacc ccaactacat ctggcgaaga tgtgcgcgat tttgccaagg tgctgaagaa 4680

taagtttcgg actaagaggt acttcgccaa gcacccccgc atggggtatc tgccagtgca 4740

gacagtgctg gaaggagaca atatggagac cgatacaatg tgagcggccg caataaaaga 4800
 
tctttatttt cattagatct gtgtgttggt tttttgtgtg tctagagcat ggctacgtag 4860

ataagtagca tggcgggtta atcattaact acaaggaacc cctagtgatg gagttggcca 4920

ctccctctct gcgcgctcgc tcgctcactg aggccgggcg accaaaggtc gcccgacgcc 4980

cgggctttgc ccgggcggcc tcagtgagcg agcgagcgcg cagctggcgt aatagcgaag 5040

aggcccgcac cgatcgccct tcccaacagt tgcgcagcct gaatggcgaa tggcgattcc 5100

gttgcaatgg ctggcggtaa tattgttctg gatattacca gcaaggccga tagtttgagt 5160

tcttctactc aggcaagtga tgttattact aatcaaagaa gtattgcgac aacggttaat 5220

ttgcgtgatg gacagactct tttactcggt ggcctcactg attataaaaa cacttctcag 5280

gattctggcg taccgttcct gtctaaaatc cctttaatcg gcctcctgtt tagctcccgc 5340

tctgattcta acgaggaaag cacgttatac gtgctcgtca aagcaaccat agtacgcgcc 5400

ctgtagcggc gcattaagcg cggcgggtgt ggtggttacg cgcagcgtga ccgctacact 5460

tgccagcgcc ctagcgcccg ctcctttcgc tttcttccct tcctttctcg ccacgttcgc 5520

cggctttccc cgtcaagctc taaatcgggg gctcccttta gggttccgat ttagtgcttt 5580

acggcacctc gaccccaaaa aacttgatta gggtgatggt tcacgtagtg ggccatcgcc 5640

ctgatagacg gtttttcgcc ctttgacgtt ggagtccacg ttctttaata gtggactctt 5700

gttccaaact ggaacaacac tcaaccctat ctcggtctat tcttttgatt tataagggat 5760

tttgccgatt tcggcctatt ggttaaaaaa tgagctgatt taacaaaaat ttaacgcgaa 5820

ttttaacaaa atattaacgc ttacaattta aatatttgct tatacaatct tcctgttttt 5880

ggggcttttc tgattatcaa ccggggtaca tatgattgac atgctagttt tacgattacc 5940

gttcatcgat tctcttgttt gctccagact ctcaggcaat gacctgatag cctttgtaga 6000

gacctctcaa aaatagctac cctctccggc atgaatttat cagctagaac ggttgaatat 6060

catattgatg gtgatttgac tgtctccggc ctttctcacc cgtttgaatc tttacctaca 6120

cattactcag gcattgcatt taaaatatat gagggttcta aaaattttta tccttgcgtt 6180

gaaataaagg cttctcccgc aaaagtatta cagggtcata atgtttttgg tacaaccgat 6240

ttagctttat gctctgaggc tttattgctt aattttgcta attctttgcc ttgcctgtat 6300

gatttattgg atgttggaat cgcctgatgc ggtattttct ccttacgcat ctgtgcggta 6360

tttcacaccg catatggtgc actctcagta caatctgctc tgatgccgca tagttaagcc 6420

agccccgaca cccgccaaca cccgctgacg cgccctgacg ggcttgtctg ctcccggcat 6480

ccgcttacag acaagctgtg accgtctccg ggagctgcat gtgtcagagg ttttcaccgt 6540

catcaccgaa acgcgcgaga cgaaagggcc tcgtgatacg cctattttta taggttaatg 6600

tcatgataat aatggtttct tagacgtcag gtggcacttt tcggggaaat gtgcgcggaa 6660

cccctatttg tttatttttc taaatacatt caaatatgta tccgctcatg agacaataac 6720

cctgataaat gcttcaataa tattgaaaaa ggaagagtat gagtattcaa catttccgtg 6780

tcgcccttat tccctttttt gcggcatttt gccttcctgt ttttgctcac ccagaaacgc 6840
 
tggtgaaagt aaaagatgct gaagatcagt tgggtgcacg agtgggttac atcgaactgg 6900

atctcaacag cggtaagatc cttgagagtt ttcgccccga agaacgtttt ccaatgatga 6960

gcacttttaa agttctgcta tgtggcgcgg tattatcccg tattgacgcc gggcaagagc 7020

aactcggtcg ccgcatacac tattctcaga atgacttggt tgagtactca ccagtcacag 7080

aaaagcatct tacggatggc atgacagtaa gagaattatg cagtgctgcc ataaccatga 7140

gtgataacac tgcggccaac ttacttctga caacgatcgg aggaccgaag gagctaaccg 7200

cttttttgca caacatgggg gatcatgtaa ctcgccttga tcgttgggaa ccggagctga 7260

atgaagccat accaaacgac gagcgtgaca ccacgatgcc tgtagcaatg gcaacaacgt 7320

tgcgcaaact attaactggc gaactactta ctctagcttc ccggcaacaa ttaatagact 7380

ggatggaggc ggataaagtt gcaggaccac ttctgcgctc ggcccttccg gctggctggt 7440

ttattgctga taaatctgga gccggtgagc gtgggtctcg cggtatcatt gcagcactgg 7500

ggccagatgg taagccctcc cgtatcgtag ttatctacac gacggggagt caggcaacta 7560

tggatgaacg aaatagacag atcgctgaga taggtgcctc actgattaag cattggtaac 7620

tgtcagacca agtttactca tatatacttt agattgattt aaaacttcat ttttaattta 7680

aaaggatcta ggtgaagatc ctttttgata atctcatgac caaaatccct taacgtgagt 7740

tttcgttcca ctgagcgtca gaccccgtag aaaagatcaa aggatcttct tgagatcctt 7800

tttttctgcg cgtaatctgc tgcttgcaaa caaaaaaacc accgctacca gcggtggttt 7860

gtttgccgga tcaagagcta ccaactcttt ttccgaaggt aactggcttc agcagagcgc 7920

agataccaaa tactgttctt ctagtgtagc cgtagttagg ccaccacttc aagaactctg 7980

tagcaccgcc tacatacctc gctctgctaa tcctgttacc agtggctgct gccagtggcg 8040

ataagtcgtg tcttaccggg ttggactcaa gacgatagtt accggataag gcgcagcggt 8100

cgggctgaac ggggggttcg tgcacacagc ccagcttgga gcgaacgacc tacaccgaac 8160

tgagatacct acagcgtgag ctatgagaaa gcgccacgct tcccgaaggg agaaaggcgg 8220

acaggtatcc ggtaagcggc agggtcggaa caggagagcg cacgagggag cttccagggg 8280

gaaacgcctg gtatctttat agtcctgtcg ggtttcgcca cctctgactt gagcgtcgat 8340

ttttgtgatg ctcgtcaggg gggcggagcc tatggaaaaa cgccagcaac gcggcctttt 8400

tacggttcct ggccttttgc tggccttttg ctcacatgtt ctttcctgcg ttatcccctg 8460

attctgtgga taaccgtatt accgcctttg agtgagctga taccgctcgc cgcagccgaa 8520

cgaccgagcg cagcgagtca gtgagcgagg aagcggaaga gc 8562
<210> 4
   <211> 564
   <212> DNA
   <213> Adeno-associated virus
<400> 4 cagccactat gggtctaggc tgcccatgta aggaggcaag gcctggggac acccgagatg 60 
cctggttata attaacccag acatgtggct gctccccccc cccaacacct gctgcctgag 120

cctcaccccc accccggtgc ctgggtctta ggctctgtac accatggagg agaagctcgc 180

tctaaaaata accctgtccc tggtgggctg tgggggactg agggcaggct gtaacaggct 240

tgggggccag ggcttatacg tgcctgggac tcccaaagta ttactgttcc atgttcccgg 300

cgaagggcca gctgtccccc gccagctaga ctcagcactt agtttaggaa ccagtgagca 360

agtcagccct tggggcagcc catacaaggc catggggctg ggcaagctgc acgcctgggt 420

ccggggtggg cacggtgccc gggcaacgag ctgaaagctc atctgctctc aggggcccct 480

ccctggggac agcccctcct ggctagtcac accctgtagg ctcctctata taacccaggg 540

gcacaggggc tgcccccggg tcac 564
<210> 5
   <211> 8409
   <212> DNA
   <213> Adeno-associated virus
<400> 5 
gcccaatacg caaaccgcct ctccccgcgc gttggccgat tcattaatgc agctggcgcg 60

ctcgctcgct cactgaggcc gcccgggcaa agcccgggcg tcgggcgacc tttggtcgcc 120

cggcctcagt gagcgagcga gcgcgcagag agggagtggc caactccatc actaggggtt 180

ccttgtagtt aatgattaac ccgccatgct aattatctac gtagccatgt ctagacagcc 240

actatgggtc taggctgccc atgtaaggag gcaaggcctg gggacacccg agatgcctgg 300

ttataattaa cccagacatg tggctgctcc ccccccccaa cacctgctgc ctgagcctca 360

cccccacccc ggtgcctggg tcttaggctc tgtacaccat ggaggagaag ctcgctctaa 420

aaataaccct gtccctggtg ggctgtgggg gactgagggc aggctgtaac aggcttgggg 480

gccagggctt atacgtgcct gggactccca aagtattact gttccatgtt cccggcgaag 540

ggccagctgt cccccgccag ctagactcag cacttagttt aggaaccagt gagcaagtca 600

gcccttgggg cagcccatac aaggccatgg ggctgggcaa gctgcacgcc tgggtccggg 660

gtgggcacgg tgcccgggca acgagctgaa agctcatctg ctctcagggg cccctccctg 720

gggacagccc ctcctggcta gtcacaccct gtaggctcct ctatataacc caggggcaca 780

ggggctgccc ccgggtcacc accacctcca cagcacagac agacactcag gagccagcca 840

gccaggtaag tttagtcttt ttgtctttta tttcaggtcc cggatccggt ggtggtgcaa 900

atcaaagaac tgctcctcag tggatgttgc ctttacttct aggcctgtac ggaagtgtta 960

cttctgctct aaaagctgcg gaattgtacc cgcggccgcc accatgctgt ggtgggagga 1020

ggtggaggat tgttatgaaa gggaggacgt gcagaagaag acttttacca agtgggtgaa 1080

cgctcagttc agcaaatttg ggaagcagca catcgagaat ctgttttccg acctgcagga 1140

tgggagacgg ctgctggatc tgctggaagg actgactggc cagaagctgc ccaaagagaa 1200

ggggagcact agggtgcacg ccctgaacaa cgtgaacaaa gctctgagag tgctgcagaa 1260

caacaacgtg gatctggtga atattggcag tactgatatc gtggacggga accacaaact 1320
 
gacactgggc ctgatctgga acattattct gcactggcag gtgaaaaatg tgatgaagaa 1380

catcatggcc gggctgcagc agaccaattc cgagaagatc ctgctgtctt gggtgcggca 1440

gagcacccgc aactatcccc aggtgaacgt gattaacttc actacatcct ggagcgacgg 1500

gctggccctg aatgctctga ttcacagcca caggcctgat ctgttcgact ggaatagcgt 1560

ggtgtgccag cagtctgcca cacagcgcct ggaacatgcc ttcaatatcg ctcggtacca 1620

gctggggatc gaaaaactgc tggacccaga ggatgtggac actacatacc cagataaaaa 1680

gtctattctg atgtacatta ctagcctgtt ccaggtgctg ccacagcagg tgtctattga 1740

agccattcag gaggtggaaa tgctgccccg cccccccaaa gtgactaaag aggagcattt 1800

tcagctgcat catcagatgc attacagcca gcagattacc gtgagcctgg ctcagggata 1860

tgagcgcacc agtagtccaa aaccacggtt caagtcctac gcttataccc aggctgccta 1920

cgtgacaact agcgacccta ctagatcccc ctttccatcc cagcacctgg aggccccaga 1980

ggacaagagc tttgggtcca gcctgatgga aagcgaggtg aatctggatc ggtaccagac 2040

agccctggag gaggtgctga gctggctgct gagtgctgaa gacacactgc aggcccaggg 2100

cgaaatttcc aatgacgtgg aagtggtgaa ggatcagttc cacacacacg agggctatat 2160

gatggacctg acagctcacc aggggcgcgt gggcaatatc ctgcagctgg gctctaaact 2220

gatcggcacc gggaaactga gtgaggacga ggaaacagaa gtgcaggagc agatgaacct 2280

gctgaacagc cgctgggagt gtctgagagt ggctagtatg gagaagcagt ccaacctgca 2340

ccgggtgctg atggacctgc agaaccagaa actgaaagag ctgaacgact ggctgacaaa 2400

gactgaggaa cgcacaagga agatggagga ggagccactg ggacccgacc tggaggatct 2460

gaagagacag gtgcagcagc ataaggtgct gcaggaggat ctggaacagg agcaggtgcg 2520

ggtgaactcc ctgacacata tggtggtggt ggtggacgaa tctagtggag atcacgccac 2580

cgccgccctg gaggaacagc tgaaggtgct gggggaccgg tgggccaaca tttgccggtg 2640

gaccgaggac aggtgggtgc tgctgcagga catcctgctg aaatggcaga ggctgaccga 2700

ggagcagtgt ctgtttagtg cttggctgag cgagaaagag gacgccgtga acaagatcca 2760

cacaaccggc tttaaggatc agaacgaaat gctgtctagc ctgcagaaac tggctgtgct 2820

gaaggccgat ctggagaaaa agaagcagag catgggcaaa ctgtatagcc tgaaacagga 2880

cctgctgagc accctgaaga acaagagcgt gacccagaag acagaagcct ggctggataa 2940

ctttgcccgc tgctgggaca acctggtgca gaaactggag aaaagtacag ctcagatctc 3000

tcaggctgtg accacaaccc agcctagcct gacccagaca accgtgatgg aaaccgtgac 3060

caccgtgaca acccgcgaac agatcctggt gaaacatgcc caggaagagc tgccacctcc 3120

acctccccag aagaagagaa ccctggagcg gctgcaggag ctgcaggaag ccactgacga 3180

actggacctg aagctgaggc aggccgaagt gattaagggg tcttggcagc ctgtgggcga 3240

tctgctgatt gattccctgc aggaccacct ggaaaaggtg aaggctctga gaggcgaaat 3300

tgctccactg aaggagaacg tgagtcatgt gaacgatctg gctagacagc tgacaacact 3360
 
gggcatccag ctgagcccat acaatctgag cacactggag gacctgaata ccaggtggaa 3420

gctgctgcag gtggctgtgg aagaccgggt gcggcagctg catgaggccc atcgcgactt 3480

cggaccagcc agccagcact ttctgagcac atccgtgcag gggccctggg agagggccat 3540

ttctcccaac aaggtgccct actatattaa tcacgagacc cagaccactt gttgggacca 3600

tcccaagatg acagaactgt accagtccct ggccgatctg aacaacgtga ggtttagcgc 3660

ttacagaacc gctatgaagc tgagacggct gcagaaggcc ctgtgcctgg atctgctgtc 3720

cctgtccgcc gcctgcgatg ccctggatca gcataatctg aagcagaacg atcagccaat 3780

ggatatcctg cagatcatca actgcctgac cactatctac gacaggctgg agcaggagca 3840

caacaacctg gtgaacgtgc ctctgtgcgt ggatatgtgc ctgaactggc tgctgaacgt 3900

gtatgacact gggcgcaccg gccggatcag agtgctgagt tttaaaactg ggattatctc 3960

cctgtgtaag gcccacctgg aggacaagta caggtacctg ttcaagcagg tggctagtag 4020

cactggattt tgtgaccagc gccgcctggg actgctgctg catgatagta tccagattcc 4080

tagacagctg ggagaggtgg ctagtttcgg aggatctaac atcgaaccca gcgtgcgcag 4140

ctgtttccag tttgccaata acaaacctga aatcgaggct gctctgttcc tggattggat 4200

gcgcctggaa ccacagagca tggtgtggct gcctgtgctg cacagagtgg ctgccgccga 4260

aactgccaag caccaggcta aatgcaacat ctgcaaggaa tgtcccatta tcggctttcg 4320

ctacaggagt ctgaaacatt ttaactacga tatttgccag agctgcttct tttccggaag 4380

agtggccaaa ggacacaaga tgcactaccc tatggtggaa tattgcaccc caactacatc 4440

tggcgaagat gtgcgcgatt ttgccaaggt gctgaagaat aagtttcgga ctaagaggta 4500

cttcgccaag cacccccgca tggggtatct gccagtgcag acagtgctgg aaggagacaa 4560

tatggagacc gatacaatgt gagcggccgc aataaaagat ctttattttc attagatctg 4620

tgtgttggtt ttttgtgtgt ctagagcatg gctacgtaga taagtagcat ggcgggttaa 4680

tcattaacta caaggaaccc ctagtgatgg agttggccac tccctctctg cgcgctcgct 4740

cgctcactga ggccgggcga ccaaaggtcg cccgacgccc gggctttgcc cgggcggcct 4800

cagtgagcga gcgagcgcgc cagctggcgt aatagcgaag aggcccgcac cgatcgccct 4860

tcccaacagt tgcgcagcct gaatggcgaa tggaagttcc agacgattga gcgtcaaaat 4920

gtaggtattt ccatgagcgt ttttcctgtt gcaatggctg gcggtaatat tgttctggat 4980

attaccagca aggccgatag tttgagttct tctactcagg caagtgatgt tattactaat 5040

caaagaagta ttgcgacaac ggttaatttg cgtgatggac agactctttt actcggtggc 5100

ctcactgatt ataaaaacac ttctcaggat tctggcgtac cgttcctgtc taaaatccct 5160

ttaatcggcc tcctgtttag ctcccgctct gattctaacg aggaaagcac gttatacgtg 5220

ctcgtcaaag caaccatagt acgcgccctg tagcggcgca ttaagcgcgg cgggtgtggt 5280

ggttacgcgc agcgtgaccg ctacacttgc cagcgcccta gcgcccgctc ctttcgcttt 5340

cttcccttcc tttctcgcca cgttcgccgg ctttccccgt caagctctaa atcgggggct 5400
 
ccctttaggg ttccgattta gtgatttacg gcacctcgac cccaaaaaac ttgattaggg 5460
tgatggttca cgtagtgggc catcgccctg atagacggtt tttcgccctt tgacgttgga 5520
gtccacgttc tttaatagtg gactcttgtt ccaaactgga acaacactca accctatctc 5580
ggtctattct tttgatttat aagggatttt gccgatttcg gcctattggt taaaaaatga 5640
gctgatttaa caaaaattta acgcgaattt taacaaaata ttaacgttta caatttaaat 5700
atttgcttat acaatcttcc tgtttttggg gcttttctga ttatcaaccg gggtacatat 5760
gattgacatg ctagttttac gattaccgtt catcgattct cttgtttgct ccagactctc 5820
aggcaatgac ctgatagcct ttgtagagac ctctcaaaaa tagctaccct ctccggcatg 5880
aatttatcag ctagaacggt tgaatatcat attgatggtg atttgactgt ctccggcctt 5940
tctcacccgt ttgaatcttt acctacacat tactcaggca ttgcatttaa aatatatgag 6000
ggttctaaaa atttttatcc ttgcgttgaa ataaaggctt ctcccgcaaa agtattacag 6060
ggtcataatg tttttggtac aaccgattta gctttatgct ctgaggcttt attgcttaat 6120
tttgctaatt ctttgccttg cctgtatgat ttattggatg ttggaagttc ctgatgcggt 6180
attttctcct tacgcatctg tgcggtattt cacaccgcat atggtgcact ctcagtacaa 6240
tctgctctga tgccgcatag ttaagccagc cccgacaccc gccaacaccc gctgacgcgc 6300
cctgacgggc ttgtctgctc ccggcatccg cttacagaca agctgtgacc gtctccggga 6360
gctgcatgtg tcagaggttt tcaccgtcat caccgaaacg cgcgagacga aagggcctcg 6420
tgatacgcct atttttatag gttaatgtca tgataataat ggtttcttag acgtcaggtg 6480
gcacttttcg gggaaatgtg cgcggaaccc ctatttgttt atttttctaa atacattcaa 6540
atatgtatcc gctcatgaga caataaccct gataaatgct tcaataatat tgaaaaagga 6600
agagtatgag tattcaacat ttccgtgtcg cccttattcc cttttttgcg gcattttgcc 6660
ttcctgtttt tgctcaccca gaaacgctgg tgaaagtaaa agatgctgaa gatcagttgg 6720
gtgcacgagt gggttacatc gaactggatc tcaacagcgg taagatcctt gagagttttc 6780
gccccgaaga acgttttcca atgatgagca cttttaaagt tctgctatgt ggcgcggtat 6840
tatcccgtat tgacgccggg caagagcaac tcggtcgccg catacactat tctcagaatg 6900
acttggttga gtactcacca gtcacagaaa agcatcttac ggatggcatg acagtaagag 6960
aattatgcag tgctgccata accatgagtg ataacactgc ggccaactta cttctgacaa 7020
cgatcggagg accgaaggag ctaaccgctt ttttgcacaa catgggggat catgtaactc 7080
gccttgatcg ttgggaaccg gagctgaatg aagccatacc aaacgacgag cgtgacacca 7140
cgatgcctgt agcaatggca acaacgttgc gcaaactatt aactggcgaa ctacttactc 7200
tagcttcccg gcaacaatta atagactgga tggaggcgga taaagttgca ggaccacttc 7260
tgcgctcggc ccttccggct ggctggttta ttgctgataa atctggagcc ggtgagcgtg 7320
ggtctcgcgg tatcattgca gcactggggc cagatggtaa gccctcccgt atcgtagtta 7380
tctacacgac ggggagtcag gcaactatgg atgaacgaaa tagacagatc gctgagatag 7440
 
gtgcctcact gattaagcat tggtaactgt cagaccaagt ttactcatat atactttaga 7500

ttgatttaaa acttcatttt taatttaaaa ggatctaggt gaagatcctt tttgataatc 7560

tcatgaccaa aatcccttaa cgtgagtttt cgttccactg agcgtcagac cccgtagaaa 7620

agatcaaagg atcttcttga gatccttttt ttctgcgcgt aatctgctgc ttgcaaacaa 7680

aaaaaccacc gctaccagcg gtggtttgtt tgccggatca agagctacca actctttttc 7740

cgaaggtaac tggcttcagc agagcgcaga taccaaatac tgtccttcta gtgtagccgt 7800

agttaggcca ccacttcaag aactctgtag caccgcgtac atacctcgct ctgctaatcc 7860

tgttaccagt ggctgctgcc agtggcgata agtcgtgtct taccgggttg gactcaagac 7920

gatagttacc ggataaggcg cagcggtcgg gctgaacggg gggttcgtgc acacagccca 7980

gcttggagcg aacgacctac accgaactga gatacctaca gcgtgagcta tgagaaagcg 8040

ccacgcttcc cgaagggaga aaggcggaca ggtatccggt aagcggcagg gtcggaacag 8100

gagagcgcac gagggagctt ccagggggaa acgcctggta tctttatagt cctgtcgggt 8160

ttcgccacct ctgacttgag cgtcgatttt tgtgatgctc gtcagggggg cggagcctat 8220

ggaaaaacgc cagcaacgcg gcctttttac ggttcctggc cttttgctgg ccttttgctc 8280

acatgttctt tcctgcgtta tcccctgatt ctgtggataa ccgtattacc gggtttgagt 8340

gagctgatac cgctcgccgc agccgaacga ccgagcgcag cgagtcagtg agcgaccaag 8400

cggaagagc 8409
<210> 6
   <211> 8611
   <212> DNA
   <213> Adeno-associated virus
<400> 6 
gcccaatacg caaaccgcct ctccccgcgc gttggccgat tcattaatgc agctggcgcg 60

ctcgctcgct cactgaggcc gcccgggcaa agcccgggcg tcgggcgacc tttggtcgcc 120

cggcctcagt gagcgagcga gcgcgcagag agggagtggc caactccatc actaggggtt 180

ccttgtagtt aatgattaac ccgccatgct aattatctac gtagccatgt ctagagttta 240

aacaagcttg catgtctaag ctagaccctt cagattaaaa ataactgagg taagggcctg 300

ggtaggggag gtggtgtgag acgctcctgt ctctcctcta tctgcccatc ggccctttgg 360

ggaggaggaa tgtgcccaag gactaaaaaa aggccatgga gccagagggg cgagggcaac 420

agacctttca tgggcaaacc ttggggccct gctgtctagc atgccccact acgggtctag 480

gctgcccatg taaggaggca aggcctgggg acacccgaga tgcctggtta taattaaccc 540

agacatgtgg ctgccccccc ccccccaaca cctgctgcct ctaaaaataa ccctgtccct 600

ggtggatccc ctgcatgcga agatcttcga acaaggctgt gggggactga gggcaggctg 660

taacaggctt gggggccagg gcttatacgt gcctgggact cccaaagtat tactgttcca 720

tgttcccggc gaagggccag ctgtcccccg ccagctagac tcagcactta gtttaggaac 780
 
cagtgagcaa gtcagccctt ggggcagccc atacaaggcc atggggctgg gcaagctgca 840
cgcctgggtc cggggtgggc acggtgcccg ggcaacgagc tgaaagctca tctgctctca 900
ggggcccctc cctggggaca gcccctcctg gctagtcaca ccctgtaggc tcctctatat 960
aacccagggg cacaggggct gccctcattc taccaccacc tccacagcac agacagacac 1020
tcaggagcca gccagcggcg cgcccaggta agtttagtct tttgtctttt tatttcaggt 1080
cccggatccg gtggtggtgc aaatcaaaga actgctcctc agtggatgtt gcctttactt 1140
ctaggcctgt acggaagtgt tacttctgct ctaaaagctg cggaattgta cccgcggccg 1200
ccaccatgct gtggtgggag gaggtggagg attgttatga aagggaggac gtgcagaaga 1260
agacttttac caagtgggtg aacgctcagt tcagcaaatt tgggaagcag cacatcgaga 1320
atctgttttc cgacctgcag gatgggagac ggctgctgga tctgctggaa ggactgactg 1380
gccagaagct gcccaaagag aaggggagca ctagggtgca cgccctgaac aacgtgaaca 1440
aagctctgag agtgctgcag aacaacaacg tggatctggt gaatattggc agtactgata 1500
tcgtggacgg gaaccacaaa ctgacactgg gcctgatctg gaacattatt ctgcactggc 1560
aggtgaaaaa tgtgatgaag aacatcatgg ccgggctgca gcagaccaat tccgagaaga 1620
tcctgctgtc ttgggtgcgg cagagcaccc gcaactatcc ccaggtgaac gtgattaact 1680
tcactacatc ctggagcgac gggctggccc tgaatgctct gattcacagc cacaggcctg 1740
atctgttcga ctggaatagc gtggtgtgcc agcagtctgc cacacagcgc ctggaacatg 1800
ccttcaatat cgctcggtac cagctgggga tcgaaaaact gctggaccca gaggatgtgg 1860
acactacata cccagataaa aagtctattc tgatgtacat tactagcctg ttccaggtgc 1920
tgccacagca ggtgtctatt gaagccattc aggaggtgga aatgctgccc cgccccccca 1980
aagtgactaa agaggagcat tttcagctgc atcatcagat gcattacagc cagcagatta 2040
ccgtgagcct ggctcaggga tatgagcgca ccagtagtcc aaaaccacgg ttcaagtcct 2100
acgcttatac ccaggctgcc tacgtgacaa ctagcgaccc tactagatcc ccctttccat 2160
cccagcacct ggaggcccca gaggacaaga gctttgggtc cagcctgatg gaaagcgagg 2220
tgaatctgga tcggtaccag acagccctgg aggaggtgct gagctggctg ctgagtgctg 2280
aagacacact gcaggcccag ggcgaaattt ccaatgacgt ggaagtggtg aaggatcagt 2340
tccacacaca cgagggctat atgatggacc tgacagctca ccaggggcgc gtgggcaata 2400
tcctgcagct gggctctaaa ctgatcggca ccgggaaact gagtgaggac gaggaaacag 2460
aagtgcagga gcagatgaac ctgctgaaca gccgctggga gtgtctgaga gtggctagta 2520
tggagaagca gtccaacctg caccgggtgc tgatggacct gcagaaccag aaactgaaag 2580
agctgaacga ctggctgaca aagactgagg aacgcacaag gaagatggag gaggagccac 2640
tgggacccga cctggaggat ctgaagagac aggtgcagca gcataaggtg ctgcaggagg 2700
atctggaaca ggagcaggtg cgggtgaact ccctgacaca tatggtggtg gtggtggacg 2760
aatctagtgg agatcacgcc accgccgccc tggaggaaca gctgaaggtg ctgggggacc 2820
 
ggtgggccaa catttgccgg tggaccgagg acaggtgggt gctgctgcag gacatcctgc 2880

tgaaatggca gaggctgacc gaggagcagt gtctgtttag tgcttggctg agcgagaaag 2940

aggacgccgt gaacaagatc cacacaaccg gctttaagga tcagaacgaa atgctgtcta 3000

gcctgcagaa actggctgtg ctgaaggccg atctggagaa aaagaagcag agcatgggca 3060

aactgtatag cctgaaacag gacctgctga gcaccctgaa gaacaagagc gtgacccaga 3120

agacagaagc ctggctggat aactttgccc gctgctggga caacctggtg cagaaactgg 3180

agaaaagtac agctcagatc tctcaggctg tgaccacaac ccagcctagc ctgacccaga 3240

caaccgtgat ggaaaccgtg accaccgtga caacccgcga acagatcctg gtgaaacatg 3300

cccaggaaga gctgccacct ccacctcccc agaagaagag aaccctggag cggctgcagg 3360

agctgcagga agccactgac gaactggacc tgaagctgag gcaggccgaa gtgattaagg 3420

ggtcttggca gcctgtgggc gatctgctga ttgattccct gcaggaccac ctggaaaagg 3480

tgaaggctct gagaggcgaa attgctccac tgaaggagaa cgtgagtcat gtgaacgatc 3540

tggctagaca gctgacaaca ctgggcatcc agctgagccc atacaatctg agcacactgg 3600

aggacctgaa taccaggtgg aagctgctgc aggtggctgt ggaagaccgg gtgcggcagc 3660

tgcatgaggc ccatcgcgac ttcggaccag ccagccagca ctttctgagc acatccgtgc 3720

aggggccctg ggagagggcc atttctccca acaaggtgcc ctactatatt aatcacgaga 3780

cccagaccac ttgttgggac catcccaaga tgacagaact gtaccagtcc ctggccgatc 3840

tgaacaacgt gaggtttagc gcttacagaa ccgctatgaa gctgagacgg ctgcagaagg 3900

ccctgtgcct ggatctgctg tccctgtccg ccgcctgcga tgccctggat cagcataatc 3960

tgaagcagaa cgatcagcca atggatatcc tgcagatcat caactgcctg accactatct 4020

acgacaggct ggagcaggag cacaacaacc tggtgaacgt gcctctgtgc gtggatatgt 4080

gcctgaactg gctgctgaac gtgtatgaca ctgggcgcac cggccggatc agagtgctga 4140

gttttaaaac tgggattatc tccctgtgta aggcccacct ggaggacaag tacaggtacc 4200

tgttcaagca ggtggctagt agcactggat tttgtgacca gcgccgcctg ggactgctgc 4260

tgcatgatag tatccagatt cctagacagc tgggagaggt ggctagtttc ggaggatcta 4320

acatcgaacc cagcgtgcgc agctgtttcc agtttgccaa taacaaacct gaaatcgagg 4380

ctgctctgtt cctggattgg atgcgcctgg aaccacagag catggtgtgg ctgcctgtgc 4440

tgcacagagt ggctgccgcc gaaactgcca agcaccaggc taaatgcaac atctgcaagg 4500

aatgtcccat tatcggcttt cgctacagga gtctgaaaca ttttaactac gatatttgcc 4560

agagctgctt cttttccgga agagtggcca aaggacacaa gatgcactac cctatggtgg 4620

aatattgcac cccaactaca tctggcgaag atgtgcgcga ttttgccaag gtgctgaaga 4680

ataagtttcg gactaagagg tacttcgcca agcacccccg catggggtat ctgccagtgc 4740

agacagtgct ggaaggagac aatatggaga ccgatacaat gtgagcggcc gcaataaaag 4800

atctttattt tcattagatc tgtgtgttgg ttttttgtgt gtctagagca tggctacgta 4860
 
gataagtagc atggcgggtt aatcattaac tacaaggaac ccctagtgat ggagttggcc 4920

actccctctc tgcgcgctcg ctcgctcact gaggccgggc gaccaaaggt cgcccgacgc 4980

ccgggctttg cccgggcggc ctcagtgagc gagcgagcgc gccagctggc gtaatagcga 5040

agaggcccgc accgatcgcc cttcccaaca gttgcgcagc ctgaatggcg aatggaagtt 5100

ccagacgatt gagcgtcaaa atgtaggtat ttccatgagc gtttttcctg ttgcaatggc 5160

tggcggtaat attgttctgg atattaccag caaggccgat agtttgagtt cttctactca 5220

ggcaagtgat gttattacta atcaaagaag tattgcgaca acggttaatt tgcgtgatgg 5280

acagactctt ttactcggtg gcctcactga ttataaaaac acttctcagg attctggcgt 5340

accgttcctg tctaaaatcc ctttaatcgg cctcctgttt agctcccgct ctgattctaa 5400

cgaggaaagc acgttatacg tgctcgtcaa agcaaccata gtacgcgccc tgtagcggcg 5460

cattaagcgc ggcgggtgtg gtggttacgc gcagcgtgac cgctacactt gccagcgccc 5520

tagcgcccgc tcctttcgct ttcttccctt cctttctcgc cacgttcgcc ggctttcccc 5580

gtcaagctct aaatcggggg ctccctttag ggttccgatt tagtgattta cggcacctcg 5640

accccaaaaa acttgattag ggtgatggtt cacgtagtgg gccatcgccc tgatagacgg 5700

tttttcgccc tttgacgttg gagtccacgt tctttaatag tggactcttg ttccaaactg 5760

gaacaacact caaccctatc tcggtctatt cttttgattt ataagggatt ttgccgattt 5820

cggcctattg gttaaaaaat gagctgattt aacaaaaatt taacgcgaat tttaacaaaa 5880

tattaacgtt tacaatttaa atatttgctt atacaatctt cctgtttttg gggcttttct 5940

gattatcaac cggggtacat atgattgaca tgctagtttt acgattaccg ttcatcgatt 6000

ctcttgtttg ctccagactc tcaggcaatg acctgatagc ctttgtagag acctctcaaa 6060

aatagctacc ctctccggca tgaatttatc agctagaacg gttgaatatc atattgatgg 6120

tgatttgact gtctccggcc tttctcaccc gtttgaatct ttacctacac attactcagg 6180

cattgcattt aaaatatatg agggttctaa aaatttttat ccttgcgttg aaataaaggc 6240

ttctcccgca aaagtattac agggtcataa tgtttttggt acaaccgatt tagctttatg 6300

ctctgaggct ttattgctta attttgctaa ttctttgcct tgcctgtatg atttattgga 6360

tgttggaagt tcctgatgcg gtattttctc cttacgcatc tgtgcggtat ttcacaccgc 6420

atatggtgca ctctcagtac aatctgctct gatgccgcat agttaagcca gccccgacac 6480

ccgccaacac ccgctgacgc gccctgacgg gcttgtctgc tcccggcatc cgcttacaga 6540

caagctgtga ccgtctccgg gagctgcatg tgtcagaggt tttcaccgtc atcaccgaaa 6600

cgcgcgagac gaaagggcct cgtgatacgc ctatttttat aggttaatgt catgataata 6660

atggtttctt agacgtcagg tggcactttt cggggaaatg tgcgcggaac ccctatttgt 6720

ttatttttct aaatacattc aaatatgtat ccgctcatga gacaataacc ctgataaatg 6780

cttcaataat attgaaaaag gaagagtatg agtattcaac atttccgtgt cgcccttatt 6840

cccttttttg cggcattttg ccttcctgtt tttgctcacc cagaaacgct ggtgaaagta 6900
 
aaagatgctg aagatcagtt gggtgcacga gtgggttaca tcgaactgga tctcaacagc 6960

ggtaagatcc ttgagagttt tcgccccgaa gaacgttttc caatgatgag cacttttaaa 7020

gttctgctat gtggcgcggt attatcccgt attgacgccg ggcaagagca actcggtcgc 7080

cgcatacact attctcagaa tgacttggtt gagtactcac cagtcacaga aaagcatctt 7140

acggatggca tgacagtaag agaattatgc agtgctgcca taaccatgag tgataacact 7200

gcggccaact tacttctgac aacgatcgga ggaccgaagg agctaaccgc ttttttgcac 7260

aacatggggg atcatgtaac tcgccttgat cgttgggaac cggagctgaa tgaagccata 7320

ccaaacgacg agcgtgacac cacgatgcct gtagcaatgg caacaacgtt gcgcaaacta 7380

ttaactggcg aactacttac tctagcttcc cggcaacaat taatagactg gatggaggcg 7440

gataaagttg caggaccact tctgcgctcg gcccttccgg ctggctggtt tattgctgat 7500

aaatctggag ccggtgagcg tgggtctcgc ggtatcattg cagcactggg gccagatggt 7560

aagccctccc gtatcgtagt tatctacacg acggggagtc aggcaactat ggatgaacga 7620

aatagacaga tcgctgagat aggtgcctca ctgattaagc attggtaact gtcagaccaa 7680

gtttactcat atatacttta gattgattta aaacttcatt tttaatttaa aaggatctag 7740

gtgaagatcc tttttgataa tctcatgacc aaaatccctt aacgtgagtt ttcgttccac 7800

tgagcgtcag accccgtaga aaagatcaaa ggatcttctt gagatccttt ttttctgcgc 7860

gtaatctgct gcttgcaaac aaaaaaacca ccgctaccag cggtggtttg tttgccggat 7920

caagagctac caactctttt tccgaaggta actggcttca gcagagcgca gataccaaat 7980

actgtccttc tagtgtagcc gtagttaggc caccacttca agaactctgt agcaccgcgt 8040

acatacctcg ctctgctaat cctgttacca gtggctgctg ccagtggcga taagtcgtgt 8100

cttaccgggt tggactcaag acgatagtta ccggataagg cgcagcggtc gggctgaacg 8160

gggggttcgt gcacacagcc cagcttggag cgaacgacct acaccgaact gagataccta 8220

cagcgtgagc tatgagaaag cgccacgctt cccgaaggga gaaaggcgga caggtatccg 8280

gtaagcggca gggtcggaac aggagagcgc acgagggagc ttccaggggg aaacgcctgg 8340

tatctttata gtcctgtcgg gtttcgccac ctctgacttg agcgtcgatt tttgtgatgc 8400

tcgtcagggg ggcggagcct atggaaaaac gccagcaacg cggccttttt acggttcctg 8460

gccttttgct ggccttttgc tcacatgttc tttcctgcgt tatcccctga ttctgtggat 8520

aaccgtatta ccgggtttga gtgagctgat accgctcgcc gcagccgaac gaccgagcgc 8580

agcgagtcag tgagcgacca agcggaagag c 8611
<210> 7
   <211> 792
   <212> DNA
   <213> Adeno-associated virus
<400> 7 
aagcttgcat gtctaagcta gacccttcag attaaaaata actgaggtaa gggcctgggt 60

aggggaggtg gtgtgagacg ctcctgtctc tcctctatct gcccatcggc cctttgggga 120
 
ggaggaatgt gcccaaggac taaaaaaagg ccatggagcc agaggggcga gggcaacaga 180

cctttcatgg gcaaaccttg gggccctgct gtctagcatg ccccactacg ggtctaggct 240

gcccatgtaa ggaggcaagg cctggggaca cccgagatgc ctggttataa ttaacccaga 300

catgtggctg cccccccccc cccaacacct gctgcctcta aaaataaccc tgtccctggt 360

ggatcccctg catgcgaaga tcttcgaaca aggctgtggg ggactgaggg caggctgtaa 420

caggcttggg ggccagggct tatacgtgcc tgggactccc aaagtattac tgttccatgt 480

tcccggcgaa gggccagctg tcccccgcca gctagactca gcacttagtt taggaaccag 540

tgagcaagtc agcccttggg gcagcccata caaggccatg gggctgggca agctgcacgc 600

ctgggtccgg ggtgggcacg gtgcccgggc aacgagctga aagctcatct gctctcaggg 660

gcccctccct ggggacagcc cctcctggct agtcacaccc tgtaggctcc tctatataac 720

ccaggggcac aggggctgcc ctcattctac caccacctcc acagcacaga cagacactca 780

ggagcagcca gc 792
<210> 8
   <211> 8629
   <212> DNA
   <213> Kanamycin Plasmid
<400> 8 
gcgcgctcgc tcgctcactg aggccgcccg ggcaaagccc gggcgtcggg cgacctttgg 60

tcgcccggcc tcagtgagcg agcgagcgcg cagagaggga gtggccaact ccatcactag 120

gggttccttg tagttaatga ttaacccgcc atgctactta tctacgtagc catgctctag 180

agtttaaaca agcttgcatg tctaagctag acccttcaga ttaaaaataa ctgaggtaag 240

ggcctgggta ggggaggtgg tgtgagacgc tcctgtctct cctctatctg cccatcggcc 300

ctttggggag gaggaatgtg cccaaggact aaaaaaaggc catggagcca gaggggcgag 360

ggcaacagac ctttcatggg caaaccttgg ggccctgctg tctagcatgc cccactacgg 420

gtctaggctg cccatgtaag gaggcaaggc ctggggacac ccgagatgcc tggttataat 480

taacccagac atgtggctgc cccccccccc ccaacacctg ctgcctctaa aaataaccct 540

gtccctggtg gatcccctgc atgcgaagat cttcgaacaa ggctgtgggg gactgagggc 600

aggctgtaac aggcttgggg gccagggctt atacgtgcct gggactccca aagtattact 660

gttccatgtt cccggcgaag ggccagctgt cccccgccag ctagactcag cacttagttt 720

aggaaccagt gagcaagtca gcccttgggg cagcccatac aaggccatgg ggctgggcaa 780

gctgcacgcc tgggtccggg gtgggcacgg tgcccgggca acgagctgaa agctcatctg 840

ctctcagggg cccctccctg gggacagccc ctcctggcta gtcacaccct gtaggctcct 900

ctatataacc caggggcaca ggggctgccc tcattctacc accacctcca cagcacagac 960

agacactcag gagcagccag cggcgcgccc aggtaagttt agtctttttg tcttttattt 1020

caggtcccgg atccggtggt ggtgcaaatc aaagaactgc tcctcagtgg atgttgcctt 1080

tacttctagg cctgtacgga agtgttactt ctgctctaaa agctgcggaa ttgtacccgc 1140
 
ggccgccacc atgctgtggt gggaggaggt ggaggattgt tatgaaaggg aggacgtgca 1200

gaagaagact tttaccaagt gggtgaacgc tcagttcagc aaatttggga agcagcacat 1260

cgagaatctg ttttccgacc tgcaggatgg gagacggctg ctggatctgc tggaaggact 1320

gactggccag aagctgccca aagagaaggg gagcactagg gtgcacgccc tgaacaacgt 1380

gaacaaagct ctgagagtgc tgcagaacaa caacgtggat ctggtgaata ttggcagtac 1440

tgatatcgtg gacgggaacc acaaactgac actgggcctg atctggaaca ttattctgca 1500

ctggcaggtg aaaaatgtga tgaagaacat catggccggg ctgcagcaga ccaattccga 1560

gaagatcctg ctgtcttggg tgcggcagag cacccgcaac tatccccagg tgaacgtgat 1620

taacttcact acatcctgga gcgacgggct ggccctgaat gctctgattc acagccacag 1680

gcctgatctg ttcgactgga atagcgtggt gtgccagcag tctgccacac agcgcctgga 1740

acatgccttc aatatcgctc ggtaccagct ggggatcgaa aaactgctgg acccagagga 1800

tgtggacact acatacccag ataaaaagtc tattctgatg tacattacta gcctgttcca 1860

ggtgctgcca cagcaggtgt ctattgaagc cattcaggag gtggaaatgc tgccccgccc 1920

ccccaaagtg actaaagagg agcattttca gctgcatcat cagatgcatt acagccagca 1980

gattaccgtg agcctggctc agggatatga gcgcaccagt agtccaaaac cacggttcaa 2040

gtcctacgct tatacccagg ctgcctacgt gacaactagc gaccctacta gatccccctt 2100

tccatcccag cacctggagg ccccagagga caagagcttt gggtccagcc tgatggaaag 2160

cgaggtgaat ctggatcggt accagacagc cctggaggag gtgctgagct ggctgctgag 2220

tgctgaagac acactgcagg cccagggcga aatttccaat gacgtggaag tggtgaagga 2280

tcagttccac acacacgagg gctatatgat ggacctgaca gctcaccagg ggcgcgtggg 2340

caatatcctg cagctgggct ctaaactgat cggcaccggg aaactgagtg aggacgagga 2400

aacagaagtg caggagcaga tgaacctgct gaacagccgc tgggagtgtc tgagagtggc 2460

tagtatggag aagcagtcca acctgcaccg ggtgctgatg gacctgcaga accagaaact 2520

gaaagagctg aacgactggc tgacaaagac tgaggaacgc acaaggaaga tggaggagga 2580

gccactggga cccgacctgg aggatctgaa gagacaggtg cagcagcata aggtgctgca 2640

ggaggatctg gaacaggagc aggtgcgggt gaactccctg acacatatgg tggtggtggt 2700

ggacgaatct agtggagatc acgccaccgc cgccctggag gaacagctga aggtgctggg 2760

ggaccggtgg gccaacattt gccggtggac cgaggacagg tgggtgctgc tgcaggacat 2820

cctgctgaaa tggcagaggc tgaccgagga gcagtgtctg tttagtgctt ggctgagcga 2880

gaaagaggac gccgtgaaca agatccacac aaccggcttt aaggatcaga acgaaatgct 2940

gtctagcctg cagaaactgg ctgtgctgaa ggccgatctg gagaaaaaga agcagagcat 3000

gggcaaactg tatagcctga aacaggacct gctgagcacc ctgaagaaca agagcgtgac 3060

ccagaagaca gaagcctggc tggataactt tgcccgctgc tgggacaacc tggtgcagaa 3120

actggagaaa agtacagctc agatctctca ggctgtgacc acaacccagc ctagcctgac 3180
 
ccagacaacc gtgatggaaa ccgtgaccac cgtgacaacc cgcgaacaga tcctggtgaa 3240

acatgcccag gaagagctgc cacctccacc tccccagaag aagagaaccc tggagcggct 3300

gcaggagctg caggaagcca ctgacgaact ggacctgaag ctgaggcagg ccgaagtgat 3360

taaggggtct tggcagcctg tgggcgatct gctgattgat tccctgcagg accacctgga 3420

aaaggtgaag gctctgagag gcgaaattgc tccactgaag gagaacgtga gtcatgtgaa 3480

cgatctggct agacagctga caacactggg catccagctg agcccataca atctgagcac 3540

actggaggac ctgaatacca ggtggaagct gctgcaggtg gctgtggaag accgggtgcg 3600

gcagctgcat gaggcccatc gcgacttcgg accagccagc cagcactttc tgagcacatc 3660

cgtgcagggg ccctgggaga gggccatttc tcccaacaag gtgccctact atattaatca 3720

cgagacccag accacttgtt gggaccatcc caagatgaca gaactgtacc agtccctggc 3780

cgatctgaac aacgtgaggt ttagcgctta cagaaccgct atgaagctga gacggctgca 3840

gaaggccctg tgcctggatc tgctgtccct gtccgccgcc tgcgatgccc tggatcagca 3900

taatctgaag cagaacgatc agccaatgga tatcctgcag atcatcaact gcctgaccac 3960

tatctacgac aggctggagc aggagcacaa caacctggtg aacgtgcctc tgtgcgtgga 4020

tatgtgcctg aactggctgc tgaacgtgta tgacactggg cgcaccggcc ggatcagagt 4080

gctgagtttt aaaactggga ttatctccct gtgtaaggcc cacctggagg acaagtacag 4140

gtacctgttc aagcaggtgg ctagtagcac tggattttgt gaccagcgcc gcctgggact 4200

gctgctgcat gatagtatcc agattcctag acagctggga gaggtggcta gtttcggagg 4260

atctaacatc gaacccagcg tgcgcagctg tttccagttt gccaataaca aacctgaaat 4320

cgaggctgct ctgttcctgg attggatgcg cctggaacca cagagcatgg tgtggctgcc 4380

tgtgctgcac agagtggctg ccgccgaaac tgccaagcac caggctaaat gcaacatctg 4440

caaggaatgt cccattatcg gctttcgcta caggagtctg aaacatttta actacgatat 4500

ttgccagagc tgcttctttt ccggaagagt ggccaaagga cacaagatgc actaccctat 4560

ggtggaatat tgcaccccaa ctacatctgg cgaagatgtg cgcgattttg ccaaggtgct 4620

gaagaataag tttcggacta agaggtactt cgccaagcac ccccgcatgg ggtatctgcc 4680

agtgcagaca gtgctggaag gagacaatat ggagaccgat acaatgtgag cggccgcaat 4740

aaaagatctt tattttcatt agatctgtgt gttggttttt tgtgtgtcta gagtcgacca 4800

gagcatggct acgtagataa gtagcatggc gggttaatca ttaactacaa ggaaccccta 4860

gtgatggagt tggccactcc ctctctgcgc gctcgctcgc tcactgaggc cgggcgacca 4920

aaggtcgccc gacgcccggg ctttgcccgg gcggcctcag tgagcgagcg agcgcgcagc 4980

tgcattaatg aatcggccaa cgcgcgggga gaggcggttt gcgtattggg cgctcttccg 5040

cttcctcgct cactgactcg ctgcgctcgg tcgttcggct gcggcgagcg gtatcagctc 5100

actcaaaggc ggtaatacgg ttatccacag aatcagggga taacgcagga aagaacatgt 5160

gagcaaaagg ccagcaaaag gccaggaacc gtaaaaaggc cgcgttgctg gcgtttttcc 5220
 
ataggctccg cccccctgac gagcatcaca aaaatcgacg ctcaagtcag aggtggcgaa 5280

acccgacagg actataaaga taccaggcgt ttccccctgg aagctccctc gtgcgctctc 5340

ctgttccgac cctgccgctt accggatacc tgtccgcctt tctcccttcg ggaagcgtgg 5400

cgctttctca tagctcacgc tgtaggtatc tcagttcggt gtaggtcgtt cgctccaagc 5460

tgggctgtgt gcacgaaccc cccgttcagc ccgaccgctg cgccttatcc ggtaactatc 5520

gtcttgagtc caacccggta agacacgact tatcgccact ggcagcagcc actggtaaca 5580

ggattagcag agcgaggtat gtaggcggtg ctacagagtt cttgaagtgg tggcctaact 5640

acggctacac tagaagaaca gtatttggta tctgcgctct gctgaagcca gttaccttcg 5700

gaaaaagagt tggtagctct tgatccggca aacaaaccac cgctggtagc ggtggttttt 5760

ttgtttgcaa gcagcagatt acgcgcagaa aaaaaggatc tcaagaagat cctttgatct 5820

tttctacggg gtctgacgct cagtggaacg aaaactcacg ttaagggatt ttggtcatga 5880

gattatcaaa aaggatcttc acctagatcc ttttaaatta aaaatgaagt tttaaatcaa 5940

tctaaagtat atatgagtaa aaatattccg gaattgccag ctggggcgcc ctctggtaag 6000

gttgggaagc cctgcaaagt aaactggatg gctttcttgc cgccaaggat ctgatggcgc 6060

aggggatcaa gatctgatca agagacagga tgaggatcgt ttcgcatgat tgaacaagat 6120

ggattgcacg caggttctcc ggccgcttgg gtggagaggc tattcggcta tgactgggca 6180

caacagacaa tcggctgctc tgatgccgcc gtgttccggc tgtcagcgca ggggcgcccg 6240

gttctttttg tcaagaccga cctgtccggt gccctgaatg aactgcagga cgaggcagcg 6300

cggctatcgt ggctggccac gacgggcgtt ccttgcgcag ctgtgctcga cgttgtcact 6360

gaagcgggaa gggactggct gctattgggc gaagtgccgg ggcaggatct cctgtcatcc 6420

caccttgctc ctgccgagaa agtatccatc atggctgatg caatgcggcg gctgcatacg 6480

cttgatccgg ctacctgccc attcgaccac caagcgaaac atcgcatcga gcgagcacgt 6540

actcggatgg aagccggtct tgtcgatcag gatgatctgg acgaagagca tcaggggctc 6600

gcgccagccg aactgttcgc caggctcaag gcgcgcatgc ccgacggcga ggatctcgtc 6660

gtgacccatg gcgatgcctg cttgccgaat atcatggtgg aaaatggccg cttttctgga 6720

ttcatcgact gtggccggct gggtgtggcg gaccgctatc aggacatagc gttggctacc 6780

cgtgatattg ctgaagagct tggcggcgaa tgggctgacc gcttcctcgt gctttacggt 6840

atcgccgctc ccgattcgca gcgcatcgcc ttctatcgcc ttcttgacga gttcttctga 6900

accggtaata ttattgaagc atttatcagg gttattgtct catgagcgga tacatatttg 6960

aatgtattta gaaaaataaa caaatagggg ttccgcgcac atttccccga aaagtgccac 7020

ctgacgtcta agaaaccatt attatcatga cattaaccta taaaaatagg cgtatcacga 7080

ggccctttcg tctcgcgcgt ttcggtgatg acggtgaaaa cctctgacac atgcagctcc 7140

cggagacggt cacagcttgt ctgtaagcgg atgccgggag cagacaagcc cgtcagggcg 7200

cgtcagcggg tgttggcggg tgtcggggct ggcttaacta tgcggcatca gagcagattg 7260
 
tactgagagt gcaccatatg cggtgtgaaa taccgcacag atgcgtaagg agaaaatacc 7320

gcatcaggcg attccaacat ccaataaatc atacaggcaa ggcaaagaat tagcaaaatt 7380

aagcaataaa gcctcagagc ataaagctaa atcggttgta ccaaaaacat tatgaccctg 7440

taatactttt gcgggagaag cctttatttc aacgcaagga taaaaatttt tagaaccctc 7500

atatatttta aatgcaatgc ctgagtaatg tgtaggtaaa gattcaaacg ggtgagaaag 7560

gccggagaca gtcaaatcac catcaatatg atattcaacc gttctagctg ataaattcat 7620

gccggagagg gtagctattt ttgagaggtc tctacaaagg ctatcaggtc attgcctgag 7680

agtctggagc aaacaagaga atcgatgaac ggtaatcgta aaactagcat gtcaatcata 7740

tgtaccccgg ttgataatca gaaaagcccc aaaaacagga agattgtata agcaaatatt 7800

taaattgtaa gcgttaatat tttgttaaaa ttcgcgttaa atttttgtta aatcagctca 7860

ttttttaacc aataggccga aatcggcaaa atcccttata aatcaaaaga atagaccgag 7920

atagggttga gtgttgttcc agtttggaac aagagtccac tattaaagaa cgtggactcc 7980

aacgtcaaag ggcgaaaaac cgtctatcag ggcgatggcc cactacgtga accatcaccc 8040

taatcaagtt ttttggggtc gaggtgccgt aaagcactaa atcggaaccc taaagggagc 8100

ccccgattta gagcttgacg gggaaagccg gcgaacgtgg cgagaaagga agggaagaaa 8160

gcgaaaggag cgggcgctag ggcgctggca agtgtagcgg tcacgctgcg cgtaaccacc 8220

acacccgccg cgcttaatgc gccgctacag ggcgcgtact atggttgctt tgacgagcac 8280

gtataacgtg ctttcctcgt tagaatcaga gcgggagcta aacaggaggc cgattaaagg 8340

gattttagac aggaacggta cgccagaatc ctgagaagtg tttttataat cagtgaggcc 8400

accgagtaaa agagtctgtc catcacgcaa attaaccgtt gtcgcaatac ttctttgatt 8460

agtaataaca tcacttgcct gagtagaaga actcaaacta tcggccttgc tggtaatatc 8520

cagaacaata ttaccgccag ccattgcaac ggaatcgcca ttcgccattc aggctgcgca 8580

actgttggga agggcgatcg gtgcgggcct cttcgctatt acgccagct 8629
<210> 9
   <211> 4977
   <212> DNA
   <213> Kanamycin Cassatte
<400> 9 
gcgcgctcgc tcgctcactg aggccgcccg ggcaaagccc gggcgtcggg cgacctttgg 60

tcgcccggcc tcagtgagcg agcgagcgcg cagagaggga gtggccaact ccatcactag 120

gggttccttg tagttaatga ttaacccgcc atgctactta tctacgtagc catgctctag 180

agtttaaaca agcttgcatg tctaagctag acccttcaga ttaaaaataa ctgaggtaag 240

ggcctgggta ggggaggtgg tgtgagacgc tcctgtctct cctctatctg cccatcggcc 300

ctttggggag gaggaatgtg cccaaggact aaaaaaaggc catggagcca gaggggcgag 360

ggcaacagac ctttcatggg caaaccttgg ggccctgctg tctagcatgc cccactacgg 420
 
gtctaggctg cccatgtaag gaggcaaggc ctggggacac ccgagatgcc tggttataat 480

taacccagac atgtggctgc cccccccccc ccaacacctg ctgcctctaa aaataaccct 540

gtccctggtg gatcccctgc atgcgaagat cttcgaacaa ggctgtgggg gactgagggc 600

aggctgtaac aggcttgggg gccagggctt atacgtgcct gggactccca aagtattact 660

gttccatgtt cccggcgaag ggccagctgt cccccgccag ctagactcag cacttagttt 720

aggaaccagt gagcaagtca gcccttgggg cagcccatac aaggccatgg ggctgggcaa 780

gctgcacgcc tgggtccggg gtgggcacgg tgcccgggca acgagctgaa agctcatctg 840

ctctcagggg cccctccctg gggacagccc ctcctggcta gtcacaccct gtaggctcct 900

ctatataacc caggggcaca ggggctgccc tcattctacc accacctcca cagcacagac 960

agacactcag gagcagccag cggcgcgccc aggtaagttt agtctttttg tcttttattt 1020

caggtcccgg atccggtggt ggtgcaaatc aaagaactgc tcctcagtgg atgttgcctt 1080

tacttctagg cctgtacgga agtgttactt ctgctctaaa agctgcggaa ttgtacccgc 1140

ggccgccacc atgctgtggt gggaggaggt ggaggattgt tatgaaaggg aggacgtgca 1200

gaagaagact tttaccaagt gggtgaacgc tcagttcagc aaatttggga agcagcacat 1260

cgagaatctg ttttccgacc tgcaggatgg gagacggctg ctggatctgc tggaaggact 1320

gactggccag aagctgccca aagagaaggg gagcactagg gtgcacgccc tgaacaacgt 1380

gaacaaagct ctgagagtgc tgcagaacaa caacgtggat ctggtgaata ttggcagtac 1440

tgatatcgtg gacgggaacc acaaactgac actgggcctg atctggaaca ttattctgca 1500

ctggcaggtg aaaaatgtga tgaagaacat catggccggg ctgcagcaga ccaattccga 1560

gaagatcctg ctgtcttggg tgcggcagag cacccgcaac tatccccagg tgaacgtgat 1620

taacttcact acatcctgga gcgacgggct ggccctgaat gctctgattc acagccacag 1680

gcctgatctg ttcgactgga atagcgtggt gtgccagcag tctgccacac agcgcctgga 1740

acatgccttc aatatcgctc ggtaccagct ggggatcgaa aaactgctgg acccagagga 1800

tgtggacact acatacccag ataaaaagtc tattctgatg tacattacta gcctgttcca 1860

ggtgctgcca cagcaggtgt ctattgaagc cattcaggag gtggaaatgc tgccccgccc 1920

ccccaaagtg actaaagagg agcattttca gctgcatcat cagatgcatt acagccagca 1980

gattaccgtg agcctggctc agggatatga gcgcaccagt agtccaaaac cacggttcaa 2040

gtcctacgct tatacccagg ctgcctacgt gacaactagc gaccctacta gatccccctt 2100

tccatcccag cacctggagg ccccagagga caagagcttt gggtccagcc tgatggaaag 2160

cgaggtgaat ctggatcggt accagacagc cctggaggag gtgctgagct ggctgctgag 2220

tgctgaagac acactgcagg cccagggcga aatttccaat gacgtggaag tggtgaagga 2280

tcagttccac acacacgagg gctatatgat ggacctgaca gctcaccagg ggcgcgtggg 2340

caatatcctg cagctgggct ctaaactgat cggcaccggg aaactgagtg aggacgagga 2400

aacagaagtg caggagcaga tgaacctgct gaacagccgc tgggagtgtc tgagagtggc 2460
 
tagtatggag aagcagtcca acctgcaccg ggtgctgatg gacctgcaga accagaaact 2520

gaaagagctg aacgactggc tgacaaagac tgaggaacgc acaaggaaga tggaggagga 2580

gccactggga cccgacctgg aggatctgaa gagacaggtg cagcagcata aggtgctgca 2640

ggaggatctg gaacaggagc aggtgcgggt gaactccctg acacatatgg tggtggtggt 2700

ggacgaatct agtggagatc acgccaccgc cgccctggag gaacagctga aggtgctggg 2760

ggaccggtgg gccaacattt gccggtggac cgaggacagg tgggtgctgc tgcaggacat 2820

cctgctgaaa tggcagaggc tgaccgagga gcagtgtctg tttagtgctt ggctgagcga 2880

gaaagaggac gccgtgaaca agatccacac aaccggcttt aaggatcaga acgaaatgct 2940

gtctagcctg cagaaactgg ctgtgctgaa ggccgatctg gagaaaaaga agcagagcat 3000

gggcaaactg tatagcctga aacaggacct gctgagcacc ctgaagaaca agagcgtgac 3060

ccagaagaca gaagcctggc tggataactt tgcccgctgc tgggacaacc tggtgcagaa 3120

actggagaaa agtacagctc agatctctca ggctgtgacc acaacccagc ctagcctgac 3180

ccagacaacc gtgatggaaa ccgtgaccac cgtgacaacc cgcgaacaga tcctggtgaa 3240

acatgcccag gaagagctgc cacctccacc tccccagaag aagagaaccc tggagcggct 3300

gcaggagctg caggaagcca ctgacgaact ggacctgaag ctgaggcagg ccgaagtgat 3360

taaggggtct tggcagcctg tgggcgatct gctgattgat tccctgcagg accacctgga 3420

aaaggtgaag gctctgagag gcgaaattgc tccactgaag gagaacgtga gtcatgtgaa 3480

cgatctggct agacagctga caacactggg catccagctg agcccataca atctgagcac 3540

actggaggac ctgaatacca ggtggaagct gctgcaggtg gctgtggaag accgggtgcg 3600

gcagctgcat gaggcccatc gcgacttcgg accagccagc cagcactttc tgagcacatc 3660

cgtgcagggg ccctgggaga gggccatttc tcccaacaag gtgccctact atattaatca 3720

cgagacccag accacttgtt gggaccatcc caagatgaca gaactgtacc agtccctggc 3780

cgatctgaac aacgtgaggt ttagcgctta cagaaccgct atgaagctga gacggctgca 3840

gaaggccctg tgcctggatc tgctgtccct gtccgccgcc tgcgatgccc tggatcagca 3900

taatctgaag cagaacgatc agccaatgga tatcctgcag atcatcaact gcctgaccac 3960

tatctacgac aggctggagc aggagcacaa caacctggtg aacgtgcctc tgtgcgtgga 4020

tatgtgcctg aactggctgc tgaacgtgta tgacactggg cgcaccggcc ggatcagagt 4080

gctgagtttt aaaactggga ttatctccct gtgtaaggcc cacctggagg acaagtacag 4140

gtacctgttc aagcaggtgg ctagtagcac tggattttgt gaccagcgcc gcctgggact 4200

gctgctgcat gatagtatcc agattcctag acagctggga gaggtggcta gtttcggagg 4260

atctaacatc gaacccagcg tgcgcagctg tttccagttt gccaataaca aacctgaaat 4320

cgaggctgct ctgttcctgg attggatgcg cctggaacca cagagcatgg tgtggctgcc 4380

tgtgctgcac agagtggctg ccgccgaaac tgccaagcac caggctaaat gcaacatctg 4440

caaggaatgt cccattatcg gctttcgcta caggagtctg aaacatttta actacgatat 4500
 
ttgccagagc tgcttctttt ccggaagagt ggccaaagga cacaagatgc actaccctat 4560

ggtggaatat tgcaccccaa ctacatctgg cgaagatgtg cgcgattttg ccaaggtgct 4620

gaagaataag tttcggacta agaggtactt cgccaagcac ccccgcatgg ggtatctgcc 4680

agtgcagaca gtgctggaag gagacaatat ggagaccgat acaatgtgag cggccgcaat 4740

aaaagatctt tattttcatt agatctgtgt gttggttttt tgtgtgtcta gagtcgacca 4800

gagcatggct acgtagataa gtagcatggc gggttaatca ttaactacaa ggaaccccta 4860

gtgatggagt tggccactcc ctctctgcgc gctcgctcgc tcactgaggc cgggcgacca 4920

aaggtcgccc gacgcccggg ctttgcccgg gcggcctcag tgagcgagcg agcgcgc 4977
The invention is further described by the following numbered paragraphs (paras):
1. A method of treating muscular dystrophy in a human subject in need thereof comprising the step of administering a recombinant adeno-virus associated (rAAV) rAAV.MHCK7.microdystrophin, wherein
   the rAAV is administered using a systemic route of administration and at a dose of about 5.0x10¹² vg/kg to about 1.0x10¹⁵
2. The method of para 1 wherein the systemic route of administration is an intravenous route and the dose of the rAAV administered is about 2x 10¹⁴ vg/kg.
3. The method of para 1 or 2 wherein the dose of rAAV is administered at a concentration of about 10 mL/kg.
4. The method of any one of paras 1-3 wherein the rAAV is administered by injection, infusion or implantation.
5. The method of any one of paras 1-4 wherein the rAAV is administered by infusion over approximately one hour.
6. The method of any one of paras 1-5 wherein the rAAV is administered by an intravenous route through a peripheral limb vein.
7. The method of any one of paras 1-6 wherein the rAAV comprises the human micro-dystrophin nucleotide sequence of SEQ ID NO: 1.
8. The method of any one of paras 1-7 wherein the rAAV comprises the MHCK7 promoter sequence of SEQ ID NO: 2 or SEQ ID NO:7
9. The method of any one of paras 1-8 wherein the rAAV is of the serotype AAVrh.74.
10. The method of any one of paras 1-9 wherein the rAAV comprises the AAVrh74.MHCK7.micro-dystrophin construct nucleotide sequence of SEQ ID NO: 9 or of nucleotides 55-5021 of SEQ ID NO: 3.
11. The method of any one of paras 1-10 wherein the muscular dystrophy is Duchenne muscular dystrophy or Becker's muscular dystrophy.
12. The method of any one of paras 1-11 wherein the human subject is suffering from Duchenne muscular dystrophy, and the rAAV is administered by intravenous infusion over approximately one hour at a dose of about 2x 10¹⁴ vg/kg, and wherein the rAAV comprises the AAVrh74.MHCK7.micro-dystrophin construct nucleotide sequence of SEQ ID NO: 9 or of nucleotides 55-5021 of SEQ ID NO: 3.
13. The method of any one of paras 1-12 wherein the level of micro-dystrophin gene expression in a cell of the subject is increased after administration of the rAAV as compared to the level of micro-dystrophin gene expression before administration of the rAAV.
14. The method of para 13 wherein expression of the micro-dystrophin gene in the cell is detected by measuring the micro-dystrophin protein level by Western blot in muscle biopsied before and after administration of the rAAV.
15. The method of para 14 wherein the level of micro-dystrophin protein is increased by at least 72% after administration of rAAV compared to level of micro-dystrophin before administration of rAAV.
16. The method of para 13 wherein expression of the micro-dystrophin gene in the cell is detected by measuring the micro-dystrophin protein level by immunohistochemistry in muscle biopsies before and after administration of the rAAV.
17. The method of para 16 wherein the level of micro-dystrophin protein is increased by at least 72% after administration of rAAV compared to the level of micro-dystrophin before administration of rAAV.
18. The method of any one of paras 1-12 wherein the serum CK level in the subject is decreased after administration of the rAAV as compared to serum CK level before administration of the rAAV.
19. The method of para 18 wherein the serum CK level in the subject is decreased by 87% by 60 days after administration of the rAAV as compared to the serum CK level before administration of the rAAV.
20. The method of any one of paras 1-12 wherein the number of micro-dystrophin positive fibers in the muscle tissue of the subject is increased after administration of the rAAV as compared to the number of micro-dystrophin positive fibers before administration of the rAAV.
21. The method of para 20 wherein the number of micro-dystrophin positive fibers is detected by measuring the micro-dystrophin protein level by Western blot in muscle biopsies before and after administration of the rAAV.
22. The method of para 20 wherein the number of micro-dystrophin positive fibers is detected by measuring the micro-dystrophin protein level by immunohistochemistry in muscle biopsies before and after administration of the rAAV.
23. The method of any one of paras 1-12 wherein the level of alpha-sarcoglycan in the subject is increased after administration of the rAAV as compared to the level of alpha-sarcoglycan before administration of the rAAV.
24. The method of para 23 wherein the level of alpha-sarcoglycan is detected by measuring the alpha-sarcoglycan protein level by Western blot in muscle biopsies before and after administration of the rAAV.
25. The method of para 23 wherein the number of alpha-sarcoglycan is detected by measuring the alpha-sarcoglycan protein level by immunohistochemistry in muscle biopsies before and after administration of the rAAV.
26. The method of any one of paras 1-12 wherein the level of beta-sarcoglycan in the subject is increased after administration of the rAAV as compared to the level of the beta-sarcoglycan before administration of the rAAV.
27. The method of para 26 wherein the level of beta-sarcoglycan is detected by measuring the beta-sarcoglycan protein level by Western blot in muscle biopsies before and after administration of the rAAV.
28. The method of para 26 wherein the number of beta-sarcoglycan is detected by measuring the beta-sarcoglycan protein level by immunohistochemistry in muscle biopsies before and after administration of the rAAV.
25. The method of any one of paras 1-12 wherein disease progression in the subject is delayed after administration of the rAAV as measured by any of: the six minute walk test, time to rise, ascend 4 steps, ascend and descend 4 steps, North Star Ambulatory Assessment (NSAA), 10 meter timed test, 100 meter timed test, hand held dynamometry (HHD), Timed Up and Go, and/or Gross Motor Subtest Scaled (Bayley-III) score.
26. The method of para 25 wherein the subject has at least a 6-point improvement in NSAA score at least 270 days after administration of the rAAV as compared to NSAA score before administration of the rAAV.
27. The method of para 25 wherein the subject has at least 0.8 second improvement in time to rise at least 270 days after administration of the rAAV as compared to time to rise before administration of the rAAV.
28. The method of para 25 wherein the subject has at least 1.2 second improvement in time to ascend 4 steps test at least 270 days after administration of the rAAV as compared to time to ascend 4 steps test before administration of the rAAV.
29. The method of para 25 wherein the subject has at least 7 second improvement in 100 m timed test at least 270 days after administration of the rAAV as compared to the 100 m timed test before administration of the rAAV.
30. A method of expressing micro-dystrophin gene in a patient cell comprising administering to the patient the AAVrh74.MHCK7.micro-dystrophin construct nucleotide sequence of SEQ ID NO: 9 or of nucleotides 55-5021 of SEQ ID NO: 3.
31. The method of para 30 wherein expression of the micro-dystrophin gene in the patient cell is detected by measuring the micro-dystrophin protein level by Western blot in muscle biopsies before and after administration of the rAAV.MHCK7.micro-dystrophin construct.
32. The method of para 30 wherein expression of the micro-dystrophin gene in the patient cell is detected by measuring the micro-dystrophin protein level by immunohistochemistry in muscle biopsies before and after administration of the rAAV.MHCK7.micro-dystrophin construct.
33. The method of para 30 wherein expression of the micro-dystrophin gene is measured in the patient by detecting greater than 1 rAAV vector genome copy per nucleus.
34. A method of decreasing serum CK levels in a patient in need thereof, the method comprising administering to the patient the AAVrh74.MHCK7.micro-dystrophin construct nucleotide sequence of SEQ ID NO: 9 or of nucleotides 55-5021 of SEQ ID NO: 3.
35. The method of para 34 wherein the serum CK level in the patient is decreased by at least 87% by 60 days after administration of the rAAV as compared to the serum CK level before administration of the rAAV.
36. A method of increasing micro-dystrophin positive fibers in a patient muscle tissue comprising administering to the patient the AAVrh74.MHCK7.micro-dystrophin construct nucleotide sequence of SEQ ID NO: 9 or of nucleotides 55-5021 of SEQ ID NO: 3.
37. The method of para 36 wherein the number of micro-dystrophin positive fibers is detected by measuring the dystrophin protein level by Western blot in muscle biopsies before and after administration of the rAAV.
38. The method of para 36 wherein the number of micro-dystrophin positive fibers is detected by measuring the dystrophin protein level by immunohistochemistry in muscle biopsies before and after administration of the rAAV.
39. The method of para 36 wherein the number of micro-dystrophin positive fibers is measured by detecting greater than 1 rAAV vector genome copy per nucleus.
40. A method of increasing the expression of alpha-sarcoglycan in a patient in need thereof comprising administering to the patient the AAVrh74.MHCK7.micro-dystrophin construct nucleotide sequence of SEQ ID NO: 9 or of nucleotides 55-5021 of SEQ ID NO: 3.
41. The method of para 40 wherein the level of alpha-sarcoglycan is detected by measuring the alpha-sarcoglycan protein level by Western blot on muscle biopsies before and after administration of the rAAV.
42. The method of para 40 wherein the number of alpha-sarcoglycan is detected by measuring the alpha-sarcoglycan protein level by immunohistochemistry in muscle biopsies before and after administration of the rAAV.
43. A method of increasing the expression of beta-sarcoglycan in a patient in need thereof comprising administering to the patient the AAVrh74.MHCK7.micro-dystrophin construct nucleotide sequence of SEQ ID NO: 9 or of nucleotides 55-5021 of SEQ ID NO: 3.
44. The method of para 43 wherein the level of beta-sarcoglycan is detected by measuring the beta-sarcoglycan protein level by Western blot in muscle biopsies before and after administration of the rAAV.
45. The method of para 43 wherein the number of beta-sarcoglycan is detected by measuring the beta-sarcoglycan protein level by immunohistochemistry on muscle biopsies before and after administration of the rAAV.
46. A method of treating a patient with Duchenne muscular dystrophy or Becker muscular dystrophy comprising administering to the patient the AAVrh74.MHCK7.micro-dystrophin construct nucleotide sequence of SEQ ID NO: 9
   or of nucleotides 55-5021 of SEQ ID NO: 3such that disease progression in the patient is delayed as measured by any of: the six minute walk test, time to rise, ascend 4 steps, ascend and descend 4 steps, North Star Ambulatory Assessment (NSAA), 10 meter timed test, 100 meter timed test, hand held dynamometry (HHD), Timed Up and Go, and/or Gross Motor Subtest Scaled (Bayley-III) score.
47. The method of para 46 wherein the subject has at least a 6-point improvement in NSAA score at least 90 days after administration of the rAAV as compared to NSAA score before administration of the rAAV.
48. The method of para 46 wherein the subject has at least 0.8 second improvement in time to rise at least 90 days after administration of the rAAV as compared to time to rise before administration of the rAAV.
49. The method of para 46 wherein the subject has at least 1.2 second improvement in time to ascend 4 steps test at least 90 days after administration of the rAAV as compared to time to ascend 4 steps test before administration of the rAAV.
50. The method of para 46 wherein the subject has at least 7 second improvement in 100 m timed test at least 90 days after administration of the rAAV as compared to the 100 m timed test before administration of the rAAV.
51. A composition for treating a muscular dystrophy in a human subject in need thereof, wherein the composition comprises a recombinant adeno-virus associated (rAAV) rAAV.MHCK7.microdystrophin, wherein the composition is formulated for a systemic route of administration and the dose of the rAAV is about 5x10¹² vg/kg to about 1.0x 10¹⁵ vg/kg.
52. The composition of para 51 wherein the systemic route of administration is an intravenous route and the dose of the rAAV is about 2x 10¹⁴ vg/kg.
53. The composition of para 51 or 52 wherein the dose of rAAV is at about 10 mL/kg.
54. The composition of any one of paras 51-53, where the composition is formulated for administration by injection, infusion or implantation.
55. The composition of any one of paras 51-53 wherein the composition is formulated for administration by infusion over approximately one hour.
56. The composition of any one of paras 51-53 wherein the dose of composition is formulated for intravenous administration through a peripheral limb vein.
57. The composition of any one of paras 51-56 wherein the rAAV comprises the human micro-dystrophin nucleotide sequence of SEQ ID NO: 1.
58. The composition of any one of paras 51-57 wherein the rAAV comprises the MHCK7 promoter sequence of SEQ ID NO: 2 or SEQ ID NO:7.
59. The composition of any one of paras 51-58 wherein the rAAV is of the serotype AAVrh.74.
60. The composition of any one of paras 51-59 wherein the rAAV comprises the AAVrh74.MHCK7.micro-dystrophin construct nucleotide sequence of SEQ ID NO: 9 or of nucleotides 55-5021 of SEQ ID NO: 3.
61. The composition of any one of paras51-60, wherein the muscular dystrophy is Duchenne muscular dystrophy or Becker's muscular dystrophy.
62. A composition for treating Duchenne muscular dystrophy in a human subject in need thereof wherein the composition comprises a recombinant adeno-virus associated (rAAV) rAAV.MHCK7.microdystrophin, wherein
   the composition is formulated for administration by intravenous infusion over approximately one hour at a dose of about 2x 10¹⁴ vg/kg, and
   wherein the rAAV comprises the AAVrh7.4.MHCK7.micro-dystrophin construct nucleotide sequence of SEQ ID NO: 9 or of nucleotides 55-5021 of SEQ ID NO: 3.
63. Use of a recombinant adeno-virus associated (rAAV) rAAV.MHCK7.microdystrophin for the preparation of a medicament for the treatment of a muscular dystrophy in a human subject in need thereof, wherein the medicament is formulated for a systemic route of administration and comprises a dose of rAAV of about 1x10¹⁴ vg/kg to about 4x 10¹⁴ vg/kg.
64. Use of para 55 wherein the medicament is formulated for intravenous administration and the dose of the rAAV is about 2x 10¹⁴ vg/kg.
65. The use of para 63 or 64 wherein the dose of rAAV is at about 10 mL/kg.
66. The use of any one of paras 63-65, wherein the medicament is formulated for administration by injection, infusion or implantation.
67. The use of any one of paras 63-65 wherein the medicament is formulated for administered by infusion over approximately one hour.
68. The use of any one of paras 63-65 wherein the medicament is formulated for intravenous administration through a peripheral limb vein.
69. The use of any one of paras 63-68 wherein the rAAV comprises the human micro-dystrophin nucleotide sequence of SEQ ID NO: 1.
70. The use of any one of paras 63-69 wherein the rAAV comprises the MHCK7 promoter sequence of SEQ ID NO: 2 or SEQ ID NO:7.
71. The use of any one of paras 63-70 wherein the rAAV is of serotype AAVrh.74.
72. The use of any one of paras 63-70 wherein the rAAV comprises the AAVrh74.MHCK7.micro-dystrophin construct nucleotide sequence of SEQ ID NO: 9 or of nucleotides 55-5021 of SEQ ID NO: 3.
73. The use of any one of paras 63-71 wherein the muscular dystrophy is Duchenne muscular dystrophy or Becker's muscular dystrophy.
74. Use of a recombinant adeno-virus associated (rAAV) rAAV.MHCK7.microdystrophin for the preparation of a medicament for treating Duchenne muscular dystrophy in a human subject in need thereof, wherein the medicament is formulated for administration by intravenous infusion over approximately one hour and comprises a dose of the rAAV of about 2x 10¹⁴ vg/kg, and wherein the rAAV comprises the AAVrh74.MHCK7.micro-dystrophin construct nucleotide sequence of SEQ ID NO: 9 or of nucleotides 55-5021 of SEQ ID NO: 3.
75. The method of any one of paras 1-50, wherein the serum CK level in the subject is decreased after administration of the rAAV as compared to the serum CK level before administration of the rAAV by a percentage level selected from the group consisting of:
   a) at least 78% by 90, 180, or 270 days after the administration;
   b) at least 46, 55, 56, 57, 58, 59, 60, 61, 62, 63, 64, 65, 66, 67, 68, 69, 70, or 85% by 270 days after the administration;
   c) at least 72, 73, 74, or 95% by 180 days after the administration;
   d) at least 87, 88, 93 or 95% by 90 days after the administration;
   e) at least 70 % by 270 days after the administration;
   f) 70 to 95% by 90, 180, or 270 days after the administration;
   g) at least 55, 56, 57, 58, 59, 60, 61, 62, 63, 64, 65, 66, 67, 68, 69, 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration; and
   h) at least 55, 56, 57, 58, 59, 60, 61, 62, 63, 64, 65, 66, 67, 68, 69, 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration.
76. The composition of any one of paras 51-62 wherein after administration of said composition to a human subject in need of treatment for muscular dystrophy, the serum CK level in the subject is decreased as compared to the serum CK level before administration of the composition by a percentage level selected from the group consisting of:
   a) at least 78% by 90, 180, or 270 days after the administration;
   b) at least 46, 55, 56, 57, 58, 59, 60, 61, 62, 63, 64, 65, 66, 67, 68, 69, 70 or 85% by 270 days after the administration;
   c) at least 72, 73, 74, or 95 % by 180 days after the administration;
   d) at least 87, 88, 93 or 95% by 90 days after the administration;
   e) at least 70 % by 270 days after the administration;
   f) 70 to 95% by 90, 180, or 270 days after the administration;
   g) at least 55, 56, 57, 58, 59, 60, 61, 62, 63, 64, 65, 66, 67, 68, 69, 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration; and
   h) at least 55, 56, 57, 58, 59, 60, 61, 62, 63, 64, 65, 66, 67, 68, 69, 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration.
77. The use according to any one of paras 63-74 wherein the serum CK level in the subject is decreased after administration of the rAAV to the subject as compared to the serum CK level before administration of the rAAV by a percentage level selected from the group consisting of:
   a) at least 78% by 90, 180, or 270 days after the administration;
   b) at least 46, 55, 56, 57, 58, 59, 60, 61, 62, 63, 64, 65, 66, 67, 68, 69, 70, or 85 % by 270 days after the administration;
   c) at least 72, 73, 74 or 95 % by 180 days after the administration;
   d) at least 87, 88, 93 or 95% by 90 days after the administration;
   e) at least 70 % by 270 days after the administration;
   f) 70 to 95% by 90, 180, or 270 days after the administration;
   g) at least 55, 56, 57, 58, 59, 60, 61, 62, 63, 64, 65, 66, 67, 68, 69, 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration; and
   h) at least 55, 56, 57, 58, 59, 60, 61, 62, 63, 64, 65, 66, 67, 68, 69, 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration.
78. A composition comprising
   a) the nucleotide sequence of SEQ ID NO: 3,
   b) the nucleotide sequence of SEQ ID NO: 8,
   c) the nucleotide sequence of SEQ ID NO: 9,
   d) rAAV comprising nucleotides 55-5021 of SEQ ID NO: 3,
   e) rAAV comprising the nucleic acid sequence of SEQ ID NO: 9,
   f) rAAV comprising nucleotides 1-4977 of SEQ ID NO: 8,
   g) rAAV particles comprising nucleotides 55-5021 of SEQ ID NO: 3,
   h) rAAV particles comprising the nucleic acid sequence of SEQ ID NO: 9, or
   i) rAAV particles comprising nucleotides 1-4977 of SEQ ID NO: 8.

## Claims

1. A recombinant adeno-associated virus (rAAV) for use in treating muscular dystrophy in a human subject in need thereof, wherein the rAAV is of the serotype rh.74 and comprises a polynucleotide comprising the nucleotide sequence of SEQ ID NO: 9, and wherein the rAAV is administered using a systemic route of administration and at a dose of 5x10¹² vg/kg to 1.0x10¹⁵ vg/kg.

2. The rAAV for use according to claim 1, wherein the systemic route of administration is an intravenous route.

3. The rAAV for use according to claim 1 or 2, wherein the composition is administered by infusion over approximately one hour.

4. The rAAV for use according to any preceding claim, wherein the muscular dystrophy is Duchenne muscular dystrophy or Becker's muscular dystrophy.

5. The rAAV for use according to any preceding claim, wherein the level of micro-dystrophin gene expression in a muscle cell of the subject is increased after administration of the rAAV as compared to the level of micro-dystrophin gene expression before administration of the rAAV.

6. The rAAV for use according to any preceding claim, wherein the level of micro-dystrophin protein is increased by at least 72% after administration of rAAV compared to level of micro-dystrophin before administration of rAAV.

7. The rAAV for use according to any preceding claim, wherein the serum CK level in the subject is decreased after administration of the rAAV as compared to serum CK level before administration of the rAAV.

8. The rAAV for use according to any preceding claim, wherein the number of micro-dystrophin positive fibers in the muscle tissue of the subject is increased after administration of the rAAV as compared to the number of micro-dystrophin positive fibers before administration of the rAAV.

9. The rAAV for use according to any preceding claim, wherein the level of alpha-sarcoglycan or beta-sarcoglycan in the subject is increased after administration of the rAAV as compared to the level of alpha-sarcoglycan or beta-sarcoglycan before administration of the rAAV.

10. The rAAV for use according to any preceding claim, wherein the serum CK level in the subject is decreased after administration of the rAAV as compared to the serum CK level before administration of the rAAV by a percentage level selected from the group consisting of:
a) at least 78% by 90, 180, or 270 days after the administration;
b) at least 46, 55, 56, 57, 58, 59, 60, 61, 62, 63, 64, 65, 66, 67, 68, 69, 70, or 85% by 270 days after the administration;
c) at least 72, 73, 74, or 95% by 180 days after the administration;
d) at least 87, 88, 93 or 95% by 90 days after the administration;
e) at least 70 % by 270 days after the administration;
f) 70 to 95% by 90, 180, or 270 days after the administration;
g) at least 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, or 95 % by 90, 180, or 270 days after the administration; and
h) at least 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, or 95 % by 90, 180, or 270 days after the administration.

11. The rAAV for use according to any preceding claim, wherein the rAAV is comprised in a composition.

12. A composition comprising the nucleotide sequence of SEQ ID NO: 9 or a recombinant adeno-associated virus (rAAV) comprising a polynucleotide comprising the nucleic acid sequence of SEQ ID NO: 9.

13. A composition comprising:
a) a rAAV comprising a polynucleotide comprising nucleotides 55-5021 of SEQ ID NO: 3,
b) a rAAV comprising a polynucleotide comprising nucleotides 1-4977 of SEQ ID NO: 8, or
c) a rAAV comprising a polynucleotide comprising nucleotides 56-5022 of SEQ ID NO: 6.

14. The composition of claim 12 or 13, wherein the composition comprises a rAAV at a dose of 5x10¹² vg/kg to 1.0x10¹⁵ vg/kg.

15. The composition of claim 14, wherein the composition comprises a rAAV at a dose of 2x10¹⁴ vg/kg.

16. The composition of any one of claims 14-15, wherein the dose of rAAV is delivered in about 10 mL/kg.

17. The composition of any one of claims 12-16, wherein the composition is formulated for administration by injection, infusion, or implantation.

18. The composition of claim 17, wherein the composition is formulated for administration by infusion over approximately one hour.

19. The composition of any one of claims 12-18, wherein the composition comprises the rAAV comprising a polynucleotide comprising nucleotides 55-5021 of SEQ ID NO: 3.

20. The composition of any one of claims 12-18, wherein the composition comprises the rAAV comprising a polynucleotide comprising nucleotides 1-4977 of SEQ ID NO: 8.

21. The composition of any one of claims 12-18, wherein the composition comprises the rAAV comprising a polynucleotide comprising nucleotides 56-5022 of SEQ ID NO: 6.

22. The composition of any one of claims 12-21, further comprising a pharmaceutically acceptable carrier.

23. The composition of any one of claims 12-22, further comprising a diluent and/or an adjuvant.
